# EUROPÄISCHE PATENTSCHRIFT

(11) **EP 0 807 183 B1**
(45) Veröffentlichungstag und Bekanntmachung des Hinweises auf die Patenterteilung: **03.01.2001**
(21) Anmeldenummer: 95931205.9
(22) Anmeldetag: 25.08.1995
(51) Int. Cl.: C12N 15/85, A61K 48/00

(54) **GENTHERAPIE VON ERKRANKUNGEN, DIE DURCH DAS IMMUNSYSTEM BEDINGT SIND, MIT HILFE EINES ZELLSPEZIFISCHEN ZELLZYKLUSREGULIERTEN WIRKSTOFFES**
GENETIC THERAPY OF DISEASES CAUSED BY THE IMMUNE SYSTEM, SAID THERAPY USING A CELL-SPECIFIC ACTIVE SUBSTANCE REGULATED BY THE CELL CYCLE
THERAPIE GENETIQUE DE MALADIES DEPENDANT DU SYSTEME IMMUNITAIRE, AVEC UTILISATION D'UNE SUBSTANCE ACTIVE SPECIFIQUE DE LA CELLULE ET REGULEE PAR LE CYCLE CELLULAIRE

(30) Priorität: 26.08.1994 GB 9417366; 29.03.1995 GB 9506466; 12.07.1995 DE 19524720
(43) Veröffentlichungstag der Anmeldung: 19.11.1997
(73) Patentinhaber: Aventis Pharma Deutschland GmbH, 65929 Frankfurt am Main (DE)
(72) Erfinder: SEDLACEK, Hans-Harald, D-35041 Marburg (DE); MÜLLER, Rolf, D-35037 Marburg (DE)
(74) Vertreter: Dost, Wolfgang, Dr.rer.nat., Dipl.-Chem.
(86) Internationale Anmeldenummer: EP9503371
(87) Internationale Veröffentlichungsnummer: WO9606941

(56) Entgegenhaltungen:
- WO-A-92/11359
- WO-A-93/25673

## Beschreibung

### Technisches Gebiet

Es wird eine DNA-Sequenz für die Gentherapie von Erkrankungen bedingt durch das Immunsystem beschrieben.

Die DNA-Sequenz besteht in ihren wesentlichen Elementen aus einer Aktivatorsequenz, einem Promotormodul und einem Gen für die Wirksubstanz.

Die Aktivatorsequenz wird zell- oder virusspezifisch aktiviert und diese Aktivierung zellzyklusspezifisch reguliert durch das Promotormodul. Die Wahl von Aktivatorsequenz und Wirksubstanz richtet sich nach dem Indikationsgebiet. Die DNA-Sequenz wird eingefügt in einen viralen oder nicht-viralen Vektor, ergänzt um einen Liganden mit Affinität zur Zielzelle.

Je nach Wahl der Aktivatorsequenz und Wirksubstanz können durch Verabreichung der DNA-Sequenz behandelt werden:
- mangelhafte Bildung von Blutzellen
- Autoimmunerkrankungen und Allergien desweiteren Abstoßungsreaktionen gegen transplantierte Organe
- chronische Gelenkentzündungen
- virale und parasitäre Infektionen, desweiteren Prophylaxe von viralen, bakteriellen und parasitären Infektionen
- Leukämien.

Ein fehlerhaftes Immunsystem verursacht äußerst vielgestaltige Erkrankungen. Zu ihnen gehören beispielsweise
- die unzureichende Bildung von Zellen des Blutes durch mangelnde Cytokine
- Allergien, Autoimmunerkrankungen und chronischen Entzündungen, im besonderen chronische Gelenkentzündungen durch Fehlfunktionen des Immunsystems
- Abstoßung transplantierter Organe durch eine unzureichende Hemmbarkeit des Immunsystems
- mangelhafte Impferfolge und chronische Infektionen, z.B. durch Viren im Gefolge einer Immunschwäche
- Leukämien und Lymphome als tumoröse Entartung des Immunsystems.

Die derzeitigen therapeutischen Möglichkeiten für derartige Erkrankungen sind bekanntermaßen unzureichend. Dieses soll an einigen Beispielen dargestellt werden.

### 1) Therapie mit Cytokinen

Mittlerweile sind eine beträchtliche Anzahl von Cytokinen und Wachstumsfaktoren bekannt, welche in die Differenzierung, Vermehrung, Reifung und Funktion von Zellen eingreifen.

So wird beispielsweise das blutbildende System gesteuert von einer Hierarchie von verschiedenen Cytokinen, welche durch ihre unterschiedlichen Funktionen die Vermehrung der einzelnen Differenzierungsstufen und über die einzelnen Differenzierungsstufen hinweg die fortlaufende Bildung von ausgereiften Blutzellen wie Erythrozyten, Thrombozyten, Granulozyten, Makrophagen und Lymphozyten gewährleisten (Dexter et al., Haematopoietic Growth Factors, Gardiner Well Communication, Macclesfield (1993)).

Des weiteren ist bekannt, daß Cytokine und Wachstumsfaktoren eine bedeutende Rolle spielen bei der Kooperation von Zellen miteinander (Pusztal et al., J. Pathol. 169, 191 (1993), Cross et al., Cell 64, 271 (1991)).

So wird beispielsweise bei der Immunabwehr die Zusammenarbeit zwischen antigenpräsentierenden Zellen, T-Lymphozyten und B-Lymphozyten gesteuert von unterschiedlichen Cytokinen, wobei deren Reihenfolge und Konzentration entscheidend ist für Art und Stärke der Immunreaktion (Aulitzky et al., Drugs 48, 667 (1994), Sedlacek et al., Immune Reactions, Springer Verlag (1995)). Des weiteren wird die Abwehr von Infektionserregern, wie beispielsweise von Viren, beeinflußt wie auch gestützt von Cytokinen wie beispielsweise Interferonen (Edgington, Biotechnol. 11, 465 (1993)).

Die Kenntnis dieser Zusammenhänge hat bereits zur Entwicklung von Cytokinen für die Therapie von Erkrankungen des Menschen geführt, beispielsweise von
- Erythropoietin für die Behebung der Anämie
- G-CSF für die Behebung der Neutropenie
- GM-CSF für die Behebung der Leukopenie
- IL-2 für die Immunabwehr gegen ausgewählte Tumoren
- IFNα für die Therapie der chronischen Virushepatitis
- IFNβ für die Therapie der multiplen Sklerose

Weitere Cytokine befinden sich derzeit in Prüfung (Aulitzky et al., Drugs 48, 667 (1994)). So beispielsweise
- Thrombopoietin für die Behebung der Thrombozytopenie (Metcalf, Nature 369, 519 (1994))
- IL-3 für die Tumortherapie (de Vries et al., Stem Cells 11, 72 (1993) und für die Unterstützung bei der Behebung von zytopenischen Zuständen des blutbildenden Systems (Freudl, Int. J. Immunopharm. 14, 421 (1992))
- IL-4 für die Tumortherapie (Manate et al., Blood 83, 1731 (1994))
- IL-6 für die Behebung von zytopenischen Zuständen des blutbildenden Systems (Brack et al., Int. J. Clin. Lab. Res. 22, 143 (1992))
- IL-10 für die Immunsuppression (Benjamin et al., Leuk. Lymph. 12, 205 (1994))
- IL-11 für die Behebung der Thrombozytopenie (Kobayashi et al., Blood 4, 889 (1993))
- IL-12 für die Tumortherapie (Tahara et al., Cancer Res. 54, 182 (1994))
- TNFα für die Tumortherapie (Porter, Tibitech 9, 158 (1991)).

Der Therapie mit allen Cytokinen gemeinsam ist der Nachteil, daß sie meist über einen längeren Zeitraum täglich parenteral zu verabreichen sind und des weiteren, daß zu ihrer bestmöglichen Wirksamkeit mehrere Cytokine in der notwendigen hierarchischen Reihenfolge nacheinander injiziert werden oder entsprechende Cytokine in ausreichender Konzentration im Körper vorhanden sein müssen.

Entscheidend für die Wirkung ist die Konzentration der jeweiligen Cytokine am Ort der zu stimulierenden Zelle. Der Einfachheit halber werden die Cytokine täglich subkutan oder i.m. injiziert. Diese Applikationsart gewährleistet eine angestrebte verzögerte systemische Verteilung, andererseits sind relativ hohe Mengen zu verabreichen, um eine ausreichende Konzentration lokal am Ort der gewünschten Wirkung zu gewährleisten. Die hierdurch bedingte erhöhte Dosis stellt aufgrund des hohen Herstellpreises der Cytokine einen beträchtlichen Kostenfaktor dar, der die Verwendung von Cytokinen erheblich einschränkt.

Darüber hinaus verursachen einige Cytokine im therapeutischen Dosisbereich beträchtliche Nebenwirkungen. So beispielsweise IL-1 (Smith et al., New Engl. J. Med. 328, 756 (1993)), IL-3 (Kurzrock et al., J. Clin. Oncol. 9, 1241 (1991)) und II-2 (Siegel et al., J. Clin. Oncol. 9, 694 (1991)).

Es besteht somit ein erheblicher Bedarf nach neuen Verfahren, Cytokine, bzw. Kombinationen von Cytokinen über einen längeren Zeitraum in ausreichender Konzentration am Ort ihrer Wirkung zur Verfügung stellen zu können.

### 2) Die chronische Gelenkentzündung

Die chronische Gelenkentzündung ist trotz verbesserter antientzündlicher und immunsuppressiver Medikamente eine nur unzulänglich therapierbare Erkrankung, welche die Lebensqualität erheblich einschränkt und sogar die Lebenserwartung verkürzen kann (Pincus et al., Bull. Rheum. Dis. 41, 1 (1992)). Durch ihre Häufigkeit (ca. 10% der Bevölkerung in der westlichen Welt leidet an Arthritis) stellt die Arthritis einen erheblichen volkswirtschaftlichen Kostenfaktor dar.

In Anbetracht der unzulänglichen medikamentösen Therapie ist für viele Patienten die chirurgische Entfernung der Synovia der Gelenkkapsel (Synovektomie) oder der chirurgische Gelenkersatz die letztmögliche Therapieform.

In Anbetracht dieser medizinischen und volkswirtschaftlichen Probleme stellt die chronische Arthritis eine Herausforderung für die Arzneimittelforschung dar.

Es ist jedoch bereits heute abzusehen, daß Medikamente, gleich welcher Art, die oral oder parenteral verabreicht werden, Schwierigkeiten haben werden, in ausreichender Konzentration den Entzündungsbereich des Gelenkes zu erreichen, da sie durch die synovialen Kapillaren passiv durch das Synovium in den Gelenkraum und von dort in die das Gelenk auskleidende Zellen diffundieren müssen (Evans et al., Gene Therapeutics, J. Wolff, Editor, page 320, Birkhäuser, Boston 1994).

Diese Diffusion wird zusätzlich dadurch erschwert, daß die Vaskularisierung des Synoviums beispielsweise bei der rheumatoiden Arthritis deutlich vermindert ist (Stevens et al., Arthritis Rheum. 34, 1508 (1991)). Eine intraartikuläre Injektion von Medikamenten umgeht zwar das Problem der Diffusion, aufgrund der hohen Resorptionsrate ist die Verweildauer des Medikamentes im Gelenk jedoch so kurz, daß über einen längeren Zeitraum mehrfache intraartikuläre Injektionen notwendig sind. Diese sind wiederum mit dem erheblichen Risiko einer Gelenkinfektion behaftet. Zudem können sie durch die notwendige hohe lokale Konzentration des Medikaments in erheblichem Maße Nebenwirkungen verursachen.

Zur Behebung dieser Probleme wurde die systemische oder die lokale intraartikuläre Verabreichung von Vektoren oder von in vitro transduzierten Synovialzellen zur Therapie der chronischen Arthritis vorgeschlagen (Bandara et al., DNA Cell Biol. 11, 227 (1992), BBA 1134, 309 (1992) Evans et al., Transplant. Proc. 24, 2966 (1992)).

Das Prinzip dieser Gentherapie ist, durch in vivo im Gelenkraum transduzierte Zellen oder durch die Injektion von in vitro transduzierten Synovialzellen in den Gelenkraum hohe Konzentrationen von antiarthritischen Substanzen zu erreichen, wie beispielsweise (Evans et al., J. Rheumatol. 21, 779 (1994))
- antiinflammatorischen Cytokinen
   (z.B. IL-1-Rezeptor Antagonist, IL-4 oder IL-10)
- Cytokininhibitoren
   (z.B. lösliche Rezeptoren für IL-1, TNFα, IL-8, TGFβ, oder für andere, die Entzündung verstärkende Cytokine und Interleukine)
- Enzyminhibitoren
   (z.B. TIMP, LIMP, IMP, PAI-1, PAI-2, andere)
- Antiadhäsionsmolekülen
   (z.B. lösliches CD-18, ICAM-1, lösliches CD44)
- Antagonisten von Sauerstoffradikalen
   (z.B. Superoxiddismutase)
- oder von
- Wachstumsfaktoren für Knorpelzellen
   (z.B. TGFβ oder IGF-1)

Tierexperimentell konnten im Kaninchen Anhaltspunkte für eine Wirksamkeit von IL-1-RA exprimiert nach intraartikuläre Injektion des entsprechenden Gens nachgewiesen werden (Bandara et al., PNAS 90, 10764 (1993), Hung et al., Gene Therapy 1, 64 (1994)).

Grundsätzlich sind jedoch diese bislang vorgeschlagenen Methoden zur Gentherapie mit erheblichen Nachteilen behaftet:
- Bei der in vitro Transduktion von Synovialzellen sind diese aus dem Gelenkraum zu entnehmen. Dieses alleine belastet den Patienten und birgt in sich das Risiko einer Gelenkinfektion. Zum anderen sind Synovialzellen nur sehr schwierig und in geringer Zahl zu gewinnen. Somit müssen die Synovialzellen in vitro vermehrt werden, um sie in ausreichender Zahl transduzieren zu können. Es ist jedoch bekannt, daß nur die Fibroblasten-ähnlichen Synovialzellen (Typ B) sich unter Standard-Zellkulturbedingungen vermehren lassen, nicht jedoch der den Makrophagen ähnelnde Typ A (Evans et al., Gene Therapeutics, page 320, J.A. Wolff, Editor, Birkäuser Boston (1994)). Die Injektion von in vitro transduzierten Synovialzellen ist somit mit erheblichen Problemen belastet und wird technisch meist nicht oder nur mit erheblichem Aufwand zu bewerkstelligen sein.
- Bei der zur Diskussion stehenden systemischen oder intraartikulären Injektion von Vektoren zur in vivo Transduktion von Zellen (Evans et al., Gene Therapeutics, page 320, J.A. Wolff, Editor, Birkäuser Boston (1994)) fehlt ein Regelmechanismus, welcher nur in solchen Zellen, welche bei der chronischen Arthritis involviert und nur dann, wenn sie im Sinne einer Entzündung aktiviert sind, eine Expression der über den Vektor transferierten Gene erlaubt. Ohne einen derartigen Regelmechanismus werden nach systemischer Verabreichung des Vektors verteilt im gesamten Körper Zellen transduziert, die jeweilige antiarthritische Substanz zu produzieren, was entweder zu einer systemischen Beeinflussung der Immunreaktion führen würde, oder aber in bezug auf den arthritischen Entzündungsprozeß gleichbedeutend wäre mit der an sich bereits als unwirksam oder unzureichend wirksam angesehenen, wiederholten systemischen Gabe von antiarthritischen Wirkstoffen.

Nach lokaler Verabreichung würden je nach verwendetem Vektor entweder vorzugsweise proliferierende Zellen (bei Verwendung eines RTV-Vektors) oder aber zusätzlich auch ruhende Zellen (bei Verwendung anderer viraler oder nichtviraler Vektoren) in vivo transduziert werden können, die antiarthritische Substanz zu produzieren. Da ein großer Teil derartiger Substanzen antiinflammatorisch wirken, würden die Immun- und Entzündungsreaktionen in den Gelenken gehemmt sein, unabhängig, ob die chronische Arthritis sich in einer Ruhephase oder aber in einem akuten Erkrankungsschub befindet. Durch die lokale Hemmung der Immunreaktion begünstigt und durch die kausalen Faktoren der chronischen Arthritis bewirkt, wäre das Risiko eine gesteigerte pathologische Immun- und Entzündungsreaktion nach Abklingen der Wirksamkeit der antiarthritisch wirkenden Substanzen, jedoch keine weitgehende Linderung oder Heilung der Arthritis.

Es besteht somit ein dringender Bedarf nach neuen therapeutischen Verfahren oder Wirkstoffen,
- welche je nach Anzahl und Schwere der chronisch entzündeten Gelenke einem Patienten lokal oder systemisch gegeben werden können
- deren Wirkung vorzugsweise wenn nicht ausschließlich auf aktivierte und proliferierende Synovialzellen oder auch Entzündungszellen beschränkt bleibt
- deren Wirkungen vorzugsweise in der längerfristigen Prophylaxe und Therapie des akuten Entzündungsschubes bestehen.

### 3) Leukämien und Lymphome

Patienten mit Tumoren des blutbildenden Systems, welche nach einer vorübergehend erfolgreichen Chemotherapie ein Rezidiv erleiden, haben eine relativ schlechte Prognose (Hiddemann et al., Blood Rev. 8, 225 (1994)). Demzufolge wurden verschiedene intensive Behandlungsstrategien entwickelt, um die Überlebenszeit zu verlängern.

Hierzu gehören unterschiedliche Kombinationen von Zytostatika (The Medica Letter 31, 49 (1989)), wie auch die Knochenmarktransplantation (De Magalhaes-Silverman et al., Cell Transplant. 2, 75 (1993)). Beide Therapieansätze sind jedoch nur begrenzt wirksam (Sloane et al., Histophathol. 22 201 (1993)). Es besteht somit weiterhin ein erheblicher medizinischer Bedarf nach neuen, wirksamen Therapeutika für Tumoren des blutbildenden Systems.

Tumorzellen des blutbildenden Systems besitzen je nach Typ des Tumors ausgeprägte molekularbiologische Veränderungen (Übersicht bei Lotter et al., Cancer Surveys 16, 157 (1993), Yunis et al., Crit. Rev. Onc. 4, 161 (1993)). Besonders ausgeprägt sind hierbei beispielsweise
- Burkitt's lymphome (BL): - Deregulierung von c-myc mit exzessiver c-myc-mRNA und c-myc-Proteinproduktion (Mc Keithan, Seminars in Oncol. 17, 30 (1990))
- Überexpression von bcl-2 (Tsujimoto et al., PNAS 86, 1958 (1989))
- B-Zell-Leukämien und - lymphome (BCL): - Überexpression von bcl-2 (in 85% der Patienten mit follikulärem Lymphom und 25% der Patienten mit diffusem Lymphom) (Yunis et al., New Engl. J. Med. 316, 79 (1987))
- Überexpression von bcl-1 in Patienten mit zentrozytischem Lymphom (Seto et al., Oncogene 7, 1401 (1992))
- Überexpression von IL-6 (Lewis et al., Nature 317, 544 (1985))
- Überexpression von IL-10 (Levine, Blood 80, 8 (1992))
- akute B-Zell-Leukämie (aBCL): - Expression des Fusionsproteins E2A-PBX-1 (Yunis et al., Crit. Rev. Onco. 4, 161 (1993))
- T-Zell-Lymphome (TCL): - Überexpression von c-myc
(Cotter, Cancer Surveys 16, 157 (1993))
- Überexpression von HOX11 (syn. TCL3)
(Hatano et al., Science 253, 79 (1991))
- chronische myeloische Leukämie (CML): - Expression des Fusionsproteins BCR-Abl (Daley et al., PNAS 88, 11335 (1991))
- akute lymphatische Leukämie (ALL): - Überexpression von IL-3 (Mecker et al., Blood 76, 285 (1990))
- akute myeloische Leukämie (AML): - Expression des Fusionsproteins PML/RARA (Alcalay et al., PNAS 89, 4840 (1992), Pandolfi et al., EMBO J. 11, 1397 (1992))

Bislang konnten diese molekularbiologischen Veränderungen jedoch noch nicht für klinische Therapieverfahren genutzt werden.

### 4) Allgemeine Beschreibung der Erfindung

Gegenstand der vorliegenden Erfindung ist nunmehr ein Wirkstoff (d. h. ein Arzneimittel), welcher lokal wie auch systemisch Patienten gegeben werden kann und welcher eine zellspezifische, zellzyklusregulierte Bildung von Wirksubstanzen zur Therapie von Erkrankungen des Immunsystems bewirkt.

Wesentlicher Bestandteil des Wirkstoffes ist ein DNA-Konstrukt folgender Zusammensetzung 
Aktivatorsequenz | zellzyklusreguliertes | Wirksubstanz
(UAS) | Promotormodul
 (DNA wird im gesamten Text dieser Anmeldung als gemeinsamer Begriff sowohl für eine komplementäre (cDNA) wie auch für eine genomische DNA-Sequenz benutzt).

### 4.1. Beschreibung des Promotormoduls

Das zentrale Element des erfindungsgemäßen Wirkstoffes stellt ein zellzyklusreguliertes Promotormodul dar.

Als zellzyklusreguliertes Promotormodul ist beispielsweise die Nukleotidsequenz -CDE-CHR-Inr- (siehe unten) zu verstehen. Die wesentliche Funktion des Promotormodulteiles ist die Hemmung der Funktion der Aktivatorsequenz in der G0/G1 Phase des Zellzyklus und eine Zellzyklusspezifische Expression in der S/G2 Phase und damit in proliferierenden Zellen.

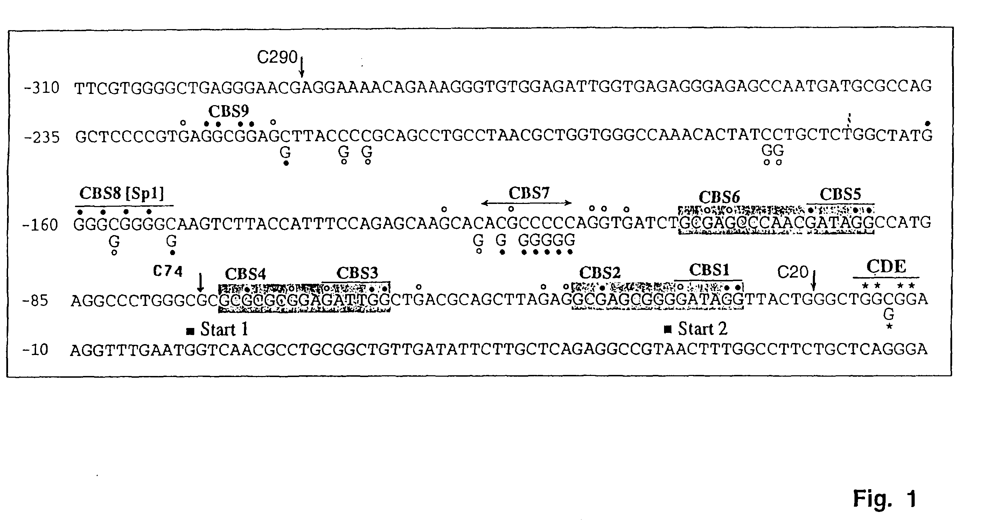

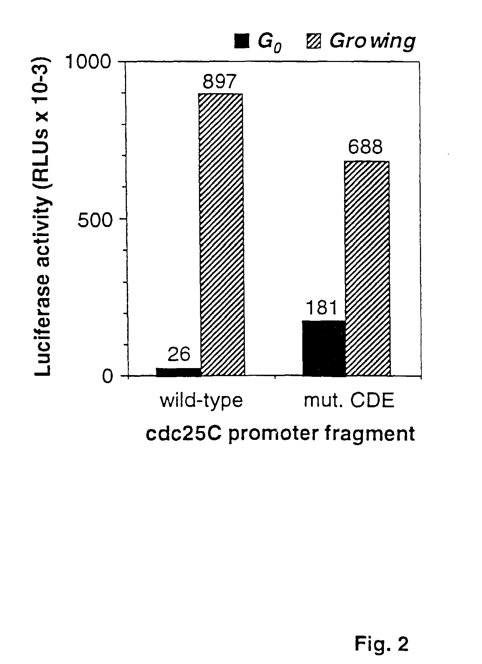

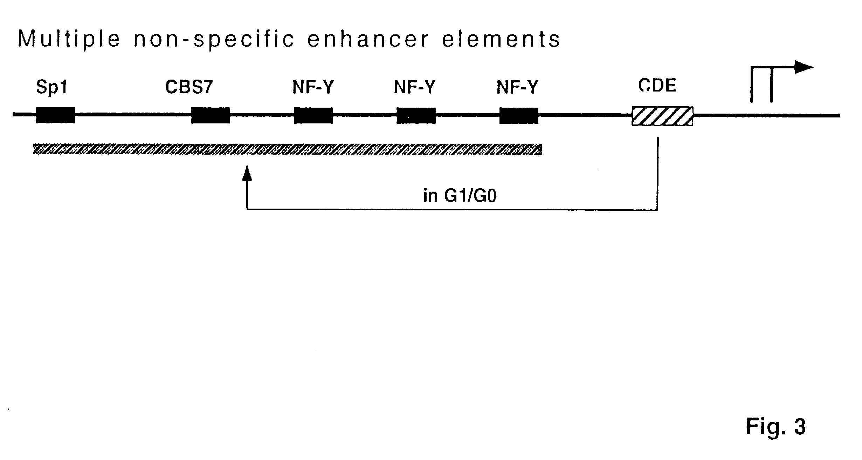

Das Promotormodul CDE-CHR-Inr wurde im Rahmen einer detaillierten Untersuchung der G2-spezifischen Expression des menschlichen cdc25C Promotors entdeckt. Ausgangspunkt war die Auffindung eines Repressorelementes ("cell cycle dependent element"; CDE), das für die Abschaltung des Promotors in der G1-Phase des Zellzyklus verantwortlich ist (Lucibello et al., EMBO J. 14, 132 (1995)). Durch genomisches Dimethylsulfat (DMS)-Footprinting und funktionelle Analysen (Fig. 1, 2) konnte gezeigt werden, daß das CDE einen Repressor ("CDE-binding factor"; CDF) G1-spezifisch bindet und hierdurch zu einer Inhibition der Transkription in nichtproliferierenden (G0) Zellen führt. Das im Bereich des Basalpromotors lokalisierte CDE ist in seiner reprimierenden Funktion abhängig von einer "upstream activating sequence" (UAS). Dies führte zu dem Schluß, daß der CDE-bindende Faktor die transkriptionsaktivierende Wirkung 5' gebundener Aktivatorproteine in einer zellzyklusabhängigen Weise, d.h. in nichtproliferierenden Zellen sowie in der G1-Phase des Zellzyklus, hemmt (Fig. 3).

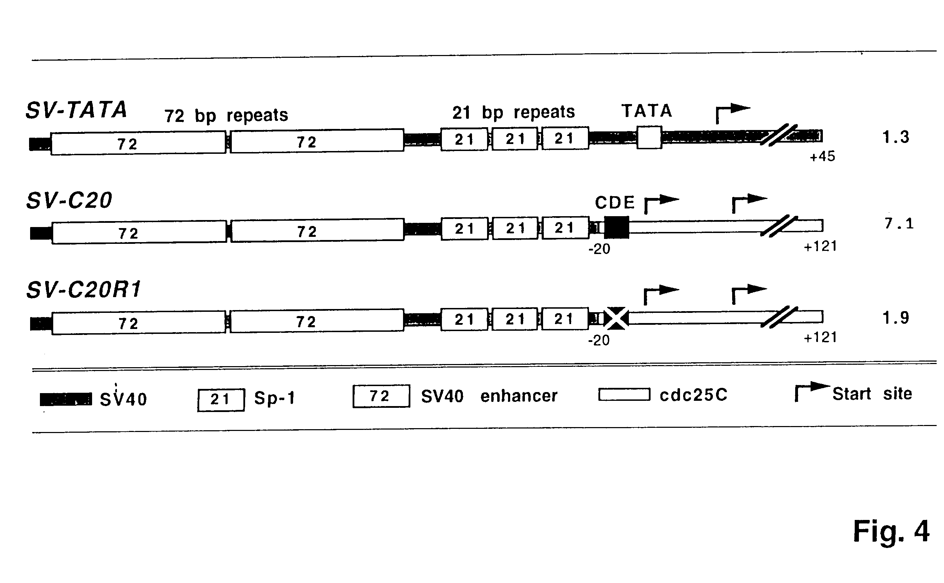

Diese Schlußfolgerung konnte durch ein weiteres Experiment bestätigt werden: Die Fusion des viralen, nicht-zellzyklusregulierten frühen SV40-Enhancers mit einem cdc25 Minimalpromotor (bestehend aus CDE und den 3' gelegenen Startstellen) führte zu einer klaren Zellzyklusregulation des chimären Promotors (Fig. 4). Nachfolgende Untersuchungen des cdc25C Enhancers haben gezeigt, daß es sich bei den vom CDF zellzyklusabhängig regulierten Transkriptionsfaktoren um NF-Y (CBF) (Dorn et al., Cell 50, 863 (1987), van Hujisduijnen et al., EMBO J. 9, 3119 (1990), Coustry et al., J. Biol. Chem. 270, 468 (1995)), Sp1 (Kadonaga et al., TIBS 11, 10 (1986) und einen möglicherweise neuen an CBS7 bindenden Transkriptionsfaktor (CIF) handelt. Ein weiterer interessanter Befund dieser Studie war die Beobachtung, daß NF-Y innerhalb des cdc25C Enhancers nur in Kooperation mit mindestens einem weiteren NF-Y Komplex oder mit CIF effizient die Transkription aktiviert. Sowohl NF-Y als auch Sp1 gehören zur Klasse der glutaminreichen Aktivatoren, was wichtige Hinweise auf den Mechanismus der Repression (z.B. Interaktion bzw. Interferenz mit bestimmten basalen Transkriptionsfaktoren oder TAFs) liefert.

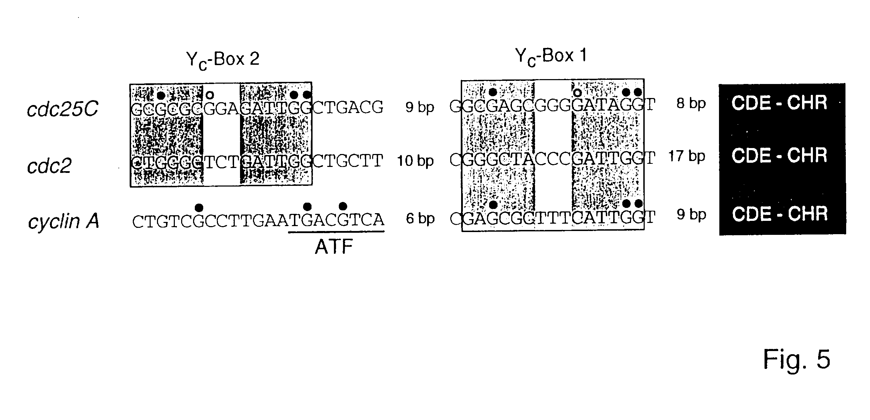

Ein Vergleich der Promotorsequenzen von cdc25C, cyclin A und cdc2 zeigte Homologien in mehreren Bereichen (Fig. 5). Nicht nur das CDE ist in allen 3 Promotoren konserviert (die vorhandenen Abweichungen sind funktionell nicht relevant), sondern auch die benachbarten Y_{c}-Boxen. Alle diese Bereiche zeigten wie erwartet Proteinbindung in vivo, im Falle des CDE in zellzyklusabhängiger Weise. Außerdem konnte gezeigt werden, daß alle 3 Promotoren durch eine Mutation des CDE dereguliert werden (Tabelle 1). Eine bemerkenswerte Ähnlichkeit wurde bei dem Vergleich der cdc25C, cyclin A und cdc2 Sequenzen auch im Bereich unmittelbar 3' vom CDE ("cell cycle genes homology region"; CHR) deutlich (Fig. 5). Dieser Bereich ist funktionell ebenso bedeutsam wie das CDE (Tabelle 1), wird in den in vivo DMS Footprinting Experimenten jedoch nicht sichtbar. Eine mögliche Erklärung hierfür ist eine Interaktion des Faktors mit der kleinen Furche der DNA. Ergebnisse aus "electrophoretic mobility shift assay" (EMSA) Experimenten deuten darauf hin, daß CDE und CHR gemeinsam einen Proteinkomplex, den CDF, binden. Diese Beobachtungen weisen darauf hin, daß die CDF-vermittelte Repression glutaminreicher Aktivatoren ein häufig vorkommender Mechanismus zellzyklusregulierter Transkription ist.

Von Bedeutung für die Regulation des cdc25C Promotors ist jedoch anscheinend nicht nur der CDE-CHR-Bereich, sondern auch eine der Initiationsstellen (Position +1) innerhalb der Nukleotidsequenz des basalen Promotors (Positionen ≤ -20 bis ≥ +30, siehe Fig. 1). Mutationen in diesem Bereich, der die in vitro Bindestelle für YY-1 (Seto und Shenk, Nature 354, 241 (1991), Usheva und Shenk, Cell 76, 1115 (1994)) einschließt, führen zu einer völligen Deregulation. In Anbetracht der Nähe des CDE-CHR zum basalen Promotor ist somit eine Interaktion des CDF mit dem basalen Transkriptionskomplex sehr wahrscheinlich.

### 4.2. Beschreibung der Aktivatorsequenz

Als Aktivatorsequenz (UAS = upstream activator sequence) ist eine Nukleotidsequenz (Promotor- oder Enhancersequenz) zu verstehen, mit der Transkriptionsfaktoren, gebildet oder aktiv in der Zielzelle, interagieren. Als Aktivatorsequenz kann der CMV-Enhancer, der CMV-Promotor (EP 0173. 177.B1), der SV40 Promotor oder jede andere, dem Fachmann bekannte Promotor- oder Enhancersequenz verwendet werden.

Im Sinne dieser Erfindung zählen zu den bevorzugten Aktivatorsequenzen jedoch genregulatorische Sequenzen bzw. Elemente aus Genen, die besonders in Zellen des blutbildenden Systems, in aktivierten Lymphozyten, in aktivierten Synovialzellen oder Makrophagen, in virusinfizierten Zellen oder in Leukämiezellen gebildete Proteine kodieren.

### 4.3. Beschreibung der Wirksubstanz

Als Wirksubstanz ist die DNA für ein Protein zu verstehen, welches am Ort der Entstehung den therapeutischen Effekt, d.h. die Behebung der Erkrankung des Immunsystems, bewirken soll. Die Auswahl der Nukleotidsequenz für die Aktivatorsequenz und für die Wirksubstanz richtet sich nach der Zielzelle und der gewünschten Wirksubstanz.

### 4.4. Herstellung des Plasmids oder Vektors

Das erfindungsgemäße DNA-Konstrukt wird in einer dem Fachmann geläufigen Weise zu einem Vektor vervollständigt; so wird es beispielsweise in einen viralen Vektor eingefügt (siehe hierzu D. Jolly, Cancer Gene Therapy 1, 51 (1994)), oder als Plasmid verwendet. Virale Vektoren oder Plasmide können mit kolloidalen Dispersionen komplexiert werden, so beispielsweise mit Liposomen (Farhood et al., Annals of the New York Academy of Sciences 716, 23 (1994)) oder aber mit einem Polylysin-Ligand-Konjugaten (Curiel et al., Annals of the New York Academy of Sciences 716, 36 (1994)).

### 4.5. Ergänzung durch einen Liganden

Derartige virale oder nicht-virale Vektoren können durch einen Liganden ergänzt werden, welcher Bindungsaffinität für eine Membranstruktur auf der ausgewählten Zielzelle hat. Die Auswahl des Liganden richtet sich somit nach der Auswahl der Zielzelle.

Der erfindungsgemäße Wirkstoff wird in folgenden Beispielen näher beschrieben:

### 5) Wirkstoff zur Behebung der mangelhaften Bildung von Zellen des Blutes

### 5.1. Auswahl der Aktivatorsequenz für blutbildenue Zellen

Im Sinne der vorliegenden Erfindung wird als Aktivatorsequenz bevorzugt eine genregulatorische Sequenz bzw. ein Element aus einem Gen verwendet, welches ein Protein kodiert, welches besonders stark oder selektiv in Zellen der Blutbildung exprimiert ist. Zu solchen genregulatorischen Sequenzen gehören Promotorsequenzen für Gene eines Cytokins oder seines Rezeptors, dessen Expression in den unreifen blutbildenden Zellen (oder in benachbarten Zellen, wie beispielsweise dem Stroma), dem nachfolgenden, auf die blutbildenden Zellen einwirkenden und als Wirksubstanz gewünschten Cytokin vorgeschaltet ist. Beispielsweise sind derartige auf unreife blutbildende Zellen einwirkende Cytokine
* Stem Cell Factor (Martin et al., Cell 63, 203 (1990)) vorgeschaltet allen haematopoietischen Faktoren (McNiece et al., Exp. Heamtol. 19, 226 (1991))
* IL-1 (Durum et al., Ann. Rev. Immunol. 3, 263 (1985))
* IL-3 (Clark-Lewis et al., J. Biol. Chem. 259, 7488 (1984), Oster et al., Int. J. Cell Clon. 9, 5 (1991))
* IL-6 (Mizel, FASEB J. 3, 2379 (1989))
* GM-CSF (Gasson, Blood 6, 1131 (1991), Dunlop et al., AntiCancer Drugs 2, 327 (1991))

Die Promotorsequenzen für diese Cytokine und ihre Rezeptoren sind durch folgende Arbeiten zugänglich:
* Stem Cell Factor-Receptor
* (Yamamoto et al., Jap. J. Cancer Res. 84, 1136 (1993)) * Stem Cell Factor
* (Szcylik et al., J. Exp. Med. 178, 997 (1993), Bowen et al., Leukemia 7, 1883 (1993), Yamamoto et al., Jp. J. Cancer Res. 84, 11 (1993))
* IL-1α
* (Hangen et al., Mol. Carcinog. 2, 68 (1986), Turner et et al., J. Immunol. 143, 3556 (1989), Mori et al., Blood 84, 1688 (1994))
* IL-1-Rezeptor
* (Ye et al., PNAS USA 90, 2295 (1993))
* IL-3
* (Mathey-Prevot et al., PNAS USA 87, 5046 (1990), Cameron et al., Blood 83, 2851 (1994), Arai et al., Lymphokine Res. 9, 551 (1990))
* IL-3-Rezeptor (∝-subunit)
* (Miyajima et al., Blood 85, 1246 (1995), Rapaport et al., Gene 137, 333 (1993), Kosugi et al., BBRC 208, 360 (1995))
* IL-3-Rezeptor (β-subunit)
* (Gorman et al., J. Biol. Chem. 267, 15842 (1992), Kitamura et al., Cell 66, 1165 (1991), Hayashida et al., PNAS USA 87, 9655 (1990))
* IL-6
* (Yukasawa et al., EMBO J. 6, 2939 (1987), Lu et al., J. Biol. Chem. 270, 9748 (1995), Ray et al., PNAS 85, 6701 (1988), Droogmans et al., DNA-Sequence 3, 115 (1992), Mori et al., Blood 84, 2904 (1994), Liberman et al., Mol Cell. Biol. 10, 2327 (1990), Ishiki et al., Mol. Cell. Biol. 10, 2757 (1990), Gruss et al., Blood 80, 2563 (1992))
* IL-6-Rezeptor
* (Yamasaki et al., Science 241, 825 (1988), Mullberg et al., J. Immunol. 152, 4958 (1994))
* GM-CSF
* (Nimer et al., Mol. Cell Biol. 10, 6084 (1990), Staynow et al., PNAS USA 92, 3606 (1995), Koyano-Nakayawa et al., Int. Immunol. 5, 345 (1993), Ye et al., Nucl. Acids Res. 22, 5672 (1994))
* GM-CSF-Rezeptor (α-Kette)
* (Nakagawa et al., J. Biol. Chem. 269, 10905 (1994))
* Interferon Regulatory Factor 1 (IRF-1)
* Der Promotor von IRF-1 wird durch IL-6 gleichermaßen aktiviert wie durch IFN-γ oder IFNβ.
* (Harrock et al., EMBO J. 13, 1942 (1994)).

### 5.2. Auswahl der Wirksubstanz für blutbildende Zellen

Als Wirksubstanz im Sinne der Erfindung ist eine DNA-Sequenz zu verstehen, deren exprimiertes Protein die Proliferation und/ oder Differenzierung von Blutzellen bewirkt.

Je nach Typ der Blutzellarmut sind beispielsweise folgende Wirksubstanzen zu wählen:
- Wirksubstanz für die Anämie:: DNA-Sequenz für Erythropoietin (Jacobs et al., Nature 313, 806 (1985), Lin et al., PNAS 82, 7580 (1985), Krantz, Blood 77, 419 (1991), Dube et al., J. Biol. Chem. 263, 17516 (1988)
- Wirksubstanz für die Leukopenie:: DNA-Sequenz für G-CSF (Nagata et al., EMBO J. 5, 575 (1986), Nagata et al., Nature 319, 415 (1986), Souza et al., Science 232, 61 (1986)) oder für GM-CSF (Gough et al., Nature 309, 763 (1984), Nicola et al., J. Biol. Chem. 254, 5290 (1979), Wong et al., Science 228, 810 (1985))
- Wirksubstanz für die Thrombozytopenie:: DNA für IL-3(Yang et al., Cell 47, 3 (1986) DNA für Leukemia Inhibitory Factor (LIF) (Metcalf, Int. J. Cell Clon. 9, 85 (1991), Sutherland et al., Leuk. 3, 9 (1989), Gough et al., PNAS USA 85, 2623 (1988), Gough et al., Ciba Found. Symp. 167, 24 (1992), Stahl et al., J. Biol. Chem. 265, 8833 (1990), Rathjan et al., Cell 62, 1105 (1990)) DNA-Sequenz für IL-11 (Kawashima et al., FEBS Lett. 283, 199 (1991), Paul et al., PNAS 87, 7512 (1990) und/oder DNA für Thrombopoietin (de Sauvage et al., Nature 369, 533 (1994), Kaushansky et al., Nature 369, 568 (1994), Wendling et al., Nature 369, 571 (1994)

Als Wirksubstanz im Sinne der Erfindung können jedoch auch DNA-Sequenzen von Fusionsproteinen zwischen den aufgeführten Cytokinen, Wachstumsfaktoren oder dem extrazellulären Teil der Rezeptoren zum einen und dem Fc-Teil des menschlichen Immunglobulins zum anderen Verwendung finden. Derartige DNA-Sequenzen und ihre Herstellung wurden in der EPA 0464 633 A1 beschrieben.

### 5.3. Kombination von gleichen oder unterschiedlen Wirksubstanzen für blutbildende Zellen

Gegenstand der Erfindung ist des weiteren ein Wirkstoff, in welchem eine Kombination der DNA-Sequenzen von mehreren gleichen Wirksubstanzen (A,A) oder unterschiedlichen Wirksubstanzen (A,B) vorliegt. Zur Expression zweier DNA-Sequenzen ist vorzugsweise die cDNA einer "internal ribosome entry site" (IRES) als regulatorisches Element zwischengeschaltet.

Derartige IRES wurden beispielsweise von Montford und Smith (TIG 11, 179 (1995), Kaufman et al., Nucl. Acids Res. 19, 4485 (1991), Morgan et al., Nucl. Acids Res. 20, 1293 (1992, Dirks et al., Gene 128, 247 (1993), Pelletier und Sonenberg, Nature 334, 320 (1988) und Sugitomo et al., BioTechn. 12, 694 (1994) beschrieben.

So kann die die cDNA der IRES-Sequenz des Poliovirus (Position ≤ 140 bis ≥ 630 des 5' UTR (Pelletier und Sonenberg, Nature 334, 320 (1988)) zur Verknüpfung der DNA der antientzündlichen Substanz A (am 3' Ende) und der DNA der antientzündlichen Substanz B (am 5' Terminus) verwendet werden.

Aktivator- zellzyklus- Wirksubstanz internal Wirksubstanz
sequenz reguliertes A ribosome A oder B
(UAS) Promotor- entry
modul site

Ein derartiger Wirkstoff weist je nach Kombination additive (A+A, A+B) oder synergistische (A+B) Wirkung auf.

### 5.4. Auswahl des Liganden für blutbildende Zellen

Ziel sollte sein, den Wirkstoff zu der Zielzelle oder zu Zellen benachbart der Zielzelle zu bringen. Hierzu können virale oder nicht-virale Vektoren mit einem Liganden versehen werden. Der Ligand sollte vorzugsweise mit Membranstrukturen oder Membranrezeptoren auf undifferenzierte oder gering differenzierte Blutzellen binden.

Zu den Liganden gehören Antikörper oder Antikörperfragmente, gerichtet gegen Rezeptoren exprimiert auf gering differenzierten Blutzellen.

Derartige Antikörper sind beispielsweise für folgende Rezeptoren beschrieben worden:
- Stem Cell Factor Receptor
- (Blechman et al., Cell 80, 103 (1995), Oez et al., Eur. Cytokine Netw. 4, 293 (1993))
- IL-1-Rezeptor (Type I)
- (McMahan et al., EMBO J. 10, 2821 (1991), Giri et al., Cytokine 4, 18 (1992),
- IL-1-Rezeptor (Type II)
- (Scapigliati et al., J. Immunol. Methods 138, 31(1991))
- IL-3-Rezeptor α
- (Sato et al., Blood 82,752(1993))
- IL-3-Rezeptor β
- (Korpelainen et al., Blood 86, 176 (1995))
- IL-6-Rezeptor
- (Daveau et al., Eur. Cytokine Netw. 5, 601 (1994), Sui et al., PNAS USA 92, 2859 (1995), Goto et al., Jpn. J. Cancer Res. 85, 958 (1994))
- GM-CSF-Rezeptor
- (Nicola et al., Blood 82, 1724 (1993))

Desweiteren gehören zu den Liganden auch monoklonale oder polyklonale Antikörper oder Antikörperfragmente, die mit ihren konstanten Domänen an Fc-γ Rezeptoren von Immunzellen binden (Rojanasakul et al., Pharm. Res. 11, 1731 (1994)).

Die murinen monoklonalen Antikörper sind bevorzugt in humanisierter Form einzusetzen. Die Humanisierung erfolgt in der von Winter et al. (Nature 349, 293 (1991)) und Hoogenbooms et al. (Rev. Tr. Transfus. Haemobiol. 36, 19 (1993)) dargestellten Weise. Antikörperfragmente werden entsprechend dem Stand der Technik hergestellt, beispielsweise in der von Winter et al. (Nature 349, 293 (1991), Hoogenboom et al. (Rev. Tr. Transfus. Hemobiol. 36, 19 (1993), Girol (Mol. Immunol. 28, 1379 (1991) und Huston et al. (Int. Rev. Immunol. 10, 195 (1993) beschriebenen Weise.

Zu den Liganden gehören des weiteren alle Substanzen, welche an Membranstrukturen oder Membranrezeptoren auf der Oberfläche von gering differenzierten Blutzellen Zellen binden. Beispielsweise gehören hierzu Wachstumsfaktoren, wie SCF, IL-1, IL-3, IL-6, GM-CSF oder deren Fragmente bzw. Teilsequenzen von ihnen, die an Rezeptoren exprimiert durch derartige Zellen binden.

### 5.5. Herstellung des Wirkstoffes für blutbildende Zellen

Die Herstellung des erfindungsgemäßen Wirkstoffes wird anhand folgender Beispiele näher beschrieben:

### a) Konstruktion des chimären Promotors SCF-Rezeptor-CDE-CHR-Inr

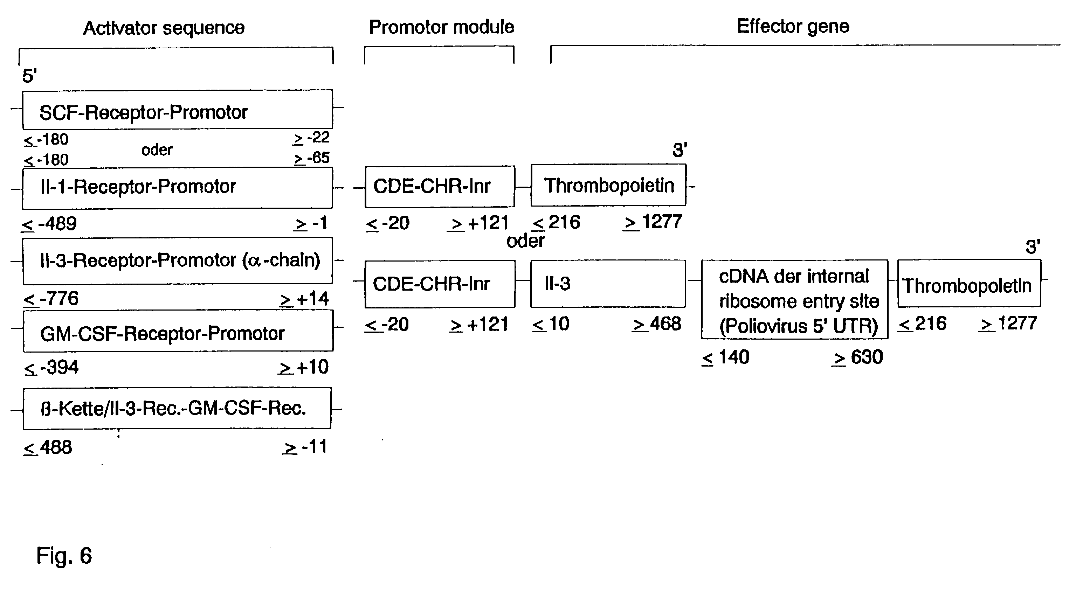

Der humane SCF-Rezeptor Promotor (Position ≤ -180 bis ≥ -22, Yamamoto et al., Jpn. J. Cancer Res. 84, 1136 (1993)) oder eine um die TATA-Box verkürzte Variante (Position ≤ -180 bis ≥ -65) werden an ihrem 3' Ende mit dem 5'-Terminus des CDE-CHR-Inr Moduls (Position ≤ -20 bis ≥ + 121) des humanen cdc25C-Gens (Lucibello et al., EMBO J., 14, 132 (1995)) verknüpft (Fig. 6). Die Verknüpfung erfolgt mit Hilfe von dem Fachmann bekannten und käuflichen Enzymen.

### b) Konstruktion eines Plasmids enthaltend den chimären Promotor SCF-Rezeptor-CDE-CHR-Inr im zentralen Bestandteil des Wirkstoffes

Die beschriebene chimäre SCF-Rezeptor-Repressormodul-Transkriptionseinheit wird an ihren 3' Enden mit dem 5'-Terminus einer DNA, die den kompletten kodierenden Bereich des Thrombopoietin (Position ≤ 216 bis ≥ 1277, (de Sauvage et al., Nature 369, 533 (1994)) enthält, verknüpft (Fig. 6). Diese DNA enthält auch die für eine Sekretion notwendige Signalsequenz. Transkriptionskontrolleinheiten und die DNA für Thrombopoietin werden in pUC19/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### c) Konstruktion des chimären Promotors IL-1-Rezeptor-CDE-CHR-Inr

Der humane IL-1-Rezeptor Promotor (Pos. ≤ -489 bis ≥ -1, Ye et al., PNAS USA 30, 2295 (1993)) wird an seinem 3' Ende mit dem 5'-Terminus des CDE-CHR-Inr Moduls des humanen cdc25C-Gens (Pos. ≤ -20 bis ≥ +121, der von Lucibello et al., EMBO J. 14, 132 (1995) publizierten Sequenz) verknüpft (siehe Fig. 6). Die Verknüpfung erfolgt mit Hilfe von dem Fachmann bekannten und käuflichen Enzymen.

### d) Konstruktion eines Plasmids enthaltend den chimären Promotor IL-1-Rezeptor-CDE-CHR-Inr im zentralen Bestandteil des Wirkstoffes

Die unter c) beschriebene chimäre IL-1-Rezeptor-Repressormodul-Transkriptionskontrolleinheit wird an ihrem 3' Ende mit dem 5'-Terminus einer DNA, die den kompletten kodierenden Bereich des Thrombopoietins enthält, verknüpft (siehe Fig. 6). Diese DNA enthält auch die für eine Sekretion notwendige Signalsequenz. Transkriptionskontrolleinheiten und die DNA für den Tissue Plasminogen Activator werden in pUC18/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### e) Konstruktion eines Plasmids enthaltend zwei Gene für Wirksubstanzen

Die unter a) geschilderte SCF-Rezeptor-CDE-CHR-Inr-Transkriptionseinheit wird an ihrem 3' Ende mit dem 5' Ende der DNA für das Interleukin-3 (Position ≤ 10 bis ≥ 468, Yang et al., Cell 47, 3 (1986)) verknüpft. Die Verknüpfung erfolgt mit Hilfe von dem Fachmann bekannten und käuflichen Enzymen.

Das 3' Ende der DNA für IL-3 wird nunmehr verknüpft mit dem 5' Ende der cDNA der internal ribosome entry site (Position≤ 140 bis ≥ 630; Pelletier und Sonnenberg, Nature 334, 320 (1988)) und anschließend wird deren 3' Ende mit dem 5' Ende der DNA für das Thrombopoietin verknüpft (siehe Fig. 6). Dieser so hergestellte Wirkstoff wird anschließend in puc18/19 oder in Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### f) Konstruktion weiterer Transkriptionseinheiten

Die Möglichkeit der Kombination des IL-3-Rezeptor (α-Kette) Promotors, GM-CSF-Rezeptor (α-Kette) Promotors oder des GM-CSF-Rezeptor (β-Kette) Promotors mit dem Repressormodul CDE-CHR-Inr und den bereits erwähnten Effektorgenen ist in Fig. 6 dargestellt.

### 6. Wirkstoff zur Therapie von Autoimmunerkrankungen, Allergien, Entzündungen und zur Verhütung von Organabstoßungen

### 6.1. Auswahl der Aktivatorsequenz für Autoimmunerkrankungen u.a.

Als Aktivatorsequenzen sind die Promotorsequenzen der Gene für solche Proteine zu verwenden, welche bei der Immunreaktion in Makrophagen und/oder in Lymphozyten verstärkt gebildet werden. Derartige Proteine sind beispielsweise
* IL-1 (Bensi et al., Gene 52, 95 (1987), Fibbe et al., Blut 59, 147 (1989))
* IL-1-Rezeptor (Colotta et al., Immunol. Today 15, 562 (1994), Sims et al., Clin. Immunol. Immunopath. 72, 9 1994), Ye et al., PNAS USA 90, 2295 (1993))
* IL-2 (Jansen et al. CII 39, 207 (1994), Ohbo et al., J. Biol. Chem. 270, 7479 (1995))
* IL-2-Rezeptor (Semenzato et al., Int. J. Clin Lab. Res. 22, 133 (1992))
* IFN γ (Kirchner, DMW 111, 64 (1986), Lehmann et al., J. Immunol. 153, 165 (1994))
* IL-4 (Paul, Blood 77, 1859 (1991), te Velde et al., Blood 76, 1392 (1990))
* IL-4-Rezeptor (Vallenga et al., Leukemia 7, 1131 (1993), Galizzi et al., Int. Immunol. 2, 669 (1990))
* IL-3 (Frendl, Int. J. Immunopharm. 14, 421 (1992))
* IL-5 (Azuma et al., Nucl. Acid Res. 14, 9149 (1986), Yokota et al., PNAS 84, 7388 (1987))
* IL-6 (Brack et al., Int. J. Clin. Lab. Res. 22, 143 (1992))
* LIF (Metcalf, Int. J. Cell Clon. 9, 95 (1991), Samal, BBA 1260, 27 (1995))
* IL-7 (Joshi et all, 21, 681 (1991))
* IL-10 (Benjamin et al., Leuk. Lymph. 12, 205 (1994), Fluchiger et al., J. Exp. Med. 179, 91 (1994))
* IL-11 (Yang et al., Biofactors 4, 15 (1992))
* IL-12 (Kiniwa et al., J. Clin. Invest. 90, 262 (1992), Gatelay, Cancer Invest. 11, 500 (1993))
* IL-13 (Punnonen et al., PNAS 90, 3730 (1993), Muzio et al., Blood 83, 1738 (1994))
* GM-CSF (Metcalf, Cancer 15, 2185 (1990))
* GM-CSF-Rezeptor (Nakagawa et al., J. Biol. Chem. 269, 10905 (1994))
* Integrin beta 2 proteins (LFA-1, MAC-1, p150/95) (Nueda et al., J. Biol. Chem. 268, 19305 (1993))

### Promotorsequenzen für diese Proteine wurden wie folgt beschrieben

* IL-1-Rezeptor
   (Ye et al., PNAS USA 90, 2295 (1993))
* IL-1α
   (Hangen et al., Mol. Carcinog. 2, 68 (1986), Turner et al., J. Immunol. 143, 3556 (1989), Mori et al., Blood 84, 1688 (1994))
* IL-1β
   (Fenton et al., J. Immunol. 138, 3972 (1987), Bensi et al., Cell Growth Diff. 1, 491 (1990), Turner et al., J. Immunol. 143, 3556 (1989), Hiscott et al., Mol. Cell. Biol. 13, 6231 (1993))
* IL-2
   (Fujita et al., Cell 46, 401 (1986), Hama et al., J. Exp. Med. 181, 1217 (1995), Kant et al., Lymph. Rec. Interact. 179 (1989), Kamps et al., Mol. Cell. Biol. 10, 5464 (1990), Williams et al., J. Immunol. 141, 662 (1988), Brunvand, FASEB J. 6, A998 (1992), Matsui et al., Lymphokines 12, 1 (1985), Tanaguchi et al., Nature 302, 305 (1983))
* IL-2-Rezeptor
   (Ohbo et al., J. Biol. Chem. 270, 7479 (1995), Shibuya et al., Nucl. Acids Res. 18, 3697 (1990), Lin et al., Mol. Cell. Biol. 13, 6201 (1993), Semenzato et al., Int. J. Clin. Lab. Res. 22, 133 (1992))
* IL-3
   (Mathey-Prevot et al., PNAS USA 87, 5046 (1990), Cameron et al., Blood 83, 2851 (1994), Arai et al., Lymphokine Res. 9, 551 (1990))
* IL-3-Rezeptor (α-subunit)
   (Miyajima et al., Blood 85, 1246 (1995), Rapaport et al., Gene 137, 333 (1993), Kosugi et al., BBRC 208, 360 (1995))
* IL-3-Rezeptor (β-subunit)
   (Gorman et al., J. Biol. Chem. 267, 15842 (1992), Kitamura et al., Cell 66, 1165 (1991), Hayashida et al., PNAS USA 87, 9655 (1990))
* IL-4
   (Rooney et al., EMBO J. 13, 625 (1994), Hama et al., J. Exp. Med. 181, 1217 (1995), Li-Weber et al., J. Immunol. 153, 4122 (1994), 148, 1913 (1992), Min et al., J. Immunol. 148, 1913 (1992), Abe et al., PNAS 89, 2864 (1992))
* IL-4-Rezeptor
   (Beckmann et al., Chem. Immunol. 51, 107 (1992), Ohara et al., PNAS 85, 8221 (1988))
* IL-5
   (Lee et al., J. Allerg. Clin. Immunol. 94, 594 (1994), Kauhansky et al., J. Immunol. 152, 1812 (1994), Staynov et al., PNAS USA 92, 3606 (1995))
* IL-6
   (Lu et al., J. Biol. Chem. 270, 9748 (1995), Gruss et al., Blood 80, 2563 (1992), Ray et al., PNAS 85, 6701 (1988), Droogmans et al., DNA-Sequence 3, 115 (1992), Mori et al., Blood 84, 2904 (1994), Liberman et al., Mol Cell. Biol. 10, 2327 (1990), Ishiki et al., Mol. Cell. Biol. 10, 2757 (1990))
* Interferon Regulatory Factor 1 (IRF-1)
   (Der Promotor von IRF-1 wird durch IL-6 gleichermaßen aktiviert wie durch IFN-γ oder IFNβ. (Harrock et al., EMBO J. 13, 1942 (1994))
* IFN-γResponsive Promotor
   (Lamb et al., Blood 83, 2063 (1994))
* IL-7
   (Pleiman et al., Mol. Cell. Biol. 11, 3052 (1991), Lapton et al., J. Immunol. 144, 3592 (1990))
* IL-8
   (Chang et al., J. Biol. Chem. 269, 25277 (1994), Sprenger et al., J. Immunol. 153, 2524 (1994))
* IL-10
   (Kim et al., J. Immunol. 148, 3618 (1992), Platzer et al., DNA Sequence 4, 399 (1994), Kube et al., Cytokine 7, 1 (1995))
* IL-11
   (Yang et al., J. Biol. Chem. 269, 32732 (1994))
* IFN-γ
   (Ye et al., J. Biol. Chem. 269, 25728 (1994), Hardy et al., PNAS 82, 8173 (1985))
* GM-CSF
   (Nimer et al., Mol. Cell. Biol. 10, 6084 (1990), Staynov et al., PNAS USA 92, 3606 (1995), Koyano-Nakayawa et al., Int. Immunol. 5, 345 (1993), Ye et al., Nucl. Acids Res. 22, 5672 (1994))
* GM-CSF-Rezeptor (α-Kette)
   (Nakagawa et al., J. Biol. Chem. 269, 10905 (1994))
* IL-13
   (Staynov et al., PNAS USA 92, 3606 (1995)
* LIF
   (Gough et al., Ciba Found. Symp. 167, 24 (1992) Stahl et al., Cytokine 5, 386 (1993))
* Makrophagen-Colony Stimulating Factor (M-CSF)-Rezeptor (Yue et al., Mol. Cell. Biol. 13, 3191 (1993), Zhang et al., Mol. Cell. Biol. 14, 373 (1994))
* Typ I und II Makrophagen Scavenger Rezeptoren
   (Moulton et al., Mol. Cell. Biol. 14, 4408 (1994))
* MAC-1 (Leukozytenfunktionsantigen)
   (Dziennis et al., Blood 85, 319 (1995), Bauer et al., Hum. Gene Ther. 5, 709 (1994), Hickstein et al., PNAS USA 89, 2105 (1992))
* LFA-1α (Leukozytenfunktionsantigen)
   (Nueda et al., J. Biol. Chem. 268, 19305 (1993), Agura et al., Blood 79, 602 (1992), Cornwell et al., PNAS USA 90, 4221(1993))
* p150,95 (Leukozytenfunktionsantigen)
   (Noti et al., DNA and Cell Biol. 11, 123 (1992), Lopezcabrera et al., J. Biol. Chem. 268, 1187 (1993))

Die Auflistung der Promotoren für Cytokine und Cytokinrezeptoren ist nur beispielhaft und soll nicht als Beschränkung verstanden werden.

Bei den verschiedenen Autoimmunerkrankungen kann beispielsweise folgende Promotorsequenz gewählt werden:
- bei Allergien: Rezeptor, IL-2, die Promotorsequenzen für IL-1, IL-1-IL-2-Rezeptor, IL-4 oder IL-4-Rezeptor
- bei Zell- oder Antikörper-mediierten Autoimmunerkrankungen: die Promotorsequenzen für IL-1, IL-1-Rezeptor, IL-2, IL-2-Rezeptor
- zur Verhinderung der Rezeptor, Organabstoßung: die Promotorsequenzen für IL-1, IL-1-IL-2, IL-2-Rezeptor

### 6.2. Auswahl der Wirksubstanz für Autoimmunerkrankungen u.a.

Die Wirksubstanz im Sinne der Erfindung ist die DNA-Sequenz für ein Cytokin, ein Chemokin, einen Wachstumsfaktor oder einer ihrer Inhibitoren, einen Antikörper oder ein Enzym. Die Auswahl der Wirksubstanz richtet sich nach der zu behandelnden Grunderkrankung und der gewählten Aktivatorsequenz.

Beispielsweise kann bei folgenden Erkrankungen eine der folgenden Wirksubstanzen gewählt werden:
- a) Wirksubstanz zur Therapie von Allergien: DNA-Sequenz für IFN∝ (Henco et al., J. Mol. Biol. 185, 227 (1985), Pestka et al., Ann. Rev. Biochem. 56, 727 (1987), Weissmann et al., Phil. Trans. R. Soc. Lond. B299, 7 (1982), Goeddel et al., Nature 290, 20 (1981))
oder IFNβ
(Sen et al., J. Biol. Chem. 267, 5017 (1992), Mark et al., EP 192 811, EP 234 599, US 45 88 585)
oder IFN-γ
(Gray et al., Nature 295, 503 (1982), Yip et al., PNAS USA 79, 1820 (1982), Rinderknecht et al., J. Biol. Chem. 259, 6790 (1984))
oder IL-10
(Moore et al., Science 248, 1230 (1990), Vieira et al., PNAS USA 88, 1172 (1991), Kim et al., J. Immunol. 148 3618 (1992))
oder lösliche IL-4-Rezeptoren
(Idzerda et al., J. Exp. Med. 171, 861 (1990), EPA 0419 091 A1, Foxwell, Eur. J. Immunol. 19, 1637 (1989), Garrone et al., Eur. J. Immunol. 21, 1365 (1991), Gallizzi et al., Int. Immunol. 2, 226 (1990), Park et al., J. Exp. Med. 166, 476 (1987))
oder IL-12
(Kobayashi et al., J. Exp. Med. 170, 827 (1989), Gabler et al., PNAS 88, 4143 (1991), Gately et al., J. Immunol. 147, 874 (1991), Schoenhaut et al., J. Immunol. 148, 3433 (1992), Wolf et al., J. Immunol. 146, 3074 (1991))
oder TGFβ
(Massague, Ann. Rev. Cell. Biol. 6, 597 (1990), Kondiah et al., J. Biol. Chem. 265, 1089 (1990), Garnier et al., J. Molec. Biol. 120, 97 (1978))
- b) Wirksubstanz zur Verhinderung der Abstoßung von transplantierten Organen: DNA-Sequenz für IL-10 (Moore et al., Science 248, 1230 (1990), Vieira et al., PNASUSA 88, 1172 (1991), Kim et al., J.Immunol. 148, 3618 (1992))
oder TGFβ
(Massague, Ann. Rev. CellBiol. 6, 597 (1990), Kondiah et al., J. Biol. Chem. 265, 1089 (1990), Garnier et al., J. Mol. Biol. 120, 97 (1978)
oder lösliche IL-1-Rezeptoren
(Sims et al., PNAS USA 86, 8946 (1989) (I), Dower et al., J. Exp. Med. 162, 501 (1985), Chizzonite et al., PNAS 86, 8029 (1989)), McMahan et al., EMBO J. 10, 2821 (1991) (II), Sims et al., Science 241, 585 (1988)) oder lösliche IL-2-Rezeptoren
(Taneguchi et al., Nature 302, 305 (1983), Greene et al., Ann. Rev. Immunol. 4, 69 (1986), Hatakeyama et al., Science 244, 551 (1989), Takeshita et al., Science 257, 379 (1992), Russel et al., Science 262, 1880 (1993))
oder IL-1-Rezeptorantagonisten (Eisenberg et al., Nature 343, 341 (1990), Carter et al., Nature 344, 633 (1990))
oder lösliche IL-6-Rezeptoren
(Mackiewicz et al., Cytokine 7, 142 (1995)) oder eine DNA-Sequenz für einen immunsuppressiven Antikörper oder dessen V_{H} und V_{L} enthaltende Fragmente oder dessen über einen Linker verbundene V_{H}- und V_{L}-Fragmente, hergestellt beispielsweise entsprechend der von beispielsweise entsprechend der von Marasco et al. (Proc. Natl. Acad. Sci. USA 90, 7889 (1993)) beschriebenen Methodik. Immunsuppressive Antikörper sind beispielsweise Antiörper spezifisch für den T-Zell-Rezeptor oder seinen CD3-Komplex, gegen CD4 oder CD8 des weiteren gegen den IL-2-Rezeptor (Strom et al., Ann. Rev. Med. 44, 343 (1993), Scheringer et al., Ann. Hematol. 66, 181 (1993)), IL-1-Rezeptor oder IL-4-Rezeptor oder gegen die Adhäsionsmoleküle CD2, LFA-1, CD28 oder CD40 (Olive et al., Drug Carriers Syst. 10, 29 (1993), Wendling et al., J. Rheumatol. 18, 325 (1991), Van der Lubbe et al., Arthritis Rheum. 34, 89 (1991)).
- c) Wirksubstanz zur Therapie von Antikörpermediierten Autoimmunerkrankungen: DNA-Sequenz für TGFβ (Massague, Ann. Rev. Cell Biol. 6, 597 (1990), Kondiah et al., J. Biol. Chem. 265, 1089 (1990), Garnier et al., J. Molec. Biol. 120, 97 (1978)) oder IFN α
(Henco et al., J. Mol. Biol. 185, 227 (1985), Pestka et al., Ann. Rev. Biochem. 56, 727 (1987), Weissmann et al., Phil. Trans. R. Soc. Lond. B299, 7 (1982), Goeddel et al., Nature 290, 20 (1981), (Sen et al., J. Biol. Chem. 267 5017 (1992), Mark et al. EP 192 811, EP 234 599, US 45 88 585)
oder IFNβ
(Sen et al., J. Biol. Chem. 267, 5017 (1992), Mark et al. EP 192 811, EP 234 599, US 45 88 585)
oder IFN-γ
(Gray et al., Nature 295, 503 (1982), Yip et al., PNAS USA 79, 1820 (1982), Rinderknecht et al., J. Biol. Chem. 259, 6790 (1984))
oder IL-12
(Kobayashi et al., J. Exp. Med. 170, 827 (1989), Gabler et al., PNAS 88, 4143 (1991), Gately et al., J. Immunol. 147, 874 (1991), Schoenhaut et al., J. Immunol. 148, 3433 (1992), Wolf et al., J. Immunol. 146, 3074 (1991))
oder lösliche IL-4-Rezeptoren
((Idzerda et al., J. Exp. Med. 171, 861 (1990), EPA 0419 091 A1, Foxwell, Eur. J. Immunol. 19, 1637 (1989). Garrone et al., Eur. J. Immunol. 21, 1365 (1991), Gallizzi et al., Int. Immunol. 2, 226 (1990), Park et al., J. Exp. Med. 166, 476 (1987)) oder lösliche IL-6-Rezeptoren
(Machiewicz et al., Cytokine 7, 142 (1995)) oder DNA-Sequenz für einen immunsuppressiven Antikörper (siehe Abschnitt 6.2.b) oder dessen V_{H}- und V_{L}-enthaltende Fragmente oder dessen über einen Linker verbunden V_{H}- und V_{L}-Fragmente, hergestellt beispielsweise entsprechend der von Marasco et al. (Proc. Natl. Acad. Sci. USA 90, 7889 (1993)) beschriebenen Methodik
- d) Wirksubstanz zur Therapie der Zellmediierten Autoimmunerkrankung: DNA-Sequenz für IL-6 (Wong et al., Immunol. Today 9, 137 (1988), Brakenhoff et al., J. Immunol. 143, 1175 (1989), Yasukawa et al., EMBO J. 6, 2939 (1987))
oder IL-9
(Yang et al., Blood 74, 1880 (1989), Mock et al, Immunogenetics 31, 265 (1990))
oder IL-10
(Moore et al., Science 248, 1230 (1990), Vieira et al., PNAS USA 88, 1172 (1991), Kim et al., J. Immunol. 148 3618 (1992))
oder IL-13
(McKenzie et al., PNAS 90, 3735 (1993), Minty et al., Nature 362, 248 (1993), McKenzie et al., J. Immunol. 150, 5436 (1993))
oder TNFα
(Beutler et al., Nature 320, 584 (1986), Kriegler et al., Cell 53, 45 (1988))
oder IL-4
(Lee et al., PNAS 83, 2061 (1986), Paul, Blood 77, 1859 (1991); Yokota et al., PNAS USA 83, 5894 (1986), von Leuven et al., Blood 73, 1142 (1989), Arai et al., J. Immunol. 142, 274 (1989))
oder TNFβ
(Gray et al., Nature 312, 721 (1984), Li et al., J. Immunol. 138, 4496 (1987), Aggarwal et al., J. Biol. Chem. 260, 2334 (1985)) oder
oder eine DNA-Sequenz für einen immunsuppressiven Antikörper oder dessen V_{H}- und V_{L}-enthaltende Fragmente dessen über einen Linker verbundene V_{H}- und V_{L}-Fragmente (siehe Abschnitt 6.2.b).

### Bei Wahl von Rezeptoren als Wirksubstanz sind deren extrazelluläre Teile zu verwenden.

Als Wirksubstanz im Sinne der Erfindung können jedoch auch DNA-Sequenzen von Fusionsproteinen zwischen den aufgeführten Cytokinen, Wachstumsfaktoren oder dem extrazellulären Teil der jeweiligen Rezeptoren zum einen und dem Fc-Teil des menschlichen Immunglobulin zum anderen Verwendung finden. Derartige DNA-Sequenzen und ihre Herstellung wurden in der EP 0464 533 A1 beschrieben.

### e) inhibierende Proteine

Als Wirksubstanz im Sinne der Erfindung ist jedoch auch ein Zellzyklusinhibitor zu verstehen. Ein Zellzyklusinhibitor im Sinne der Erfindung ist eine DNA-Sequenz, deren exprimiertes Protein die Proliferation von Zellen inhibiert. Zu diesen Zellzyklusinhibitoren gehören beispielsweise die DNA-Sequenzen für folgende Proteine:
- das Retinoblastomprotein (pRb=p110) oder die verwandten p107 und p130 Proteine (La Thangue, Curr. Opin. Cell Biol. 6, 443 (1994))
- das p53 Protein (Prives et al., Genes Dev. 7, 529 (1993))
- das p21 (WAF-1) Protein (EI-Deiry et al., Cell 75, 817 (1993))
- das p16 Protein (Serrano et al., Nature 366, 704 (1993), Kamb et al., Science 264, 436 (1994), Nobori et al., Nature 368, 753 (1994))
- andere cdK-Inhibitoren (Übersicht bei Pines, TIBS 19, 143 (1995))
- das GADD45 Protein (Papathanasiou et al., Mol. Cell. Biol. 11, 1009 (1991), Smith et al., Science 266, 1376 (1994))
- das bak Protein (Farrow et al., Nature 374, 731 (1995), Chittenden et al., Nature 374, 733 (1995), Kiefer et al., Nature 374, 736 (1995)).

Um eine schnelle intrazelluläre Inaktivierung dieser Zellzyklusinhibitoren zu verhindern, sind bevorzugt solche Gene zu verwenden, welche Mutationen für die Inaktivierungsstellen der exprimierten Proteine aufweisen, ohne daß diese hierdurch in ihrer Funktion beeinträchtigt werden.

Das Retinoblastomprotein (pRb/p110) und die verwandten p107 und p130 Proteine werden durch Phosphorylierung inaktiviert. Bevorzugt wird somit eine pRb/p110 -, p107 - oder p130 cDNA-Sequenz verwendet, die derart punktmutiert ist, daß die Phosphorylierungsstellen des kodierten Proteins gegen nicht phosphorylierbare Aminosäuren ausgetauscht sind.

Entsprechend Hamel et al. (Mol. Cell Biol. 12, 3431 (1992) wird die cDNA-Sequenz für das Retinoblastomprotein (p110) durch Austausch der Aminosäuren in den Positionen 246, 350, 601, 605, 780, 786, 787, 800 und 804 nicht mehr phosphorylierbar, seine Bindungsaktivität mit dem großen T-Antigen wird jedoch nicht beeinträchtigt. Beispielsweise werden die Aminosäuren Thr-246, Ser-601, Ser-605, Ser-780, Ser-786, Ser-787 und Ser-800 mit Ala, die Aminosäure Thr-350 mit Arg und die Aminosäure Ser-804 mit Glu ausgetauscht.

In analoger Weise wird die DNA-Sequenz für das p107 Protein oder das p130 Protein mutiert.

Das Protein p53 wird in der Zelle inaktiviert entweder durch Bindung an spezielle Proteine, wie beispielsweise MDM2 oder durch Oligomerisierung des p53 über das dephosphorylierte C-terminale Serin 392 (Schikawa et al., Leukemia und Lymphoma 11, 21 (1993) und Brown, Annals of Oncology 4, 623 (1993)). Bevorzugt wird somit eine DNA-Sequenz für ein p53 Protein verwendet, welches C-terminal verkürzt ist um das Serin 392.

### f) zytostatische oder zytotoxische Proteine

Als Zellzyklusinhibitor ist desweiteren eine DNA-Sequenz zu verstehen, die ein zytostatisches oder zytotoxisches Protein exprimiert.

Zu derartigen Proteinen zählen beispielsweise
- Perforin (Lin et al., Immunol. Today 16, 194 (1995))
- Granzym (Smyth et al., Immunol. Today 16, 202 (1995))
- TNF (Porter, TibTech 9, 158 (1991), Sidhu et al., Pharmc. Ther. 57, 79 (1993)), im speziellen
   * TNFα (Beutler et al., Nature 320, 584 (1986), Kriegler et al., Cell 53, 45 (1988)
   * TNFβ (Gray et al., Nature 312, 721 (1984), Li et al., J. Immunol. 138, 4496 (1987), Aggarwal et al., J. Biol. Chem. 260, 2334 (1985)

### g) Enzyme für die Aktivierung von Vorstufen von Zytostatika

Als Zellzyklusinhibitor ist jedoch auch die DNA-Sequenz für ein Enzym zu verstehen, welches eine inaktive Vorstufe eines Zytostatikums in ein Zytostatikum umwandelt.

Derartige Enzyme, welche inaktive Vorsubstanzen (Prodrugs) in aktive Zytostatika (Drugs) spalten und die jeweils zugehörigen Prodrugs und Drugs sind bereits von Deonarain et al. (Br. J. Cancer 70, 786 (1994), von Mullen, Pharmac. Ther. 63, 199 (1994) und Harris et al., Gene Ther. 1, 170 (1994)) übersichtlich beschrieben worden.

Beispielsweise ist die DNA-Sequenz folgender Enzyme zu verwenden:
- Herpes Simplex Virus Thymidinkinase
   (Garapin et al., PNAS USA 76, 3755 (1979), Vile et al., Cancer Res. 53, 3860 (1993), Wagner et al., PNAS USA 78, 1441 (1981), Moelten et al., Cancer Res. 46, 5276 (1986), J. Natl. Cancer Inst. 82, 297 (1990))
- Varizella Zoster Virus Thymidinkinase
   (Huber et al., PNAS USA 88, 8039 (1991), Snoeck, Int. J. Antimicrob. Agents 4, 211 (1994))
- bakterielle Nitroreduktase
   (Michael et al., FEMS Microbiol. Letters 124, 195 (1994), Bryant et al., J. Biol. Chem. 266, 4126 (1991), Watanabe et al., Nucleic Acids Res. 18, 1059 (1990))
- bakterielle β-Glucuronidase
   (Jefferson et al., PNAS USA 83, 8447 (1986))
- pflanzliche β-Glucuronidase aus Secale cereale
   (Schulz et al., Phytochemistry 26, 933 (1987))
- humane β-Glucuronidase
   (Bosslet et al., Br. J. Cancer 65, 234 (1992), Oshima et al., PNAS USA 84, 685 (1987))
- humane Carboxypeptidase (CB) z.B.
   * CB-A der Mastzelle
      (Reynolds et al., J. Clin. Invest. 89, 273 (1992))
   * CB-B des Pankreas
      (Yamamoto et al., J. Biol. Chem. 267, 2575 (1992), Catasus et al., J. Biol. Chem. 270, 6651 (1995))
- bakterielle Carboxypeptidase
   (Hamilton et al., J. Bacteriol. 174, 1626 (1992), Osterman et al., J. Protein Chem. 11, 561 (1992))
- bakterielle β-Laktamase
   (Rodrigues et al., Cancer Res. 55, 63 (1995), Hussain et al., J. Bacteriol. 164, 223 (1985), Coque et al., Embo J. 12, 631 (1993)
- bakterielle Cytosindeaminase
   (Mullen et al., PNAS USA 89, 33 (1992), Austin et al., Mol. Pharmac. 43, 380 (1993), Danielson et al., Mol. Microbiol. 6, 1335 (1992)
- humane Catalase bzw. Peroxidase
   (Ezurum et al., Nucl. Acids Res. 21, 1607 (1993))
- Phosphatase, im besonderen
   * humane alkalische Phosphatase
      (Gum et al., Cancer Res. 50, 1085 (1990))
   * humane saure Prostataphosphatase
      (Sharieff et al., Am. J. Hum. Gen. 49, 412 (1991), Song et al., Gene 129, 291 (1993), Tailor et al., Nucl. Acids Res. 18, 4928 (1990))
   * Typ 5 saure Phosphatase
      (Gene 130, 201 (1993))
- Oxidase, im besonderen
   * humane Lysyloxidase
      (Kimi et al., J. Biol. Chem. 270, 7176 (1995))
   * humane saure D-aminooxidase
      (Fukui et al., J. Biol. Chem. 267, 18631 (1992))
- Peroxidase, im besonderen
   * humane Gluthation Peroxidase
      (Chada et al., Genomics 6, 268 (1990), Ishida et al., Nucl. Acids Res. 15, 10051 (1987))
   * humane Eosinophilen-Peroxidase
      (Ten et al., J. Exp. Med. 169, 1757 (1989), Sahamaki et al., J. Biol. Chem. 264, 16828 (1989))
   * humane Schilddrüsen-Peroxidase
      (Kimura, PNAS USA 84, 5555 (1987)).

Zur Erleichterung der Sekretion der aufgeführten Enzyme kann die jeweils in der DNA-Sequenz enthaltende homologe Signalsequenz ersetzt werden durch eine heterologe, die extrazelluläre Ausschleusung verbessernde Signalsequenz.

So kann beispielsweise die Signalsequenz der β-Glucuronidase (DNA Position ≤ 27 bis 93; Oshima et al., PNAS 84, 685 (1987)) ersetzt werden durch die Signalfrequenz für das humane Immunglobulin (DNA Position ≤ 63 bis ≥ 107; Riechmann et al., Nature 332, 323 (1988).

Des weiteren sind bevorzugt DNAs solcher Enzyme zu wählen, welche durch Punktmutationen in einem geringeren Maße in Lysosomen gespeichert werden. Derartige Punktmutationen wurden beispielsweise für die β-Glucuronidase beschrieben (Shipley et al., J. Biol. Chem. 268, 12193 (1993)).

### 6.3. Kombination von gleichen oder unterschiedlichen Wirksubstanzen für Autoimmunerkrankungen u.a.

Gegenstand der Erfindung ist des weiteren ein Wirkstoff, in welchem eine Kombination der DNA-Sequenzen von mehreren gleichen Wirksubstanzen (A,A) oder unterschiedlichen Wirksubstanzen (A,B) vorliegt. Zur Expression z.B. von mehreren DNA-Sequenzen ist vorzugsweise die cDNA einer "internal libosome entry site" (IRES) als regulatorisches Element zwischengeschaltet. Derartige IRES wurden von Mountford und Smith (TIG 11, 179 (1995), Kaufman et al., Nucl. Acids Res. 19, 4485 (1991), Morgan et al., Nucl. Acids Res. 20, 1293 (1992) und Dirks et al., Gene 129, 247 (1993), Pelletier und Sonenberg, Nature 334, 320 (1988), Sugitomo et al., BioTechn. 12, 694 (1994) beschrieben.

Aktivator- | zellzyklus- | Wirksubstanz | internal | Wirksubstanz
sequenz | reguliertes | A | ribosome | A oder B
(UAS) | Promotor- | | entry |
| modul | | site |

Ein derartiger Wirkstoff weist je nach Kombination additive oder synergistische Wirkung im Sinne der Erfindung auf.

### 6.4. Auswahl des Liganden für Autoimmunerkrankungen u.a.

Als Ligand für virale und nicht-virale Vektoren, beispielsweise in kolloidalen Dispersionen hergestellt mit Polylysin-Ligand-Konjugaten, werden Substanzen bevorzugt, welche an die Oberfläche von Immunzellen (Makrophagen, Lymphozyten) spezifisch binden. Hierzu gehören Antikörper oder Antikörperfragmente gerichtet gegen Membranstrukturen von Immunzellen, wie sie beispielsweise von Powelson et al., Biotech. Adv. 11, 725 (1993) beschrieben wurden.

Des weiteren gehören zu den Liganden auch monoklonale oder polyklonale Antikörper oder Antikörperfragmente, die mit ihren konstanten Domänen an Fc-γ- oder -Rezeptoren von Immunzellen binden (Rojanasakul et al., Pharm. Res. 11, 1731 (1994)).

Die murinen monoklonalen Antikörper sind bevorzugt in humanisierter Form einzusetzen. Die Humanisierung erfolgt in der von Winter et al. (Nature 349, 293 (1991)) und Hoogenbooms et al. (Rev. Tr. Transfus. Hemobiol. 36, 19 (1993)) dargestellten Weise. Antikörperfragmente werden entsprechend dem Stand der Technik hergestellt, beispielsweise in der von Winter et al. (Nature 349, 293 (1991), Hoogenboom et al. (Rev. Tr. Transfus. Hemobiol. 36, 19 (1993), Girol (Mol. Immunol. 28, 1379 (1991) und Huston et al. (Int. Rev. Immunol. 10, 195 (1993) beschriebenen Weise.

Zu den Liganden gehören des weiteren alle Substanzen, welche an Membranstrukturen oder Membranrezeptoren auf der Oberfläche von Immunzellen binden. Beispielsweise gehören hierzu Wachstumsfaktoren, wie Zytokine, EGF, TGF, FGF oder PDGF, oder deren Fragmente bzw. Teilsequenzen von ihnen, die an Rezeptoren exprimiert durch derartige Zellen binden.

Hierzu gehören des weiteren Liganden, welche an Zellmembranstrukturen, wie beispielsweise den Mannose 6-Phosphat-Rezeptor auf Makrophagen in Milz, Leber, Lunge und andere Gewebe binden.

Diese Liganden und Membranstrukturen sind übersichtlich bei Perales et al., Eur. J. Biochem. 226, 255 (1994) beschrieben.

### 6.5. Herstellung des Wirkstoffes für Autoimmunerkrankungen u.a.

Die Herstellung des erfindungsgemäßen Wirkstoffes wird anhand folgender Beispiele näher beschrieben:

### a) Konstruktion des chimären Promotors IL-2-CDE-CHR-lnr

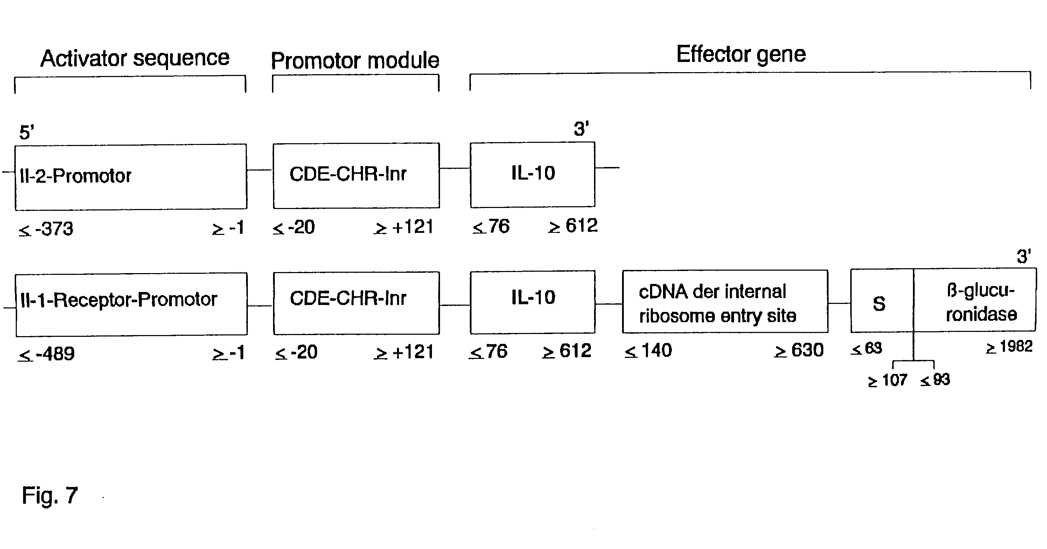

Der humane IL-2 Promotor (Position ≤ -373 bis ≥ -1, Wiliams et al., J. Immunol. 141, 662 (1988)) wird an seinem 3' Ende mit dem 5'-Terminus des CDE-CHR-Inr Moduls (Position ≤ -20 bis ≥ + 121) des humanen cdc25C-Gens (Lucibello et al., EMBO J., 14, 132 (1995)) verknüpft (Fig. 7). Die Verknüpfung erfolgt mit Hilfe von dem Fachmann bekannten und käuflichen Enzymen.

### b) Konstruktion eines Plasmids enthaltend den chimären Promotor IL-2-CDE-CHR-lnr im zentralen Bestandteil des Wirkstoffes

Die beschriebene chimäre IL-2 Repressormodul-Transkriptionseinheit wird an ihren 3' Enden mit dem 5'-Terminus einer DNA, die den kompletten kodierenden Bereich des IL-10 (Position ≤ 76 bis ≥ 612, Moore et al., Science 248, 1230 (1990)) enthält, verknüpft (Fig. 7). Diese DNA enthält auch die für eine Sekretion notwendige Signalsequenz. Transkriptionskontrolleinheiten und die DNA für IL-10 werden in pUC19/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### c) Konstruktion eines Plasmids enthaltend zwei Gene für Wirksubstanzen

Der humane IL-1-Rezeptor Promotor (Pos. ≥ -489 bis ≥ -1, Ye et al., PNAS USA 90, 229 (1993)) wird an seinem 3' Ende mit dem 5'-Terminus des CDE-CHR-Inr Moduls des humanen cdc25C-Gens (Pos. -20 bis +121 (Lucibello et al., EMBO J. 14, 132 (1995)) verknüpft (siehe Fig. 7). Die Verknüpfung erfolgt mit Hilfe von dem Fachmann bekannten und käuflichen Enzymen.

Die so hergestellte chimäre IL-1-Rezeptor-Repressormodul-Transkriptionskontrolleinheit wird an ihrem 3' Ende mit dem 5'-Terminus einer DNA, die den kompletten kodierenden Bereich des IL-10 enthält, verknüpft (siehe Fig. 7). Diese DNA enthält auch die für eine Sekretion notwendige Signalsequenz.

Das 3' Ende der DNA für IL-10 wird nunmehr verknüpft mit dem 5' Ende der cDNA der internal ribosome entry site (Position ≤ 140 bis ≥ 630; Pelletier und Sonnenberg, Nature 334, 320 (1988)) und anschließend wird deren 3' Ende mit dem 5' Ende der DNA für die Signalsequenz des Immunglobulin verknüpft (Position ≤ 63 bis ≥ 107, Riechmann et al., Nature 332, 323 (1988)). An derem 3' Ende wird das 5' der DNA für β-Glucuronidase verknüpft (Position ≤ 93 bis ≥ -1982, cDNA Sequenz ohne Signalsequenz, Oshima et al., PNAS USA 84, 685 (1985)). Dieser so hergestellte Wirkstoff wird anschließend in puc18/19 oder in Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### 7) Wirkstoff zur Behandlung der Arthritis

### 7.1. Auswahl der Aktivatorsequenz für Arthritis

Als Aktivatorsequenz ist eine Nukleotidsequenz (Promotor- oder Enhancersequenz) zu verstehen, mit der Transkriptionsfaktoren gebildet oder aktiv in Synovialzellen und Entzündungszellen interagieren. Im Sinne dieser Erfindung zählen zu den bevorzugten Aktivatorsequenzen genregulatorische Sequenzen bzw. Elemente aus Genen, die für besonders in Synovialzellen und Entzündungszellen exprimierte Proteine kodieren. Dieses sind beispielsweise:
- Metalloproteinasen (MMP) (Kollagenasen, Gelatinasen, Strome lysin)
   im speziellen
   * MMP-1 (interstitielle Kollagenase)
      (Lewis et al., Int. J. Immunopharm. 14, 497 (1992))
   * MMP-2 (72 kD Gelatinase)
      (Okada et al., Eur. J. Biochem. 194, 721 (1990))
   * MMP-3 (Stromelysin)
      (Saus et al., J. Biol. Chem. 263, 6742 (1988), Tetlow et al., Rheum. Internat. 13, 53 (1993))
   * MMP-9 (92 kD Gelatinase)
      (Tetlow et al., Rheum. Internat. 13, 53 (1993))
   Promotorsequenzen für die Metalloproteinasen wurden beispielsweise wie folgt publiziert:
   * MMP-1 (interstitielle Kollagenase)
      (Angel et al., Mol. Cell. Biol. 7, 2256 (1987))
   * MMP-3 (Stromelysin/Transin)
      (Matrisian et al., Mol. Cell. Biol. 6, 1679 (1986), Kerr et al., Cell 61, 267 (1999)
- Tissue inhibitors of Metalloproteinasen (TIMP)
   im speziellen
   * TIMP-1
      (Kolkenbrock et al., Eur. J. Biochem. 198, 775 (1991), Faucher et al., Path. Biol. 31, 199 (1989))
   * TIMP-2
      (Kolkenbrock et al., Eur. J. Biochem. 198, 775 (1991))
   * TIMP-3
      (Wick et al., J. Biol. Chemistry 269, 18953 (1994)).
   Die Promotorsequenzen für TIMPs wurden wie folgt publiziert:
   * TIMP-1 (Stearns et al., Proc. Annu. Meet. Am. Assoc. Cancer Res. 33, A131 (1992))
   * TIMP-2 (De Clerck et al., Gene 139, 185 (1994))
   * TIMP-3:
   Gegenstand der Erfindung ist des weiteren die 500 Basenpaare umfassende Promotorsequenz für das von Wick et al. (J. Biol. Chemistry 269, 18953 (1994)) beschriebene TIMP-3 Gen. Diese Promotorsequenz besteht u.a. aus den Bindungsstellen für die Transkriptionsfaktoren NF-1 (Mcisterernst et al., Nucl. Acids Res. 16, 4419 (1988), Santoro et al., Nature 334, 218 (1988)), Sp1 (Kadonaga et al., TIBS 11, 10 (1986)) und C/EBP (Cao et al., Genes Dev. 5, 1538 (1991), Landschulz et al., Science 243, 1681 (1989)) und flankiert die transkribierte TIMP-3 Gensequenz am 5' Ende.

### 7.1.1. Charakterisierung der humanen TIMP-3-Promotorsequenz

### a) Isolierung und Sequenzanalyse der 5'-flankierenden

### Promotorsequenz des humanen TIMP-3-Gens

Die Induktion der TIMP-3-mRNA-Expression während der G₀→S-Progression beruht hauptsächlich auf einer Aktivierung der Transkription des TIMP-3-Gens (Wick et al., J. Biol. Chem. 269, 18963 (1994)). Die 5'-flankierende Sequenz des menschlichen TIMP-3-Gens wurde kloniert, der Startpunkt der Transkription der TIMP-3-mRNA bestimmt und der angrenzende Promotorbereich einer Struktur-Funktions-Analyse unterzogen. Diese Untersuchungen sollten die Regulationsmechanismen klären, die der spezifischen TIMP-3-Expression während der G₀→S- und G₁→S-Progression zugrundeliegen.

Durch eine genomische Southern Blot-Analyse wurde vorab bestimmt, ob TIMP-3 im menschlichen Genom ein Einzelgen darstellt oder mehrere Loci für das TIMP-3-Gen bzw. eventuell auch TIMP-3-Pseudogene existieren. Hierzu wurde genomische DNA aus WI-38-Zellen isoliert, mit den Restriktionsendonukleasen EcoRI, Pstl und Hindlll behandelt und einer Southern Blot-Analyse unterzogen. Als radioaktiv-markierte Sonde wurde ein 690 bp langes 3'-TIMP-3-cDNA-Fragment eingesetzt. Da die Sonde in allen Fällen nur ein spezifisches DNA-Fragment erkannte, ist davon auszugehen, daß nur ein singuläres TIMP-3-Gen im menschlichen Genom vorliegt.

Zur Isolierung der 5'-flankierenden TIMP-3-Gensequenz wurden ca. 7x10⁵ Phagen einer genomischen WI-38-Genbibliothek mit einem 300 bp langen 5'-TIMP-3-cDNA-Fragment hybridisiert. Von den dreizehn nach dieser Primäruntersuchung isolierten rekombinanten Phagenklonen wurden vier auch von einem 30 bp langen Oligonukleotid aus dem 5'-Endbereich der TIMP-3-cDNA erkannt. Da diese Phagen wahrscheinlich auch den das ATG-Startcodon flankierenden, 5'-Sequenzbereich enthielten, wurde einer der Phagenklone für eine nähere Charakterisierung und Analyse ausgewählt. Durch kombinierte Behandlung mit verschiedenen Restriktionsendonukleasen und nachfolgender Southern Blot-Analyse konnte bestimmt werden, daß das 13 kb lange genomische DNA-"Insert" dieses Phagen ca. 4,7 kb der 5'-flankierenden TIMP-3-Gensequenz enthielt.

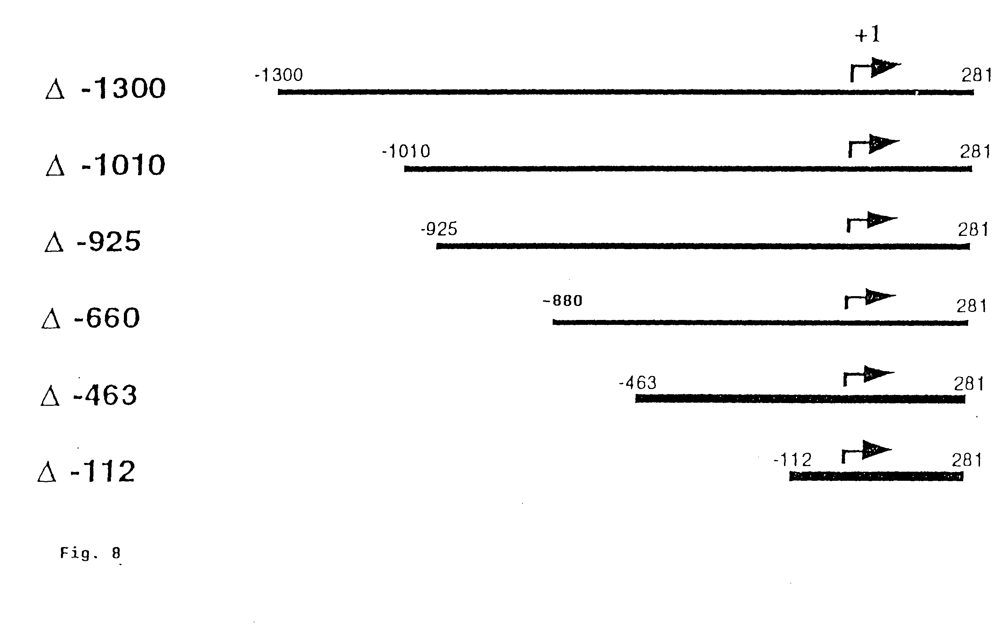

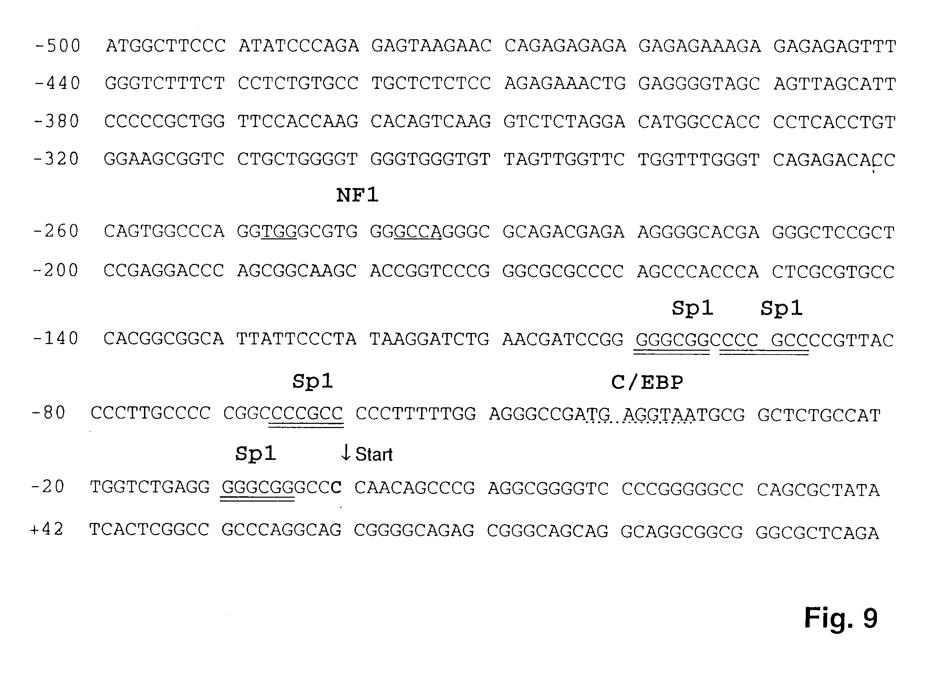

Durch Sequenzanalyse beider Stränge wurde die Nukleotidsequenz von annähernd 1500 bp des 5'-flankierenden Genbereichs bestimmt. Die hierfür durch eine Exonuklease III-Behandlung hergestellten 5'-Verkürzungen des klonierten 5'-Genbereichs sind in Fig. 8 illustriert.
Der Sequenzbereich, der sich durch die nachfolgend beschriebenen Struktur-Funktions-Analysen als besonders bedeutend für die TIMP-3-Promotor-Funktion herausstellte, ist in Fig. 9 gezeigt. Durch Computergestützte Analyse wurden in der TIMP-3-Promotorsequenz eine Reihe von Elementen identifiziert, die den Bindungsstellen bekannter Transkriptionsfaktoren ähneln, u.a. 4 Sp1-Bindungsstellen, eine mögliche NF1- sowie eine C/EBP-Bindungsstelle (markiert in Fig. 9).

### b) Kartierung des Transkriptionsstartpunktes der TIMP-3-mRNA

Um den oder die Startpunkt(e) der Transkriptionsinitiation zu ermitteln, wurde das 5'-Ende der TIMP-3-mRNA durch eine Primer-Extension-Analyse bestimmt. Hierbei wurde eine Transkriptionsstartstelle (Nucleotidsequenz: GGGCGGGCCCAACAGCCCG) identifiziert, die 364 bp 5' vom ATG-Startcodon lokalisiert ist (markiert in Fig. 9). Trotz genauer Untersuchung der aufwärts der Startstelle gelegenen Nukleotidsequenz wurde weder eine TATA-Box noch TATA-ähnliche Sequenzen gefunden.

### c) Untersuchungen zur Aktivität der TIMP-3-Promotorsequenz

Um die Aktivität der TIMP-3-Promotorsequenz in normal proliferierende, ruhende und serumstimulierende Zellen zu bestimmen und erste Hinweise auf funktionell wichtige Promotorbereiche zu enthalten, wurden die zur Sequenzierung verwendeten 5'-verkürzten Promotorfragmente (s. Fig. 8) vor das Luziferasegen in den promotorlosen pXP-2-Vektor (Nordeen, Biotechniques 6, 454 (1988)) kloniert. Durch seine äußerst geringe Basalaktivität eignet sich dieses "Reporterkonstrukt" besonders zur Durchführung transienter Expressionsanalysen.

### d) Aktivität der TIMP-3-Promotorsequenz in normal proliferierenden und serumstimulierten NIH3T3-Zellen

Um zu zeigen, daß die isolierte TIMP-3-Promotorsequenz in transienten Expressionsanalysen aktiv ist, d.h. die Transkription des Luziferase-Reportergens steuern kann, wurde das TIMP-3-Promotor-Deletionskonstrukt Δ-1010 (umfaßt die Nukleotide -1010 bis +281, s. Fig. 8) in NIH3T3-Zellen transfiziert und die Luziferase-Aktivität in diesen normal proliferierenden oder serumstimulierten transfizierten Zellen bestimmt. Zum Vergleich wurde zusätzlich die Expression von weiteren Luziferase-Promotorkonstrukten ermittelt, die den Herpes Virus tk-Promotor (pT81; Lucibello und Müller, Meth. Mol. Cell Biol. 1, 9 (1989)), einen 5xTRE-Minimalpromotor (Angel et al., Mol. Cell Biol. 7, 2256 (1987)), einen RSV-LTR (Setoyama et al., Proc. Natl. Acad. Sci. USA 83, 3213 (1986) oder ein 937 bp langes Fragment des menschlichen Cyclin D1-Promotors (Herber et al., Oncogene 9, 1295 (1994)) enthielten. Die Ergebnisse dieser Untersuchungen sind in Tabelle 2 aufgeführt.

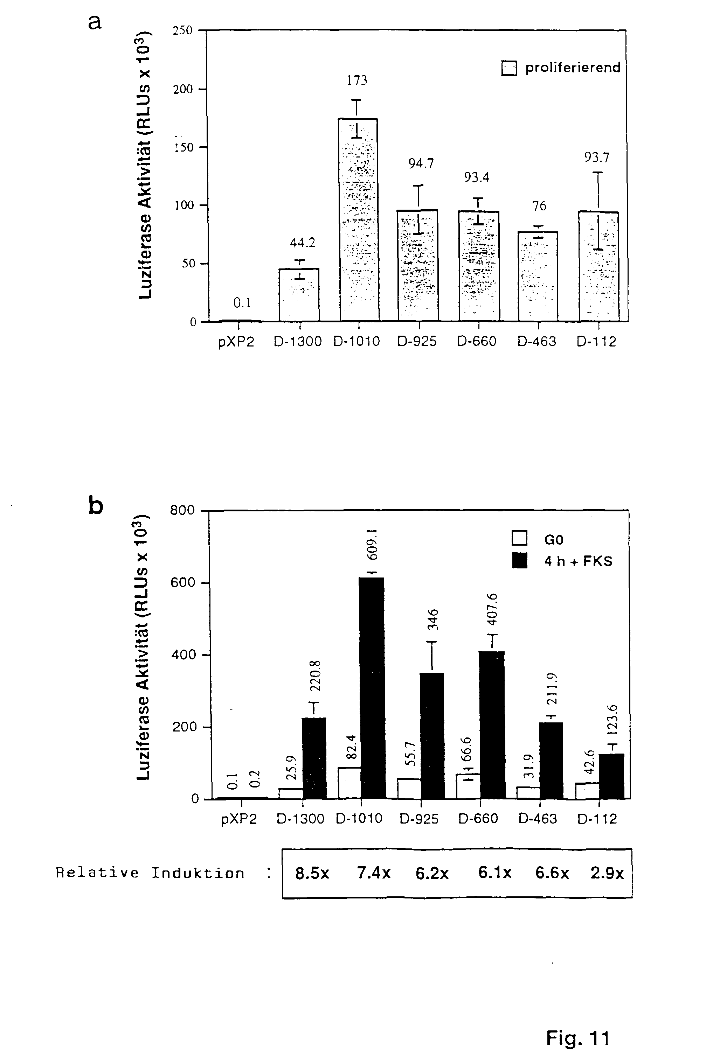

In normal proliferierenden NIH3T3-Zellen (Tab. 2A) wurde das TIMP-3-Promotorkonstrukt Δ-1010 ca. 3-fach höher exprimiert als der 5xTRE-Minimalpromotor und zeigt eine 7-fach höhere Expression als das Cyclin D1-Promotorkonstrukt. Einzig das RSV-LTR-Reporterplasmid wies eine ca. 2-fach höhere Aktivität als das TIMP-3-Promotorkonstrukt auf. Diese Ergebnisse deuten darauf hin, daß der menschliche TIMP-3-Promotor durch eine vergleichsweise hohe transkriptionelle Aktivität gekennzeichnet ist. Wie in Tab. 2B und Fig. 11 gezeigt, wurde das TIMP-3-Promotor-konstrukt Δ-1010 außerdem deutlich in Zellen induziert, die nach zweitägigem Serumentzug für 4 h mit 20% FKS stimuliert worden waren. Verglichen mit ruhenden (G₀) Zellen stieg die Expression dabei ca. 7- bis 8-fach an, was etwa 3,5-fach bzw. 2,4-fach höher lag als die beobachteten Induktionswerte des 5xTRE-Reporterkonstruktes bzw. des Cyclin D1-Promotorkonstrukts. Dagegen zeigte das Herpes simplex tk-Promotor-Luziferasekonstrukt (pT81) keine Induktion seiner Expression nach Serumstimulation.

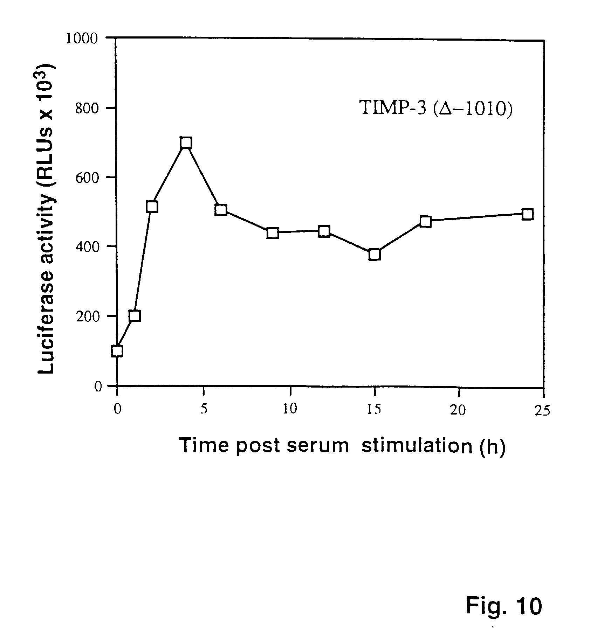

In Fig. 10 ist die Kinetik der Induktion des Δ-1010 TIMP-3-Promotor-konstrukts nach Serumstimulation ruhender Zellen gezeigt. Die Luziferase-Aktivität stieg bereits nach 1 h an und erreichte mit einer 7-fachen Induktion nach 4 h Maximalwerte.

Zusammenfassend zeigen diese Ergebnisse, daß das verwendete Δ-1010 TIMP-3-Promotorkonstrukt die wesentlichen, wenn nicht alle regulatorischen Elemente aufweist, die für eine effiziente Transkription sowie für die Induzierbarkeit durch Serum benötigt werden.

### e) Struktur- und Funktionsanalyse der TIMP-3-Promotorsequenz

Eine Struktur- und Funktionsanalyse der isolierten TIMP-3-Promotorsequenz sollte erste Hinweise auf diejenigen Promotorregionen liefern, die für die Basalexpression sowie die Seruminduzierbarkeit funktionell bedeutend sind. Hierzu wurde die Aktivität der verschiedenen, für die Promotorsequenzierung hergestellten und in den pXP-2-Vektor umklonierten, TIMP-3-Promotor-Deletionskonstrukte (s. Fig. 8) in transienten Expressionsanalysen bestimmt. Die Analyse der basalen Expression der verschiedenen Deletionskonstrukte in normal proliferierenden NIH3T3-Zellen (Fig. 11) ergab drei wesentliche Ergebnisse:
1. Die stärkste Expression wies das Promotorkonstrukt Δ-1010 auf. Eine Verkürzung um weitere 85 bp (Konstrukt Δ-925) führte zu einem annähernd 2-fachen Absinken der Promotoraktivität. Dies deutet auf die Präsenz von einem oder mehreren Elementen in der Region zwischen Position -1010 und -925 hin, die an der Transkriptionsaktivierung beteiligt sind.
2. Durch weitere Verkürzungen des 5'-Endes bis hin zu Position -112 wurde die Promotoraktivität nicht signifikant beeinflußt. Die Region zwischen -925 und -112 enthält daher wahrscheinlich keine für die Promotoraktivität wichtigen Sequenzbereiche.
3. Die Region zwischen Position -1300 und -1010 scheint dagegen einen negativen Effekt auf die Prornotoraktivität auszuüben, was sich in der im Vergleich zum Promotorkonstrukt Δ-1010 ca. 4-fach reduzierten Expression des Δ-1300 Deletionskonstrukts ausdrückt.

Im abschließenden Experiment wurde die Seruminduzierbarkeit der verschiedenen TIMP-3-Promotor-Deletionskonstrukte analysiert. Die Ergebnisse dieser, wie in Tabelle 2 beschriebenen durchgeführten, Expressionsanalysen sind in Fig. 11b dargestellt. Auffällig ist das ähnliche Expressionsprofil zwischen normal proliferierenden (Fig. 11a), ruhenden und serumstimulierten Zellen (Fig. 11b). Die Expressionswerte in ruhenden Zellen lagen aber ca. 2-fach niedriger als in proliferierenden Zellen und wurden 4 h nach Serumstimulation 2,9- bis 8,5-fach induziert. Wie in den proliferierenden Zellen gezeigt (Fig. 11a), übt die Region zwischen Position Δ-1300 und Δ-1010 einen negativen Effekt auf die Promotoraktivität in ruhenden und serumstimulierten Zellen aus, hat aber keinen Einfluß auf die Seruminduzierbarkeit des Konstruktes Δ-1300 (8,5-fache Induktion). Auch hier wurden die höchsten Luziferaseaktivitäten wieder mit dem Deletionskonstrukt -1010 gemessen.

Weitere Verkürzungen des 5'-Endes bis zu Position -660 führten nur zu einem 1,5- bis 2-fachen Absinken der Promotoraktivität. Alle diese Konstrukte (Δ-1300, Δ-1010, Δ-925, Δ-660) zeigten aber eine deutliche, 6-bis 8-fache Induktion nach Serumzugabe. Die Verkürzung um weitere 200 bp bis zu Position -463 (Δ-463) bewirkte ein erneutes 2-faches Absinken der Aktivität, hatte aber ebenfalls keinen Einfluß auf die Seruminduzierbarkeit des Konstrukts. Erst das Konstrukt Δ-112 zeigte mit einem nur 3-fachen Expressionsanstieg nach Serumstimulation eine 50-65%ige Reduktion seiner Seruminduzierbarkeit. Dies deutet darauf hin, daß die Region zwischen Position -463 und -112 Element(e) enhält, die für die Seruminduzierbarkeit des TIMP-3-Promotors von Bedeutung sind. Zusätzliche Regionen zwischen Position -463 und -660 sowie -925 und-1010 verstärken generell und zellzyklusunabhängig die seruminduzierte Promotoraktivität.

Die Ergebnisse der Charakterisierung sowie Struktur- und Funktionsanalyse des 5'-flankierenden TIMP-3-Genbereiches lassen sich wie folgt zusammenfassen:

TIMP-3 stellt ein TAT-Box-loses Gen dar. Die Transkription wird aber dennoch an nur einer Startstelle 364 bp aufwärt vom ATG-Startcodon initiiert. Verglichen mit anderen Promotoren weist die TIMP-3-Promotorsequenz eine relativ hohe Aktivität auf, für die die ersten 112 bp ausreichend sind. In dieser Region befinden sich zahlreiche Sp1-Bindungsstellen. Außerdem zeigt er eine deutliche Induktion seiner Aktivtät nach Serumstimulation ruhender Zellen, deren Kinetik der TIMP-3-mRNA-Expression während der G₀-S-Progression entspricht. Die für die Seruminduzierbarkeit verantwortlichen Regulationselemente sind in der Region zwischen Position -112 und -463 lokalisiert.

Aktivatorsequenzen im Sinne dieser Erfindung sind des weiteren Promotoren für den
- GM-CSF-Rezeptor
   (Nakagawa et al., J. Biol. Chem. 269, 10905 (1994))
- Makrophagen-Colony Stimulating Factor (M-CSF)-Rezeptor
   (Yue et al., Mol. Cell. Biol. 13, 3191 (1993), Zhang et al., Mol. Cell. Biol. 14, 373 (1994))
- Typ I und II Makrophagen Scavenger Rezeptoren
   (Mouton et al., Mol. Cell. Biol. 14, 4408 (1994))

### 7.2. Auswahl der Wirksubstanz für Arthritis

Als Wirksubstanz im Sinne der Erfindung ist eine DNA-Sequenz zu verstehen, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert. Zu diesen Proteinen zählen beispielsweise folgende Proteine (die DNA-Sequenz für das jeweilige Protein ist den angegebenen Literaturstellen zu entnehmen):
- IL-1-Rezeptorantagonist (IL-1-RA)
   (Thompson et al. (1992), Eisenberg et al., Nature 343, 341 (1990), Carter et al., Nature 344, 63 (1990))
   IL-1-RA inhibiert die Bindung von IL-1∝,β an den spezifischen Rezeptor (Conti et al., (1992), Granowietz et al., (1992)), IL-1 aktiviert Synovialzellen und ist hierdurch entzündungsfördernd (Dayer et al., Eur. Cytokine Network 5/6, 563 (1994))
- löslicher IL-1-Rezeptor
   (Sims et al., Clin. Immun. Immunopath. 72, 9 (1994), Sims et al., Nature 35, 88 (1988), Sims et al., PNAS USA 86, 8946 (1989) (I), Dower et al., J. Exp. Med. 162, 501 (1985), Chizzonite et al., PNAS 86, 8029 (1989), McMahan et al., EMBO J. 10, 2821 (1991) (II), Sims et al., Science 241, 585 (1988))
   Löslicher IL-1-Rezeptor bindet und inaktiviert IL-1 (Fanslow et al., Science 248, 739 (1990), Jacobs et al., J. Immunol. 146, 2983 (1991))
- IL-6
   (Hirano, Int. J. Cell Cloning 9, 166 (1991), Brach et al., Int. J. Clin Lab. Rec. 22, 143 (1992), Wong et al., Immunol. Today 9, 137 (1988), Brakenhoff et al., J. Immunol. 143, 1175 (1989), Yasukawa et al, EMBO J. 6, 2939 (1987))
   IL-6 erhöht die Sekretion von TIMP und Superoxiden und vermindert die Sekretion von IL-1 und TNF∝ durch Synovialzellen und Chondrozyten (Shingu et al., Clin. Exp. Immunol. 94, 145 (1993), Shingu et al., Inflammation 18, 613 (1994)).
- löslicher TNF-Rezeptor
   (Olson et al., Eur. Cytokine Network 4, 169 (1993), Tartaglia et al., Immunol. Today 13, 151 (1992), Nophar et al., EMBO J. 9, 3269 (1990), Himmler et al., DNA Cell Biol. 9, 705 (1990), Aggarwal et al., Nature 318, 665 (1985), Gray et al., PNAS 87, 7380 (1990), Tartaglia et al., Immunol. Today 13, 151 (1992), Loetcher et al., Cell 61, 351 (1990), Schall et al., Cell 61, 361 (1990), Smith et al., Science 248, 1019 (1990), Goodwin et al., Mol. Cell. Biol. 11, 3020 (1991))
   Löslicher TNF-Rezeptor bindet und inaktiviert TNF. TNF aktiviert Synovialzellen zur erhöhten Sekretion von Metalloproteinasen (Dayer et al., Eur. Cytokine Network 5/6, 563, 1994))
- IL-4
   (Paul, J. Am. Soc. Hemat. 77, 1859 (1991), Yokota et al., PNAS USA 83, 5894 (1986), Paul, Blood 77, 1859 (1991), von Leuven et al., Blood 73, 1142 (1989), Arai et al., J. Immunol. 142, 274 (1989))
   IL-4 inhibiert die Bildung und Sekretion von IL-1, TNF∝ und MMP (Corcoran et al., J. Biol. Chemistry 267, 515 (1992), Dayer et al., Eur. Cytokine Network 5/6, 563 (1994), te Velde et al., Blood 76, 1392 (1990))
- IL-10
   (Moore et al., Science 248, 1230 (1990), Vieira et al., PNAS USA 88, 1172 (1991), Kim et al., J. Immunol. 148, 3618(1992))
   IL-10 inhibiert die Bildung und Sekretion von IL-1, TNFα und MMP und erhöht die Sekretion von TIMP (Dayer et al., Eur. Cytokine Network 5/6, 563 (1994))
- Insulin-like growth factor (IGF-1)
   (Jansen et al., Nature 306, 609 (1983), Ullrich et al., EMBO J. 3, 361 (1984), Bell et al., PNAS 82, 6450 (1985), Rotwein et al., PNAS 83, 77 (1986), J. Biol. Chem. 261, 4828 (1986), Jansen et al., FEBS Lett. 179, 243 (1985))Tobin et al., Mol. Endocrin. 4, 1914 (1990), Macaulay, Brit. J. Cancer 65, 311 (1992))
   IGF-1 stimuliert die Synthese von extrazellulärer Matrix.
- TGFβ
   im speziellen
   * TGFβ1 und TGFβ2
      (Massague, Ann. Rev. Cell. Biol. 6, 597 (1990), Kondiah et al., J. Biol. Chem. 265, 1089 (1990), Garnier et al., J. Molec. Biol. 120, 97 (1978), Wahl et al., Immunol. Today 10, 258 (1989), Dupuy D'Angeac et al., J. Cell Physiol. 147, 460 (1991))
      TGFβ stimuliert die Synthese von extrazellulärer Matrix.
- Superoxiddismutase (Folz et al., Genomics 22, 162 (1994), Wan et al. 13/11, 1127 (1994))
- TIMP (Tissue Inhibitors of Metalloproteinases)
   im speziellen
   * TIMP-1 (Docherty et al., Nature 318, 66 (1985))
   * TIMP-2 (Stetler-Stevenson et al., J. Biol. Chem. 265, 13933 (1990))
   * TIMP-3 (Wick et al., J. Biol. Chemistry, 269, 18953 (1994))

Als Wirksubstanz im Sinne der Erfindung können jedoch auch DNA-Sequenzen von Fusionsproteinen zwischen den aufgeführten Cytokinen, Wachstumsfaktoren oder dem extralzelluären Teil der Rezeptoren zum einen und dem Fc-Teil des menschlichen Immunglobulins zum anderen Verwendung finden. Derartige cDNA-Sequenzen und ihre Herstellung wurden in der EPA 0464 633 A1 beschrieben.

### 7.3. Kombination gleicher oder unterschiedlicher Wirksubstanzen für Arthritis

Gegenstand der Erfindung ist des weiteren ein Wirkstoff, in welchem eine Kombination der DNA-Sequenzen von mehreren gleichen antientzündlichen Substanzen (A,A) oder von unterschiedlichen antientzündlichen Substanzen (A,B) vorliegt. Zur Expression von zwei DNA-Sequenzen wird vorzugsweise die cDNA einer "internal ribosome entry site" (IRES) als regulatorisches Element zwischengeschaltet.

Aktivator- | zellzyklus- | antientzünd- | internal | antienzünd-
sequenz | reguliertes | liche | ribosome | liche
(UAS) | Repressor- | Substanz | entry | Substanz
 | modul | A | site | A oder B

Derartige IRES wurden beispielsweise von Mountford und Smith (TIG 11, 179 (1995), Kaufman et al., Nucl. Acids, Res. 19, 4485 (1991), Morgan et al., Nucl. Acids Res. 20, 1293 (1992), Dirks et al., Gene 128, 247 (1993), Pelletier und Sonenberg, Nature 334, 320 (1988) und Sugitomo et al., BioTechn. 12, 694 (1994)) beschrieben.

So kann die cDNA der IRES-Sequenz des Poliovirus (Position ≤ 140 bis ≥ 630 des 5' UTR (Pelletier und Sonenberg, Nature 334, 320 (1988)) zur Verknüpfung der DNA der antientzündlichen Substanz A (am 3' Ende) mit der DNA der antientzündlichen Substanz B (am 5' Terminus) verwendet werden.

Ein derartiger Wirkstoff weist, je nach Kombination, additive (A+A, A+B₁) oder synergistische Wirkung im Sinne der Erfindung auf.

### 7.4. Auswahl des Liganden für Arthritis

Als Ligand für virale und nicht-virale Vektoren, beispielsweise in Polylysin-Ligand-Konjugaten, werden Substanzen bevorzugt, welche an die Oberfläche von Synovialzellen binden. Hierzu gehören monoklonale oder polyklonale Antikörper oder Antikörperfragmente, die mit ihren variablen Domänen an Membranstrukturen von Synovialzellen oder Entzündungszellen binden, welche beispielsweise sind
- Vimentin (Miettinen et la., Am. J. Pathol. 117, 18 (1984))
- Fibronectin (Wojciak et al., Clin. Exp. Immunol. 93, 108 (1993))

Hierzu gehören auch monoklonale oder polyklonale Antikörper oder Antikörperfragmente, die mit ihren konstanten Domänen an Fc-Rezeptor binden (Rojanasakul et al., Pharm. Res. 11, 1731 (1994)).

Die murinen monoklonalen Antikörper sind bevorzugt in humanisierter Form einzusetzen. Die Humanisierung erfolgt in der von Winter et al. (Nature 349, 293 (1991) und Hoogenbooms et al. (Rev. Tr. Transfus. Hemobiol. 36, 19 (1993) dargestellten Weise. Antikörperfragmente werden gemäß dem Stand der Technik hergestellt, beispielsweise in der von Winter et al., Nature 349, 293 (1991), Hoogenboom et al., Rev. Tr. Transfus. Hemobiol. 36, 19 (1993), Girol, Mol. Immunol. 28, 1379 (1991) oder Huston et al., Int. Rev. Immunol. 10, 195 (1993) beschriebenen Weise.

Hierzu gehören des weiteren alle Wirkstoffe, welche an Membranstrukturen oder Membranrezeptoren auf Synovialzellen binden. Beispielsweise gehören hierzu Cytokine oder Wachstumsfaktoren oder deren Fragmente bzw. Teilsequenzen von ihnen, die an Rezeptoren exprimiert durch Synovialzellen binden wie beispielsweise IL-1-RA, TNFα, IL-4 IL-6, IL-10, IGF, TGFβ.

Desweiteren gehören hierzu Liganden, deren wesentlicher Bestandteil endständige Mannose ist, welche an Mannose-6-phosphatrezeptoren auf Makrophagen bindet (Perales et al., Eur. J. Biochem. 226, 255 (1994)).

### 7.5. Herstellung des Wirkstoffes für Arthritis

### a) Konstruktion des chimären Promotors TIMP-3-CDE-CHR-Inr

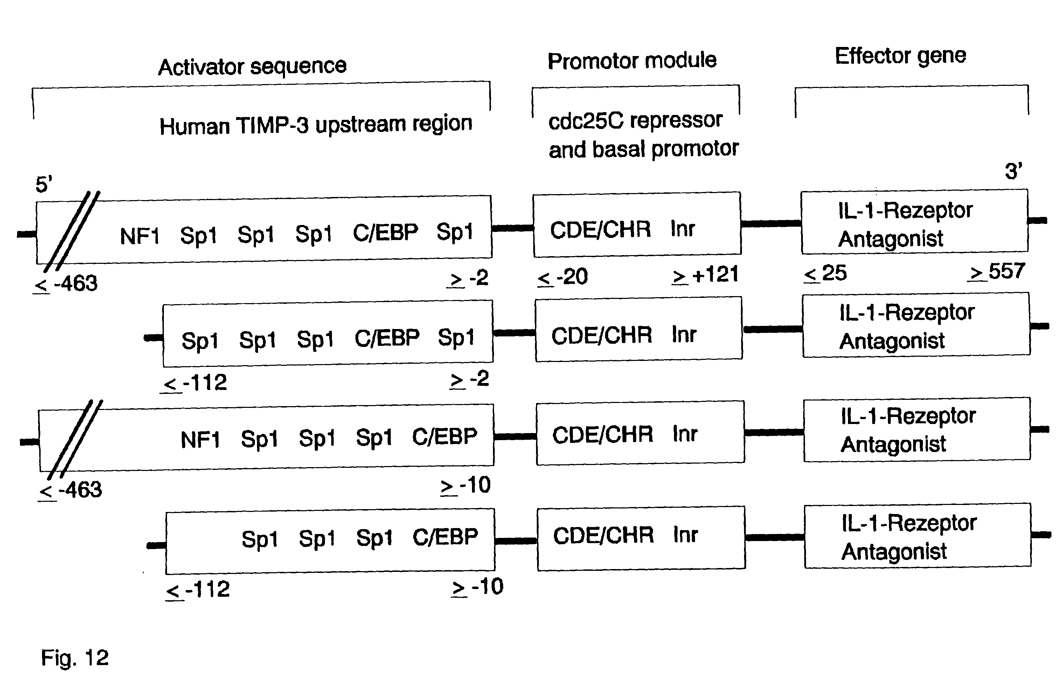

Der humane TIMP-3 Promotor (Pos. ≤ -463 bis ≥ -2) oder verkürzte Varianten (Pos. ≤ -112 bis ≥ -2 oder ≤ -463 bis ≥ -10 oder ≤ -112 bis ≥ -10) werden an ihrem 3' Ende mit dem 5'-Terminus des CDE-CHR-Inr Moduls (Pos. ≤ -20 bis ≥ + 121) des humanen cdc25C-Gens (Pos. ≤ -20 bis ≥ +121) verknüpft (Fig. 12). Die Verknüpfung erfolgt mit Hilfe von dem Fachmann bekannten und käuflichen Enzymen. Es werden verschiedene Fragmente der TIMP-3 Promotorsequenz verwendet, um (1) ein möglichst kurzes Promotorfragment (bei möglichst effizienter Transkription) einsetzen zu können und (2) ungewünschte Effekte des TIMP-3 Initiators (Bereich bei +1) auf die Regulation durch CDE/CHR auszuschalten.

### b) Konstruktion eines Plasmids enthaltend den zentralen Bestandteil des Wirkstoffes

Die unter a) beschriebenen chimären TIMP-3 Promotormodul-Transkriptionskontrolleinheiten werden an ihren 3' Enden mit dem 5'-Terminus einer DNA, die den kompletten kodierenden Bereich des 152 Aminosäuren langen IL-1-Rezeptor-Antagonisten enthält (DNA Pos. ≤ 25 bis ≥ 557; Eisenberg et al., Nature 343, 341 (1990)), verknüpft. Diese DNA enthält auch die für eine Sekretion notwendige Signalsequenz (25 N-terminale Aminosäuren) (Fig. 12). Transkriptionskontrolleinheiten und IL-1-Rezeptor-Antagonist DNA werden in pUC18/19 oder Bluescript-abgeleiteten Plasmidvektoren einkloniert, die direkt (Yovandich et al., Hum. Gene Ther. 6, 603 (1995)) oder in kolloidalen Dispersionssystemen für eine in vivo Applikation zur Transduktion von Synovialzellen genutzt werden können. Alternativ können die zusammengefügten Transkriptionskontrolleinheiten und IL-1-Rezeptor-Antagonisten DNA in virale Vektoren oder andere geeignete Vektoren transferiert und injiziert werden.

### 8) Herstellung eines Wirkstoffes gegen Infektionserreger

Der Wirkstoff kann in zwei grundsätzlich unterschiedlichen Formen hergestellt werden
- für die Therapie von Virusinfektionen und Parasiteninvasionen oder aber
- für die Prophylaxe von Infektionserkrankungen durch Viren, Bakterien oder Parasiten.

Zur Prophylaxe von Infektionserkrankungen dienen Impfstoffe. Die Möglichkeiten, auf konventionellem Wege wirkungsvolle Impfstoffe herzustellen, sind jedoch beschränkt (Brown, Int.J. Technol. Assessm. Health Care 10, 161 (1994), Ellis, Adv. Exp. Med. Biol. 327, 263 (1992), Arnon et al., FASEB J. 6, 3265 (1992)).

Demzufolge wurde die Technologie der DNA-Vakzine entwickelt. Diese DNA-Vakzinen werfen jedoch Fragen zur Sicherheit und zu Nebenwirkungen auf (Fynan et al., Int. J. Immunopharm. 17, 79 (1995), Donnelly et al., Immunol. 2, 20 (1994)).

Wirkstoffe zur Prophylaxe von Infektionserkrankungen im Sinne dieser Erfindung zeichnen sich wegen ihrer Zellspezifität und Zellzyklusregulation durch ein hohes Maß an Sicherheit aus.

### 8.1. Auswahl der Aktivatorsequenz

### a) zur Therapie von Infektionserkrankungen

Als Aktivatorsequenz sind Promotorsequenzen von Proteinen auszuwählen, welche besonders von Bakterien oder Parasiten gebildet werden oder es sind Promotorsequenzen solcher Viren auszuwählen, die die von ihnen infizierten Zellen transformieren und zur Proliferation anregen.

Zu diesen Viren gehören beispielsweise HBV, HCV, HSV; HPV, HIV, EBV und HTLV.

Promotorsequenzen für derartige Viren wurden wie folgt beschrieben:
* HBV
   (Sato et al., Annals Int. Med. 122, 241 (1995), Raney et al., J. Gen. Virol. 75, 2671 (1994), Raney et al. J. Virol. 66, 6912 (1992), Zhang et al., J. Virol. 67, 1472 (1993), Guo et al., J. Virol. 65, 6686 (1991))
* HCV
   (Matsuura et al., Intervirol. 37, 114 (1994), Kumar et al., J. General Virol. 73, 1521 (1992), Kim et al., Jap. J. Med. Sci. Biol. 47, 211 (1994))
* HSV
   (Greco et al., J. Gen. Virol. 75, 1693 (1994), Papavassiliou et al., J. Biol. Chem. 265, 9402 (1990))
* HPV
   (May et al., EMBO J. 13, 1460 (1994), Thierry et al., EMBO J. 6, 3391 (1987))
* EBV
   (Chen et al., DNA and Cell Biol. 14, 205 (1995), Nonkwelo et al., Virol. 206, 183 (1995), Smith et al., J. Virol. 66, 706 (1992), Lear et al., J. Virol. 66, 7461 (1992), Rooney et al., J. Virol. 66, 496 (1992))
* HTLV
   (Ohtani et al., EMBO J. 6, 389 (1987))
* HIV
   (Koken et al., Virol. 191, 968 (1992), Berghout et al., J. Virol. 66, 139 (1992), Cherrington et al., EMBO J. 11, 1513 (1992), Rosen et al., Cell 41, 813 (1985))

Die HIV- (long terminal repeat) LTR-Sequenz dient als Bindungsstelle für zelluläre transaktivierende Faktoren, die in zahlreichen unterschiedlichen Zellen und Geweben vorkommen (Levy, AIDS 4, 1051 (1990)). Zu diesen gehören die Trankriptionsfaktoren SP1, EBP-1, UBP-1, NF-KB, LBP-1 und CTN-NF (Garcia et al., EMBO J. 6, 3761 (1987)). Am 3' Ende des LTR befindet sich die Transaktivatorregion (TAR), an welche das Transaktivatorprotein (TAT) von HIV bindet (Cullen, Cell 63, 655 (1986), Selby et al., Genes and Dev. 3, 547 (1989)). Eine weitere Bindungsstelle für das TAT Protein wurde in der NF-KB Domäne des HIV-LTR beschrieben (Taylor et al., EMBO J. 11, 3395 (1992)). Das Transaktivatorprotein (TAT) von HIV kann die Expression des LTR Genes von HIV um mehr als das hundertfache steigern (Dayton et al., Cell 44, 941 (1986), Rosen et al., Nature 319, 555 (1986), Laspia et al., Cell 59, 283 (1989)). Die TAR-Region ist somit integraler Bestandteil der Transkription und Translation von HIV-LTR (Garcia et al., EMBO J., 8, 765 (1989)). HIV-LTR kann als Promotor nicht nur für HIV-Gene sondern auch für heterologe Reportergene und für letztere auch ohne die Anwesenheit von HIV-TAT dienen (Banerjee et al., Hepatol. 10, 1008 (1989), Virology 179, 410 (1990)). Anhaltspunkte bestehen, daß diese Promotoraktivität durch zelluläre Transkriptionsfaktoren, die dem HIV-TAT funktionell ähneln, aktiviert werden. Diese Aktivierung durch zelluläre TAT-ähnliche Faktoren ist im allgemeinen jedoch geringer als durch HIV-TAT (Sodroski et al., Science 229, 74 (1985), Dayton et al., Cell 44, 941 (1986), Rosen et al., Nature 319, 555 (1986)). Eine relativ starke Aktivierung des HIV-LTR durch zelluläre TAT-ähnliche Faktoren konnte jedoch in Leberzellen beobachtet werden (Pizzela und Banerjee, DNA and Cell Biol. 13, 67 (1994)).

TAR liegt sowohl als DNA als auch als RNA vor. Experimentelle Studien zeigen jedoch, daß die TAR als RNA durch deren Sekundärstruktur an TAT bindet und funktionell aktiv ist (Roy et al., J. Virol 64, 1402 (1990)), d.h. an die korrespondierende Promotor DNA bindet und diese aktiviert (Berkhout et al., Cell 62, 757 (1990)). TAR ist, wenn überhaupt, nur geringfügig aktiv in Verbindung mit einem heterologen Promotor (Maesing et al., Cell 48, 691 (1987), Berkhout et al., Cell 62, 757 (1990)) des weiteren ist eine optimale Funktion von TAR nur bei direkter Nachbarschaft zu den am 5' Terminus von TAR angrenzenden Nukleotidsequenzen des HIV-LTR Promotors, insbesondere der NF-KB/SP1-Bindungsregion, gewährleistet (Berkhout et al., Cell 62, 757 (1990)).

Für HIV ist somit die gesamte LTR-Sequenz einschließlich der TAR-Sequenz (Position ≤ -453 bis ≥ +80, Rosen et al., Cell 41, 813 (1985) als virusspezifischer Promotor einzusetzen.

### c) zur Prophylaxe von Infektionskrankheiten

Als Aktivatorsequenz sind Promotorsequenzen der Gene solcher Proteine auszuwählen, welche in aktivierten Makrophagen und aktivierten Lymphozyten in besonderem Maße gebildet werden. Beispiele für derartige Proteine und ihre Gene wurden im Abschnitt 6.1. aufgeführt.

### 8.2. Auswahl der Wirksubstanz

### a) zur Therapie von Infektionserkrankungen

Als Wirksubstanz ist die DNA eines Proteins auszuwählen, welches zytostatische, zytotoxische oder antivirale Wirkungen aufweist. Beispiele für zytotoxische oder zytostatische Proteine wurden schon im Abschnitt 6.2. e-g) aufgeführt. Bei Wahl eines Enzyms (siehe hierzu Abschnitt 6.2.g) ist nachfolgend die durch dieses Enzym spaltbare Vorstufe einer antiviralen zytotoxischen oder antiparasitären Substanz zu verabreichen.

Wirksubstanzen für antivirale Proteine im Sinne dieser Erfindung sind des weiteren antiviral wirksame Cytokine und Wachstumsfaktoren. Hierzu zählen beispielsweise die DNA-Sequenzen für folgende Wirksubstanzen:
- IFNα
   (Henco et al., J. Mol. Biol. 185, 227 (1985), Pestka et al., Ann. Rev. Biochem. 56, 727 (1987), Weissmann et al., Phil. Trans. R. Soc. Lond. B299, 7 (1982), Goeddel et al., Nature 290, 20 (1981))
- IFNβ
   (Sen et al., J. Biol. Chem. 267 5017 (1992), Mark et al. EP 192 811, EP 234 599, US 45 88 585)
- IFN-γ
   (Gray et al., Nature 295, 503 (1982), Yip et al., PNAS USA 79, 1820 (1982), Rinderknecht et al., J. Biol. Chem. 259, 6790 (1984))
- TNFβ
   (Gray et al., Nature 312, 721 (1984), Li et al., J. Immunol. 138, 4496 (1967), Aggarwal et al., J. Biol. Chem. 260, 2334 (1985))
- TNFα
   (Beutler et al., Nature 320, 584 (1986), Kriegler et al., Cell 53, 45 (1988)
- IL-1
   (Furntani et al., Nucl. Acids Res. 14, 3167 (1986), Lafage et al., Blood 73, 104 (1989), March et al., Nature 315, 641 (1985), Bensi et al., Gene 52, 95 (1987), Auron et al., PNAS 81, 7907 (1984), Clark et al., Nucl. Acids Res. 14, 7897 (1986))
- TGFβ
   (Massague, Ann. Rev. Cell Biol. 6, 597 (1990), Kondiah et al., J. Biol. Chem. 265, 1089 (1990), Garnier et al., J. Molec. Biol. 120, 97 (1978))

Als Wirksubstanz im Sinne der Erfindung können jedoch auch DNA-Sequenzen von Fusionsproteinen zwischen den aufgeführten Cytokinen, Wachstumsfaktoren oder dem extrazellulären Teil der Rezeptoren zum einen und dem Fc-Teil des menschlichen Immunglobulins zum anderen Verwendung finden. Derartige DNA-Sequenzen und ihre Herstellung wurden in der EP 0 464 633 A1 beschrieben.

Desweiteren ist Wirksubstanz im Sinne dieser Erfindung die DNA-Sequenz für einen Antikörper einer Spezifität, die das jeweilige Virus inaktiviert oder dessen V_{H} und V_{L} enthaltende Fragmente oder dessen über einen Linker verbundene V_{H} und V_{L} Fragmente, hergestellt beispielsweise entsprechend der von Marasco et al. (Proc. Natl. Acad. Sci. USA 90, 7889 (1993)) beschriebenen Methodik. Beispiele für Antikörper einer derartigen Spezifität gegen Viren werden im Abschnitt 8.4. aufgefünrt.

Desweiteren ist Wirksubstanz im Sinne dieser Erfindung die DNA-Sequenz für ein Rev bindendes Protein. Diese Proteine binden an die Rev-RNA und inhibieren Rev-abhängige posttranskriptionelle Stufen der Retrovirus-Genexpression. Beispiele für Rev-bindende Proteine sind:
- RBP9-27
   (Constantoulakis et al., Science 259, 1314 (1993), Reid et al., PNAS USA 86, 840 (1989))
- RBP1-8U
   (Kerr und Stark, FEBS Lett. 285, 194 (1991), Friedman et al., Cell 38, 745 (1984))
- RBP1-8D
   (Lewin et al., Eur. J. Biochem. 199, 417 (1991))
- Pseudogene von RBP1-8
   (Lewin et al., Eur. J. Biochem. 199, 417 (1991)).

### b) zur Prophylaxe von Infektionserkrankungen

Als Wirksubstanz ist die DNA eines vom Infektionserreger gebildeten Proteins auszuwählen, welches durch eine Immunreaktion, d.h. durch Antikörperbindung und/oder durch zytotoxische T-Lymphozyten zur Neutralisierung und/oder zur Abtötung des Erregers führt. Derartige Neutralisationsantigene werden als Impfantigene bereits angewandt (siehe Übersicht bei Ellis, Adv. Exp. Med. Biol. 327, 263 (1992)). Beispiele für DNA-Sequenzen, die Neutralisationsantigene kodieren, sind durch die folgenden Arbeiten zugänglich:
- Influenza A-Virus-Antigen
   (Ulmer et al., Science 259, 1745 (1993), Robinson et al., Vaccine 11, 957 (1993), Fynan et al., Int. J. Immunopharmac. 17, 79 (1995))
- HIV-Antigene
   (Wang et al., PNAS USA 90, 4156 (1993))
- Tollwut-Virus-Antigen
   (Donnelly et al., Immunol. 2/1, 20 (1994))
- HSV (Herpes Simplex Virus)-Antigen
   (Fleckenstein et al., Nature 274, 57 (1978))
- RSV (Respiratory Syncytial Virus)-Antigen
   (Du et al., Bio/Tech. 12, 813 (1994), Hall, Science 265, 1393 (1993))
- Parainfluenza-Virus-Antigen
   (Du et al., Bio/Techn. 12, 813 (1994))
- Rotavirus-Antigen
   (Albert et al., J. Clin. Microbiol. 25, 183 (1987), Anderson et al., J. Infect. Dis. 153, 823 (1986), Battaglia et al., J. Infect. Dis. 155, 140 (1987), Chanock et al., J. Infect. Dis. 148, 49 (1983), Dyall-Smith et al., J. Virol. 38, 1099 (1981), Glass et al., Science 265, 1389 (1994))
- VZV (Varizella Zoster Virus)-Antigen
   (Straus et al., Ann. Intern. Med. 109, 438 (1988), Gershon, Pediatr. Infect. Dis. 2, 171 (1991), Kinchington et al., J. Virol. 64, 4540 (1990))
- CMV (Cytomegalo-Virus)-Antigen
   (Plotkin, Science 265, 1383 (1994))
- Masern-Virus-Antigen
   (Katz und Kellin, Science 265, 1391 (1994))
- HPV (Humanes Papillomvirus)-Antigen
   (Tindl und Frazer, Curr. Topics Microbiol. Immunol. 186, 217 (1994))
- HBV (Hepatitis B-Virus)-Antigen
   (Valenzuela et al., Nature 280, 815 (1979), Heerman et al., J. Virol. 52, 396 (1984))
- HCV (Hepatitis C-Virus)-Antigen
   (Cerny et al., Curr. Topics Microbiol. Immunol. 189, 169 (1994), Esteban et al., Progr. Liver Dis. 10, 253 (1992), Jung et al., Eur. J. Clin. Invest. 24, 641 (1994))
- HDV (Hepatitis D-Virus)-Antigen
   (Iwarson, Scand. J. Infect. Dis. 24, 129 (1992), Consolo et al., Nephron. 61, 251 (1992))
- HEV (Hepatitis E-Virus)-Antigen
   (Iwarson, Scand. J. Infect. Dis. 24, 129 (1992), Consolo et al., Nephron. 61, 251 (1992))
- HAV (Hepatitis A-Virus)-Antigen
   (d'Hondt, Vaccine 10, 48 (1992), Andre, J. Infect. Dis. 171, 33 (1995), Lemon et al., Vaccine 10, 40 (1992), Melnick et al., Vaccine 10, 24 (1992), Flehmig, Baillieres Clin. Gastroenterol. 4, 707 (1990))
- Vibrio Cholera-Antigen
   (Levine und Kaper, Vaccine 11, 207 (1993))
- Borrelia Burgdorferi-Antigen
   (Schaible et al., Immunol. Letters 36, 219 (1993), Wallich et al., Lab. Med. 17, 669 (1993))
- Helicobacter pylori-Antigen
   (Crabtree et al., Lancet 338, 332 (1991), Blaser, J. Infect. Dis. 161, 626 (1990), Cover und Blaser, J. Biol. Chem. 267, 10570 (1993), Cover et al., Infect. Immunol. 58, 603 (1990), Dunn et al., J. Biol. Chem. 265, 9464 (1990), Dunn e tal., Infect. Immunol. 60, 1946 (1992), Lage et al., Acta Gastroenterol. Belg. 56 (suppl.), 61 (1993), Mobley et al., Scand. J. Gastroint. 26 (suppl. 187), 39 (1991))
- Malaria-Antigen
   (Nussenzweig und Long, Science 265, 1381 (1994), Maurice, Science 267, 320 (1995), Enders et al., Vaccines 10, 920 (1992), Knapp et al., Infect. Imm. 60, 2397 (1992))

Zu derartigen Wirksubstanzen im Sinne der Erfindung gehört jedoch auch die DNA eines Antiidiotyp-Antikörpers oder seiner Antigen-bindenden Fragmente, dessen Antigenbindungsstrukturen, die "complementary determining regions", Kopien der Protein- oder Kohlenhydratstruktur des Neutralisationsantigens des Infektionserregers darstellen.

Derartige Antiidiotyp-Antikörper können besonders Kohlenhydratantigene bei bakteriellen Infektionserregern ersetzen.

Derartige antiidiotypische Antikörper und ihre Spaltprodukte wurden von Hawkins et al. (J. Immunother. 14, 273 (1993)) und Westerink und Apicella (Springer Seminars in Immunopathol. 15, 227 (1993)) übersichtlich beschrieben.

### 8.3. Kombination gleicher oder unterschiedlicher Wirksubstanzen für die Therapie oder Prophylaxe von Infektionserkrankungen

Gegenstand der Erfindung ist desweiteren ein Wirkstoff, in welchem eine Kombination der DNA-Sequenzen von gleichen Wirksubstanzen (A,A) oder unterschiedlichen Wirksubstanzen (A,B) vorliegt. Zur Expression von zwei Sequenzen ist vorzugsweise die cDNA einer "internal ribosome entry site" (IRES) als regulatorisches Element zwischengeschaltet.

Derartige IRES wurden beispielsweise von Montford und Smith (TIG 11, 179 (1995), Kaufman et al., Nucl. Acids Res. 19, 4485 (1991), Morgan et al., Nucl. Acids Res. 20, 1293 (1992, Dirks et al., Gene 128, 247 (1993), Pelletier und Sonenberg, Nature 334, 320 (1988) und Sugitomo et al., BioTechn. 12, 694 (1994) beschrieben.

So kann die cDNA der IRES-Sequenz des Poliovirus (Position ≤ 140 bis ≥ 630 des 5' UTR (Pelletier und Sonenberg, Nature 334, 320 (1988)) zur Verknüpfung der DNA der viralen Substanz A (am 3' Ende) und der DNA der antiviralen Substanz B (am 5' Terminus) verwendet werden.

Aktivator-
sequenz
(UAS)

zellzyklus-
reguliertes
Promotor-
modul

Wirksubstanz
A

internal
ribosome
entry
site

Wirksubstanz
A oder B

Ein derartiger Wirkstoff weist je nach Kombination additive (A+A, A+B1) oder synergistische Wirkung im Sinne der Erfindung auf.

So können beispielsweise für die Therapie von Viruserkrankungen zwei gleiche oder zwei unterschiedliche antivirale Wirksubstanzen miteinander kombiniert werden.

Bei der Prophylaxe von Infektionserkrankungen können mehrere Wirksubstanzen, die für unterschiedliche Antigene eines Infektionserregers oder unterschiedlicher Infektionserreger kodieren, miteinander kombiniert werden. Des weiteren kann die Wirksubstanz, die für das Antigen eines Infektionserregers kodiert, kombiniert werden mit einer Wirksubstanz, die kodiert für ein Cytokin oder einen Cytokinrezeptor.

Die sich so (nach Injektion des Wirkstoffes) gleichzeitig mit dem Infektionserregerantigen bildenden Cytokine oder Cytokinrezeptoren können Einfluß auf die Art und Stärke der sich entwickelnden Immunreaktion nehmen.

DNA-Sequenzen für Cytokine und Cytokinrezeptoren, welche die humorale Immunreaktion verstärken, sind bereits unter 6.2.d) beschrieben, solche zur Verstärkung der zellulären Immunreaktion unter 6.2.a) und 6.2.c).

DNA-Sequenzen für Cytokine, welche die Immunreaktion insgesamt verstärken, sind beispielsweise:
- IL-1α
   (Fenton, Int. J. Immunopharm. 14, 401 (1992), Furntani et al., Nucl. Acids Res. 14, 3167 (1986), Lafage et al., Blood 73, 104 (1989), March et al., Nature 315, 641 (1985))
- IL-1β
   (Bensi et al., Gene 52, 95 (1987), Auron et al., PNAS 81, 7907 (1984), Clark et al., Nucl. Acids Res. 14, 7897 (1986))
- IL-2
   (Fletscher et al., Lymphok. Res. 6, 45 (1987), Matsui et al., Lymphokines 12, 1 (1985), Tanaguchi et al., Nature 302, 305 (1983))
- GM-CSF
   (Gough et al., Nature 309, 763 (1984), Nicola et al., J. Biol. Chem. 254, 5290 (1979), Wong et al., Science 228, 810 (1985))

### 8.4. Auswahl der Liganden für Infektionserreger

Zur Therapie von Infektionserkrankungen gehören zu den Liganden Antikörper oder Antikörperfragmente, gerichtet gegen die Infektionserreger. Beispielsweise sind dies bei Virusinfektionen die Virusantigene exprimiert auf der Zellmembran von virusinfizierten Zellen.

Derartige Antikörper sind beispielsweise für mit folgenden Viren infizierte Zellen beschrieben worden:
* HBV (Shonval et al., PNAS USA 79, 650 (1982), Intercell. Intracell. Comm. 2, 221 (1986), Klein et al., Virus Genes 5, 157 (1991))
* HCV (Takahashi et al., Virol. 191, 431 (1992))
* HSV (Sanchez-Pescador et al., J. Infect. dis. 166, 623 (1992))
* HPV (Doorbar et al., Virol. 187, 353 (1992))
* HIV (Nishino et al., Vaccine 10, 677 (1992))
* EBV (Thorley-Lawson et al., Cell 30, 415 (1982))
* HTLV (Robert-Garoff et al., J. Virol. 53, 214 (1985), Matsushita et al., J. Virol. 62, 2107 (1988)).

Desweiteren gehören zu den Liganden auch monoklonale oder polyklonale Antikörper oder Antikörperfragmente, die mit ihren konstanten Domänen an Fc-γ- oder -Rezeptoren von Immunzellen binden (Rojanasakul et al., Pharm. Res. 11, 1731 (1994)).

Die murinen monoklonalen Antikörper sind bevorzugt in humanisierter Form einzusetzen. Die Humanisierung erfolgt in der von Winter et al. (Nature 349, 293 (1991)) und Hoogenbooms et al. (Rev. Tr. Transfus. Hemobiol. 36, 19 (1993)) dargestellten Weise. Antikörperfragmente werden entsprechend dem Stand der Technik hergestellt, beispielsweise in der von Winter et al. (Nature 349, 293 (1991), Hoogenboom et al. (Rev. Tr. Transfus. Hemobiol. 36, 19 (1993), Girol (Mol. Immunol. 28, 1379 (1991) und Huston et al. (Int. Rev. Immunol. 10, 195 (1993) beschriebenen Weise.

Zu den Liganden gehören des weiteren alle Substanzen, welche an Membranstrukturen oder Membranrezeptoren auf der Oberfläche von virusinfizierten Zellen binden. Beispielsweise gehören hierzu Wachstumsfaktoren, wie Zytokine, EGF, TGF, FGF oder PDGF, oder deren Fragmente bzw. Teilsequenzen von ihnen, die an Rezeptoren exprimiert durch derartige Zellen binden.

Hierzu gehören des weiteren Liganden, welche an Zellmembranstrukturen binden, welche selektiv sind für bestimmte Gewebe. Hierzu zählen beispielsweise:

| Membranstruktur | Liganden | Gewebezellen |
|---|---|---|
| Asialoglycoprotein- | Asialoorosomucoid | Leberzellen |
| Rezeptor | Neoglycoprotein Galactose | |
| Transferrin-Rezeptor | Transferrin | Leber, andere Gewebezellen |
| Insulin-Rezeptor | Insulin | Leberzelle, andere Gewebezellen |
| Mannose 6-Phosphat-Rezeptor | Mannose | Makrophagen in Milz, Leber, Lunge, andere Gewebe |
| Fc-γ-Rezeptoren | Immunglobulin G | retikuloendo theliales System, andere Gewebe |

Diese Liganden und Membranstrukturen sind übersichtlich bei Perales et al., Eur. J. Biochem. 226, 255 (1994) beschrieben.

Für die Prophylaxe von Infektionserkrankungen sind als Liganden alle Substanzen geeignet, welche an Zellmembranstrukturen von Makrophagen und/oder Lymphozyten binden. Derartige Liganden wurden bereits im Abschnitt 6.4. beschrieben.

### 8.5. Auswahl der Liganden für einen Wirkstoff zur Prophylaxe von Infektionserregern

Als Liganden für virale und nicht-virale Vektoren, beispielsweise in kolloidalen Dispersionen oder in Polylysin-Ligand-Komplexen werden Substanzen bevorzugt, welche an die Oberfläche von Makrophagen und/oder Lymphozyten spezifisch binden. Derartige Liganden sind bereits im Abschnitt 6.4. beschrieben worden.

Diese Liganden sind Bestandteil der Vektoren. Im Sinne dieser Erfindung können jedoch auch Liganden den Vektoren zugemischt werden. Für diese Mischung sind besonders Liganden zu verwenden, welche in der Lage sind, Makrophagen und/oder Lymphozyten zu aktivieren. Hierzu gehören beispielsweise:
- Cytokine wie beispielsweise IL-1α, IL-1β, IL-2, IL-3, IL-4, IL-5, IL-6, IL-10, IL-12, IFN-γ, GM-CSF oder M-CSF
   (Hadden, Int. J. Immunopharm. 16, 703 (1994))
- lösliche Cytokinrezeptoren wie beispielsweise IL-4-Rezeptor.

Alternativ oder zusätzlich können Adjuvantien hinzugemischt werden. Hierzu gehören beispielsweise:
- synthetische Adjuvantien
   (Übersichten bei: Parant, Int. J. Immunopharm. 16, 445 (1994), Cernescu, Int. J. Immunopharm. 16, 369 (1994))
- Liposomen
   (Übersichten bei: Alving, J. Immunol. Methods 140, 1 (1991) und BBA 1113, 307 (1992), Sato und Sanamoto, Prog. Lipid Res. 31, 345 (1992))
- Lipopolysaccharide oder Lipid A
   (Übersicht bei: Alving, Immunobiol. 187, 430 (1993))
- bioabbaubare Polymere wie beispielsweise
   * Poly (DL-Lactide-Co-Glycolide)
      (Eldridge et al., Infect. Immun. 59, 2978 (1991))
   * Pseudolatexes
      (Coffin und McGiuity, Pharmaceut. Res. 9, 200 (1992))
- Muramyldipeptide
   (Morin et al., Int. J. Immunopharm. 16, 451 (1994)).

Desweiteren ist es im Sinne der Erfindung, Substanzen den Vektoren zuzumischen, welche sie für eine Aufnahme über die Schleimhaut und für beispielsweise orale Immunisierung geeignet machen.

Derartige Substanzen und Formulierungen wurden von Walker (Vaccine 12, 387 (1994)) übersichtlich dargestellt.

### 8.6. Herstellung des Wirkstoffes gegen Virusinfektionen

Die Herstellung des erfindungsgemäßen Wirkstoffes wird anhand folgender Beispiele näher beschrieben:

### a) Konstruktion des chimären Promotors HIV-LTR-TAR-CDE-CHR-Inr

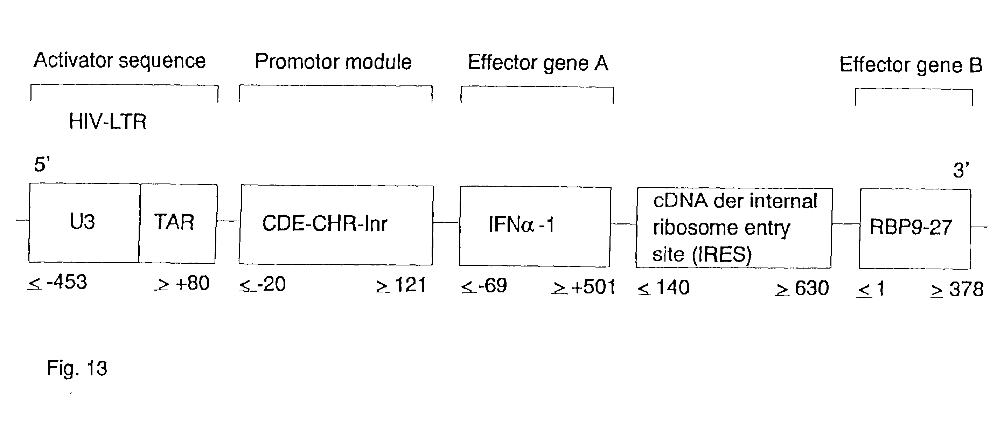

Der HIV-LTR-TAR Promotor (Position ≤ -453 bis ≥ +80, (Rosen et al., Cell 41, 813 (1985)) wird an seinem 3' Ende mit dem 5'-Terminus des CDE-CHR-Inr Moduls des humanen cdc25C-Gens (Position ≤ -20 bis ≥ +121, Lucibello et al., EMBO J., 14, 132 (1995)) verknüpft (Fig. 13). Die Verknüpfung erfolgt mit Hilfe von dem Fachmann bekannten und käuflichen Enzymen.

### b) Konstruktion eines Plasmids enthaltend den chimären Promotor HIV-LTR-TAR-CDE-CHR-Inr im zentralen Bestandteil des Wirkstoffes

Die beschriebene chimäre Promotormodul-Transkriptionseinheit wird an ihren 3' Enden mit dem 5'-Terminus einer DNA, die den kompletten kodierenden Bereich des Interferon α-1 (Position ≤ -69 bis ≥ +501, (Streuli et al., Science 209, 1343 (1980)) enthält, verknüpft. Diese DNA enthält auch die für eine Sekretion notwendige Signalsequenz. Transkriptionskontrolleinheiten und die DNA für Interferonα-1 werden in pUC19/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### c) Konstruktion eines Plasmids enthaltend zwei Gene für Wirksubstanzen

Die, wie unter a) beschriebene, HIV-LTR-TAR-CDE-CHR-Inr-Transkriptionseinheit wird an ihrem 3' Ende mit dem 5' Ende der DNA für das Interferonα-1 (Position ≤ -69 bis ≥ +501; Streuli et al., Science 209, 1343 (1980)) verknüpft. Die Verknüpfung erfolgt mit Hilfe von dem Fachmann bekannten und käuflichen Enzymen.

Das 3' Ende der DNA für Interferon∝-1 wird nunmehr verknüpft mit dem 5' Ende der cDNA der internal ribosome entry site (Position ≤ 140 bis ≥ 630; Pelletier und Sonenberg, Nature 334, 320 (1988)) und ausschließend deren 3' Ende mit dem 5' Ende der DNA für das Rev-bindende Protein (RbP9-27) verknüpft (Position ≤ 1 bis ≥ 378, Reid et al., PNAS USA 86, 840 (1989)) (siehe Fig. 13). Dieser so hergestellte Wirkstoff wird anschließend in puc18/19 oder in Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### 9) Herstellung eines Wirkstoffes gegen Leukämien (und Lymphome)

### 9.1. Auswahl der Aktivatorsequenz für Leukämien

Als Aktivatorsequenz (UAS = upstream activator sequence) ist eine Nukleotidsequenz (Promotor- oder Enhancersequenz) vorgesehen, mit der Transkriptionsfaktoren, gebildet oder aktiv in Leukämiezellen, interagieren.

Im Sinne dieser Erfindung zählen zu den bevorzugten Aktivatorsequenzen jedoch genregulatorische Sequenzen bzw. Elemente aus Genen, die besonders in Leukämiezellen gebildete Proteine kodieren.

Hierzu zählen beispielsweise die in nachfolgenden Literaturzitaten aufgeführten Promotorsequenzen für Gene, die für folgende Proteine kodieren:
- c-myc
   (Bentley et al., Mol. Cell. Biol. 6, 3481 (1986), Lang et al., Oncogene 6, 2067 (1991), Meulia et al., Mol. Cell. Biol. 12, 4590 (1992), Desjardins, Mol. Cell. Biol. 13, 5710 (1993))
- HSP-70
   (Taira et al., BBA 1130, 166 (1992))
- bcl-1/cyclin D-1
   (Herber et al., Oncogene 9, 1295 (1994))
- bcl-2
   (Young et al., Mol. Cell. Biol. 13, 3686 (1993))
- IL-6
   (Droogmans et al., DNA-Sequence 3, 115 (1992), Mori et al., Blood 84, 2904 (1994), Liberman et al., Mol. Cell. Biol. 10, 2327 (1990), Ishiki et al., Mol. Cell. Biol. 10, 2757 (1990))
- 11-10
   (Kim et al., J. Immunol. 148, 3618 (1992), Kube et al., Cytokine 7, 1 (1995), Platzer et al., DNA-Sequence 4, 399 (1994), Kube et al., Cytokine 7, 1 (1995))
- NFα, TNFβ
   (Sidhu et al., Pharmac. Ther. 57, 79 (1993), Vilcek et al., J. Biol. Chem. 266, 7313 (1991), Tahashiba et al., Gene 131, 307 (1993), Nedwin et al., Nucl. Acids Res. 13, 6361 (1985), Paul et al., J. Virol. 64, 5412 (1990), Shakhov et al., J. Exp. Med. 171, 35 (1990), van der Ake et al., Nucleic Acids Res. 21, 5636 (1993)).

Desweiteren gehören hierzu Bindungssequenzen für Proteine, gebildet von folgenden Genen:
- HOX-11
   (Dear et al., PNAS USA 90, 4431 (1990))
- BCR-Abl
   (Zhu et al., Nucl. Acid Res. 18, 7119 (1990), Shah et al., Mol. Cell. Biol. 11, 1854 (1991))
- E2A-PBX-1
   (Monica et al., Mol. Cell. Biol. 14, 8304 (1994), Numata et al., Leukemia 7, 1441 (1993), von Dijk et al., PNAS USA 90, 6061 (1993))
- PML-RARA
   (Promyelocytic Leukemia - Retinoic Acid Receptor) (Potter et al., Leukemia 7, 1302 (1993), Yoshida et al., Genes, Chromosomes Cancer 12, 37 (1995), Brand et al., Nucl. Acids Res. 18, 6799 (1990))
- c-myc
   c-myc-Proteine binden an und aktivieren Multimere der als Myc E-Box bezeichneten Nukleotidsequenz (5'-GGAAGCAGACCACGTGGTCT-GCTTCC-3')
   (Blackwood und Eisenman, Science 251, 1211 (1991))

### 9.2. Auswahl der Wirksubstanz für Leukämien

Als Wirksubstanz im Sinne der Erfindung ist eine DNA-Sequenz zu verstehen, deren exprimiertes Protein die Proliferation von Zellen, insbesondere auch von Leukämiezellen, inhibiert. Zu diesen Zellzyklusinhibitoren gehören beispielsweise die DNA-Sequenzen für inhibitorische zytostatische und ztyotoxische Proteine und Enzyme, wie sie bereits im Abschnitt 6.2. e-g) beschrieben wurden.

Als Zellzyklusinhibitor ist des weiteren eine DNA-Sequenz zu verstehen, welche ein Protein exprimiert, welches direkt oder indirekt eine zytostatische oder zytotoxische Wirkung auf Leukämien aufweist. Zu derartigen Proteinen zählen beispielsweise:
- IL-1α
   (Fenton, Int. J. Immunopharm. 14, 401 (1992), Furntani et al., Nucl. Acids Res. 14, 3167 (1986), Lafage et al., Blood 73, 104 (1989), March et al., Nature 315, 641 (1985))
- IL-1β
   (Bensi et al., Gene 52, 95 (1987), Auron et al., PNAS 81, 7907 (1984), Clark et al., Nucl. Acids Res. 14, 7897 (1986))
- IL-2
   (Fletscher et al., Lymphok. Res. 6, 45 (1987), Matsui et al., Lymphokines 12, 1 (1985), Tanaguchi et al., Nature 302, 305 (1983))
- IL-4
   (Lee et al., PNAS 83, 2061 (1986); Paul, Blood 77, 1859 (1991), Yokota et al., PNAS USA 83, 5894 (1986), von Leuven et al., Blood 73, 1142 (1989), Arai et al., J. Immunol. 142, 274 (1989))
- IL-10
   (Vieira et al., PNAS USA 88, 1172 (1991), Moore et al., Science 248, 1230 (1990), Kim et al., J. Immunol. 148, 3618 (1992))
- IL-12
   (Gubler et al., PNAS USA 88, 4143 (1991), Wolf et al., J. Immunol. 146, 3074 (1991), Kobayashi et al., J. Exp. Med. 170, 827 (1989), Gately et al., J. Immunol. 147, 874 (1991), Schoenhaut et al., J. Immunol. 148, 3433 (1992),
- Interferone, wie beispielsweise
   * IFNα (Henco et al., J. Mol. Biol. 185, 227 (1985), Pestka et al., Annu. Rev. Biochem. 56, 727 (1987), Weissmann et al., Phil. Trans. R. Soc. Lond. B299, 7 (1982), Goeddel et al., Nature 290, 20 (1981))
   * IFNβ (Sen et al., J. Biol. Chem. 267, 5017 (1992), Mark et al. EP 192.811, EP 234.599, US 4588.585
   * IFN-γ(Gray et al., Nature 295, 503 (1982), Yip et al., PNAS USA 79,
- Leukemia inhibitory Factor (LIF)
   (Metcalf, Int. J. Cell Clon. 9, 85 (1991), Sutherland et al., Leuk. 3, 9 (1989), Gough et al., PNAS USA 85, 2623 (1988), Gough et al., Ciba Found. Symp. 167, 24 (1992), Stahl et al., J. Biol. Chem. 265, 8833 (1990), Rathjan et al., Cell 62, 1105 (1990))
- TNF
   (Porter, TiBTech 9, 158 (1991); Sidhu et al., Pharmac. Ther. 57, 79 (1993)) im speziellen
   * TNFα (Beutler et al., Nature 320, 584 (1986), Kriegler et al., Cell 53, 45 (1988))
   * TNFβ (Gray et al., Nature 312, 721 (1984), Li et al., J. Immunol. 138, 4496 (1987), Aggarwal et al., J. Biol. Chem. 260, 2334 (1985))
- TGFβ
   (Kehrl et al., J. Immunol. 137, 3855 (1986), J. Exp. Med. 163, 1037 (1986), Ten Dikje et al., PNAS USA 85, 4715 (1988), Derynck et al., EMBO J. 7, 3737 (1988), Massague, Ann. Rev. Cell Biol. 6, 597 (1990), Kondiah et al., J. Biol. Chem. 265, 1089 (1990), Garnier et al., J. Mol. Biol. 120, 97 1978))
- Oncostatin M
   (Brown et al., J. Immunol. 147, 2175 (1991); Grove et al., J. Biol. Chem. 266, 18194 (1991); Hamilton et al., Biochem. Biophys. Res. Commun. 180, 652 (1991), Malik et al., Mol. Cell. Biol. 9, 2847 (1989), Kallstad et al., J. Biol. Chem. 266, 8940 (1991)

Als Wirksubstanz im Sinne der Erfindung können jedoch auch DNA-Sequenzen von Fusionsproteinen zwischen den aufgeführten Cytokinen, Wachstumsfaktoren oder dem extrazellulären Teil der Rezeptoren zum einen und dem Fc-Teil des menschlichen Immunglobulins zum anderen Verwendung finden. Derartige DNA-Sequenzen und ihre Herstellung wurden in der EP 0464 633 A1 beschrieben.

Die Auswahl des Zellzyklusinhibitors ist abhängig vom Typ der Leukämie.
- So sind IL-4 und IL-6 besonders antiproliferativ wirksam bei der B-CLL (von Kooten et al., Leuk. Lymph. 12, 27 (1993)).
- TGFβ inhibiert bevorzugt die Lymphozytenproliferation (Kehrl et al., J. Immunol. 143, 1868 (1989)).
- TNF, insbesondere TNF∝, inhibiert myeloische Leukämiezellen (Porter, FEMS Microbiol. Immunol. 64, 193 (1990)) und Lymphomzellen (Sidhu et al., Pharm. Therp. 57, 79 (1993)).
- IFN-γ inhibiert Myelomzellen (Portier et al., Blood 81, 3076 (1993)).
- IFNα inhibiert die Haarzell-Leukämie (Gutterman, PNAS USA 91, 1198 (1994)), wie auch Non-Hodgkin Lymphome (Solal-Celigny et al, New Engl. J. Med. 329, 1608 (1993), CLL, T-CLL, CML und ALL (Gutterman, PNAS USA 91, 1198 (1994), Dorr, Drugs 45, 177 (1993)).
- LIF inhibiert die Proliferation von CML Zellen (Metcalf, Int. J. Cell Clon. 9, 95 (1991)).
- IL-10 induziert Apoptose in B-CLL-Zellen (Fluchinger et al., J. Exp. Med. 179, 91(1994)).

Andererseits können inbesondere IL-1, IL-2, IL-4, IL-12 oder Interferone durch Aktivierung von Immunzellen, benachbart den transduzierten Leukämiezellen eine Entzündungsreaktion auslösen (Fenton et al., Int. J. Immunopharm. 14, 401 (1992), Janssen et al., Cancer Immunol. Immunother. 39, 207 (1994), Kirchner, DMW 111, 64 (1986), Paul, Blood 77, 1859 (1991), Gateley et al., Cancer Invest. 11 500 (1993)), welche die Leukämiezellen abtötet.

Voraussetzung für die Verwendung der DNA-Sequenz eines der aufgeführten Cytokine als Zellzyklusinhibitor im Wirkstoff ist jedoch, daß vor Gabe des Wirkstoffes geprüft wurde, daß für die Leukämiezellen des jeweiligen Patienten der ausgewählte Zellzyklusinhibitor kein Wachstumsfaktor darstellt.

### 9.3. Kombination gleicher oder unterschiedlicher Wirksubstanzen für Leukämien

Gegenstand der Erfindung ist des weiteren ein Wirkstoff, in welchem eine Kombination der DNA-Sequenzen von zwei gleichen Zellzyklusinhibitoren (A,A) oder zwei unterschiedlichen Zellzyklusinhibitoren (A,B) vorliegt. Zur Expression beider DNA-Sequenzen ist vorzugsweise die cDNA einer "internal ribosome entry site" (IRES) als regulatorisches Element zwischengeschaltet.

Aktivator- | zellzyklus- | Wirksubstanz | internal | Wirksubstanz
sequenz | reguliertes | (Zellzyklus- | ribosome | (Zellzyklus-
(UAS) | Promotor- | inhibitor) | entry | inhibitor)
| modul | A | site | A oder B

Derartige IRES wurden beispielsweise von Montford und Smith (TIB 11, 179 (1995), Kaufman et al., Nucl. Acids Res. 19, 4485 (1991), Morgan et al., Nucl. Acids Res. 20, 1293 (1992), Dirks et al., Gene 128, 247 (1993), Pelletier und Sonenberg, Nature 334, 320 (1988) und Sugitomo et al., BioTechn. 12, 694 (1994) beschrieben.
So kann die cDNA der IRES-Sequenz des Poliovirus (Position < 140 bis ≥ 630 des 5' UTR) zur Verknüpfung der DNA des Zellzyklusinhibitor A (am 3' Ende) und der DNA des Zellzyklusinhibitors B (am 5' Terminus) verwendet werden.

Ein derartiger Wirkstoff weist je nach Kombination additive (A+A, A+b1) oder synergistische Wirkung im Sinne der Erfindung auf.

### 9.4. Auswahl des Liganden für Leukämien

Als Liganden für virale Vektoren oder nicht-virale Vektoren, beispielsweise in Polylysin-Ligand-Konjugaten, werden Substanzen bevorzugt, welche an die Oberfläche von Leukämiezellen binden. Hierzu gehören Antikörper oder Antikörperfragmente, gerichtet gegen Membranstrukturen von Leukämiezellen. Eine große Anzahl derartiger monoklonaler Antikörper sind bereits für diagnostische und therapeutische Verfahren beschrieben worden (Übersichten bei Kristensen, Danish Medical Bulletin 41, 52 (1994), Schranz, Therapia Hungarica 38, 3 (1990), Drexler et al., Leuk. Res. 10, 279 (1986), Naeim, Dis. Markers 7, 1 (1989), Stickney et al., Current Op. Oncol. 4, 847 (1992), Drexler et al., Blut 57, 327 (1988), Freedman et al., Cancer Invest. 9, 69 (1991)). Je nach Typ der Leukämie sind als Liganden beispielsweise folgende monoklonale Antikörper oder deren antigenbindende Antikörperfragmente geeignet:

| Zellen | Membranantigen | monoklonalen Antikörper beschrieben von |
|---|---|---|
| AML | CD13 - | Kaneko et al., Leuk. Lymph. 14, 219 (1994) Muroi et al., Blood 79, 713 (1992) |
| | CD14 | Ball, Bone Marrow Transplant. 3, 387 (1988) |
| | CD15 | Guyotat et al., Bone Marrow Trans plant. 6, 385 (1990) Campos et al., Eur. J. Cancer 28, 37 (1992) |
| | CD33 | Jurcic et al., Leukemia 9, 244 (1995), Caron et al., Cancer 73, 1049 (1994) |
| | CAMAL | Shellard et al., Exp. Hematol. 19, 136 (1991) |
| | Sialosyl-Le | Muroi et al., Blood 79, 713 (1992) |
| B-CLL | CD5 | Kaminski et al., Cancer Treat. Res. 38, 253 (1988) Tassone et al. Immunology Lett. 39, 137 (1994) |
| | CD1c CD23 | Orazi et al., Eur. J. Haematol. 47, 28 (1991) |
| | Idiotypen und Isotypen der Membranimmunglobuline | Schroeder et al., Immunol. Today 15, 289 (1994) |
| T-CLL | CD33 M38 | Imai et al., J. Immunol. 151, 6470 (1993) |
| | IL-2-Rezeptoren T-Zell-Rezeptoren | Waldmann et al., Blood 82, 1701 (1993) |
| ALL | CALLA | Morishima et al., Bone Marrow Transplant. 11, 255 (1993) |
| | CD19 Non-Hodgkin Lymphoma | Anderson et al., Blood 80, 84 (1993) Okazaki et al., Blood 81, 84 (1993) |

Die murinen monoklonalen Antikörper sind bevorzugt in humanisierter Form einzusetzen. Die Humanisierung erfolgt in der von Winter et al. (Nature 349, 293 (1991)) und Hoogenboom et al. (Rev. Tr. Transfus. Hemobiol. 36, 19 (1993)) dargestellten Weise. Antikörperfragmente werden entsprechend dem Stand der Technik hergestellt, beispielsweise in der von Winter et al., Nature 349, 293 (1991), Hoogenboom et al., Rev. Tr. Transfus. Hemobiol. 36, 19 (1993), Girol, Mol. Immunol. 28, 1379 (1991) oder Huston et al., Int. Rev. Immunol. 10, 195 (1993) beschriebenen Weise.

Zu den Liganden gehören desweiteren alle Wirkstoffe, welche an Membranstrukturen oder Membranrezeptoren von Leukämiezellen binden. Beispielsweise gehören hierzu Wachstumsfaktoren oder deren Fragmente bzw. Teilsequenzen von ihnen, die an Rezeptoren exprimiert durch Leukämiezellen binden.

Derartige Wachstumsfaktoren wurden bereits beschrieben (Übersichten bei Cross et al., Cell 64, 271 (1991), Aulitzky et al., Drugs 48, 667 (1994), Moore, Clin. Cancer Res. 1, 3 (1995), Van Kooten et al., Leuk. Lymph. 12, 27 (1993)). Beispielsweise gehören zu ihnen:
- IFNα bei Non-Hodgkin Lymphomen
   (Hiddemann et al., Blood Rev. 8, 225 (1994))
- IL-2, besonders bei T-Zell-Leukämien
   (Waldmann, J. Nat. Cancer Inst. 81, 914 (1989), Kreitman et al., Int. J. Immunopharm. 14, 465 (1992)
- FGF bei T-Zell-, monozytären, myeloiden, erythroiden und
   megakaryoblastischen Leukämien (Armstrong et al., Cancer Res. 52, 2004 (1992)
- TGFβ bei Leukämien
   (Keller et al. J. Cell Biochem. 39, 79 (1989))
- Retinoide, z.B. "Retinoic acid" bei akuter promyelozytärer Leukämie
   (Cornic et al., Anticancer Res. 14, 2339 (1994)).

### 9.5. Herstellung des Wirkstoffes für Leukämien

Die Herstellung des Wirkstoffes wird an folgenden Beispielen verdeutlicht:

### a) Konstruktion des chimären Promotors Myc E-Box-CDE-CHR-Inr

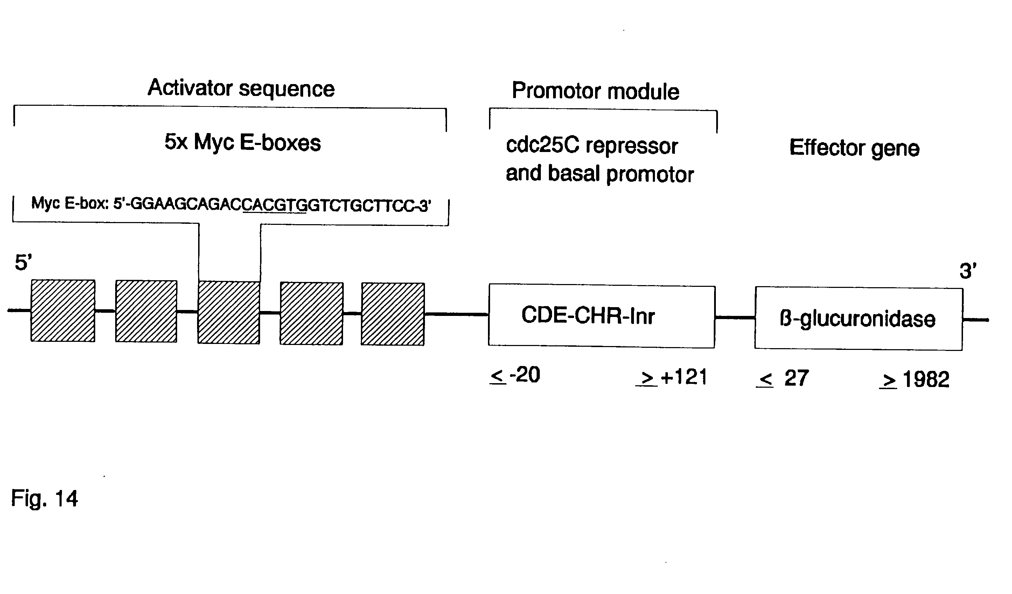

5 Kopien der humanen Myc E-Box (Nukleotidsequenz 5'-GGAAGCAG-ACCACGTGGTCTGCTTCC-3'; Blackwood und Eisenman, Science 251, 1211 (1991)) werden in 5'-3' Orientierung miteinander verknüpft und an ihrem 3' Ende mit dem 5'Terminus des CDE-CHR-Inr Moduls des humanen cdc25-Gens (Position -20 bis >_ +121 der von Lucibello et al., EMBO J. 14, 132 (1995)) verbunden (siehe Fig. 14).

Die Verknüpfungen erfolgen mit Hilfe von dem Fachmann bekannten und käuflichen Enzymen. Die so hergestellte chimäre Myc E-Box-Promtormodul-Transkriptionseinheit wird an ihrem 3' Ende mit dem 5' Terminus einer DNA, die den kompletten kodierenden Bereich der humanen β-Glucuronidase (DNA Position ≤ 27 bis ≥ 1982, der von Oshima et al., PNAS USA 84, 684 (1987)) enthält, verknüpft (siehe Fig. 14).

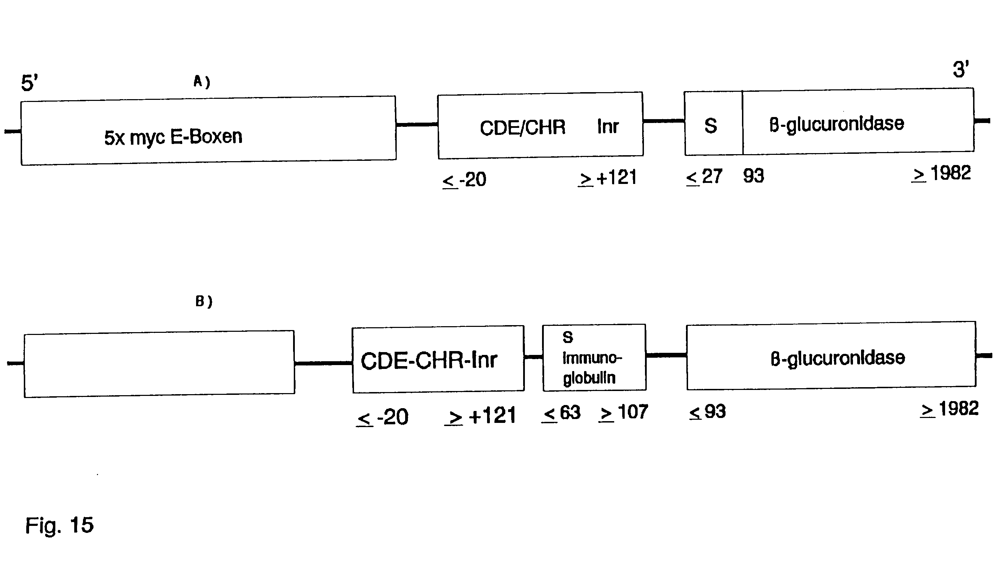

Diese DNA enthält auch die für eine Sekretion notwendige Signalsequenz (22N-terminale Aminosäure). Zur Erleichterung der zellulären Ausschleusung ist diese Signalsequenz bevorzugt auszutauschen gegen die Signalsequenz des Immunglobulins (Position < 63 bis ≥ 107; Riechmann et al., Nature 332, 323 (1988), siehe Fig. 15). Transkriptionskontrolleinheiten und die DNA für die humane β-Glucuronidase werden in pUC18/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### 10) Wirksamkeit des Wirkstoffes

Ein Wirkstoff gemäß der vorliegenden Erfindung ermöglicht nach lokaler (z.B. in Gewebe, Körperhöhlen, Magen-Darm-Trakt, Gewebezwischenräumen, Gelenkspalten oder Cytokine) oder nach systemischer, bevorzugt intravenöser oder intraarterieller Gabe eine vorwiegende, wenn nicht ausschließliche Wirkung an den Zielzellen, da durch die Kombination aus gewebespezifischer Aktivatorsequenz und zellzyklusreguliertem Promotormodul gewährleistet ist, daß die Wirksubstanz überwiegend oder ausschließlich in sich teilenden Zielzellen exprimiert wird.

So kann eine langanhaltende Behebung von haematopoietischen Cytopenien und eine deutliche Linderung von Allergien und Autoimmunerkrankungen erreicht werden. Bei chronischen Gelenkentzündungen bewirkt die intraartikuläre Injektion des Wirkstoffes eine Hemmung der Synovialzellproliferation. Bei chronischen Virusinfektionen ist durch den Wirkstoff eine Therapie möglich. Desweiteren bietet der Wirkstoff eine wirksame und sichere Möglichkeit der Impfung gegen Infektionserreger. Bei Leukämien ist die Chance für eine therapeutische Wirkung gegeben.

Da der Wirkstoff sowohl durch seine Zell- als auch Zellzyklusspezifität ein hohes Maß an Sicherheit verspricht, kann er auch in hohen Dosierungen und, falls notwendig, mehrmals in Abständen von Tagen, Wochen oder Monaten zur Prophylaxe oder zur Therapie angewandt werden.

Legende zu Fig. 1 - 15:

Fig. 1:
Nukleotidsequenz des cdc25C - Promotorbereichs mit den *in vivo* gefundenen Proteinbindungsstellen (genomisches DMS Footprinting; 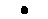 (gefüllte Kreise): vollständiger konstitutiver Schutz; o (offene Kreise): partieller konstitutiver Schutz; * (Sternchen): zellzyklusregulierter, G1-spezifischer Schutz). CBS: constitutive binding site; CDE: cell cycle-dependent element. Grau unterlegte Bereiche zeigen die Y_{c}-Boxen (NF-Y Bindestellen) an. Startstellen sind durch gefüllte Quadrate markiert.

Fig. 2:
Derepression des cdc25C-Promotors spezifisch in G₀ durch Mutation des cdc.

Fig. 3:
Schematische Darstellung der cdc25C Enhancer-Regulation durch das CDE.

Fig. 4:
G₀ / G₁ - spezifische Repression des SV40-Enhancers durch das CDE.

Fig. 5:
Homologien im CDE-CHR-Bereich und den 5' gelegenen Yc-Boxen in den cdc25C, cyclin A und cdc2-Promotoren

Fig. 6:
Chimäre Promotoren zur Expression von Thrombopoietin Positionsangaben beziehen sich auf folgende Literatur:
- SCF-Rezeptor-Promotor:: Yamamoto et al., Jpn. J. Cancer Res. 84, 1136(1993)
- IL-1-Rezeptor-Promotor:: Ye et al., PNAS USA 90, 2295 (1993)
- IL-3-Rezeptor (α)-Promotor:: Miyajima et al., Blood 85, 1246 (1995)
- GM-CSF-Rezeptor (α)-Promotor:: Nakagawa et al., J. Biol. Chem. 269, 10905 (1994)
- β-Kette (IL-3-Rez./GM-CSF):: Gorman et al., J. Biol. Chem. 267, 15842 (1992)
- CDE-CR-Inr:: Lucibello et al., EMBO J. 14, 132 (1995)
- IL-3:: Yang et al., Cell 47, 3 (1986)
- internal ribosome entry site:: Pelletier und Sonenberg, Nature 334, 320 (1988)
- Thrombopoietin:: de Sauvage et al., Nature 369, 533 (1994)

Fig. 7:
Chimäre Promotoren für die Prophylaxe oder Therapie von Autoimmunerkrankungen und/oder Allergien.
Positionsangaben beziehen sich auf folgende Literatur:
- IL-2-Promotor:: Williams et al., J. Immunol. 141, 662 (1988)
- IL-1-Rezeptor-Promotor:: Ye et al., PNAS USA 90, 2295 (1993)
- CDE-CHR-Inr:: Lucibello et al., EMBO J. 14, 132 (1995)
- IL-10:: Moore et al., Science 248, 1230 (1990)
- internal ribosome entry site:: Pelletier und Sonenberg, Nature 334, 320 (1988)
- β-Glucuronidase:: Oshima et al., PNAS USA 84, 685 (1985)
- Signalsequenz Immunglobulin:: Riechmann et al., Nature 332. 323 (1988)

Fig. 8:
Schematische Darstellung der Exonuklease III-Verkürzungen des 5'-flankierenden TIMP-3-Genbereichs. Zur Erleichterung der Sequenzierung wurden annähernd 1600 bp des 5'-flankierenden TIMP-3-Genbereichs durch Behandlung mit Exonuklease III vom 5'-Ende her verkürzt und in den Bluescript SK(-)-Vektor kloniert. Die Namen der Plasmide bezeichnen ihre 5'-Verkürzung (z.B. Δ-1300 enthält 1300 bp 5' der Transkriptionsstartstelle). Die Transkriptionsstartstelle ist mit +1 markiert.

Fig. 9:
Nukleotidsequenz von 500 bp menschlichen TIMP-3-Promotors sowie 101 bp der 5'-untranslatierten Region. GC-Boxen (Sp1-Bindungsstellen), zwei Halbseiten einer möglichen NF1-Bindungsstelle sowie ein der C/EBP-Bindungsstelle ähnelndes Element sind markiert. Die Transkriptionsstartstelle ist durch einen Pfeil gekennzeichnet.

Fig. 10:
Induktionskinetik des Δ-1010-TIMP-3-Promotor-Luziferase-konstrukts nach Stimulation ruhender NIH3T3-Zellen mit 20 % FKS.
Graphische Darstellung. Nach DEAE-Transfektion von 7 µg Plasmid-DNA wurden die NIH3T3^{RT}-Zellen für 40 h in serumfreiem Medium inkubiert, mit 20 % FKS stimuliert und zu den angegebenen Zeitpunkten die Expression des Luziferase-Reportergens bestimmt.

Fig. 11:
Transiente Expressionsanalyse 5'-verkürzter TIMP-3-Promotor-Luziferasekonstrukte in normal wachsenden, ruhenden und serumstimulierten NIH3T3^{RT}-Zellen.
Die Plasmide wurden entsprechend ihrer Verkürzungen bezeichnet (s. Fig. 8)
(a) Analyse in normal wachsenden NIH3T3-Zellen
(b) Analyse der gleichen Konstrukte wie in (a) in ruhenden versus für 4 h mit 20 % FKS stimulierten NIH3T3-Zellen.
Die Experimente in (a) und (b) wurden, wie in Tab. 1 beschrieben, dreimal mit unabängig voneinander präparierten Plasmid-DNAs durchgeführt. Standardabweichungen sind als Balken dargestellt. Ein Fehlen der Balken indiziert eine sehr niedrige, im Graph nicht mehr darstellbare Standardabweichung.

Fig. 12:
Chimäre Konstrukte bestehend aus verschiedenen Anteilen des humanen TIMP-3 Promotors, dem 3' fusionierten Promotormodul mit den CDE und CHR Repressorelementen sowie einer DNA für den IL-1-Rezeptor-Antagonisten (kompletter kodierender Bereich) als Effektor. Positionsangaben beziehen sich auf die Hauptstartstelle des TIMP-3 Gens, auf das von Lucibello et al. (EMBO J. 15, 132 (1995)) verwendete System für cdc25C bzw. auf Positionen in der IL-1-Rezeptor-Antagonist DNA (Eisenberg et al., Nature 343, 341 (1990)).

Fig. 13:
Chimäre Promotoren für die Therapie der HIV-Infektion
Die Positionsangaben beziehen sich auf folgende Literatur:
- HIV-LTR: (Rosen et al., Cell 41, 813 (1985))
- CDE-CHR-Inr: (Lucibello et al., EMBO J. 14, 132 (1995))
- IFN α: (Streuli et al., Science 209, 1343 (1980))
- IRES: (Pelletier und Sonenberg, Nature 334, 320 (1988))
- RBP9-27: (Reid, PNAS USA 86, 840 (1989))

Fig. 14:
Chimäre Konstrukte bestehend aus 5 Myc E-Boxen (Blackwood und Eisenman, Science 251, 1211 (1991)), dem 3' fusionierten cdc25C Basalpromotor mit den CDE und CHR Repressorelementen sowie einer DNA für β-Glucuronidase (kompletter kodierender Bereich) als Effektor.
Positionsangaben beziehen sich auf das von Lucibello et al. (1995) verwendete System für cdc25C bzw. auf Positionen in der β-Glucuronidase DNA (Oshima et al., 1987).

Fig. 15:
Positionsangaben der Signalsequenz (MGWSCIILFLVATAT) des Immunglobulins (HuVHCAMP) beziehen sich auf Riechmann et al., Nature 332, 323 (1988)
B) Alternative: Einbau des Signalpeptids des Ig zur besseren extrazellulären Ausschleusung der β-glucuronidase

### Tabelle 1: Rolle von CDE und CHR bei der zellzyklusregulierten Transkription von cdc25C, cyclin A und cdc2

**Tab. 1**

| | ***G***_{***0***} | ***Growing*** | ***Factor*** |
|---|---|---|---|
| ***wt*** | | | |
| cdc25C | 0.8 | 13.1 | 17.5 |
| | | | |
| cyclin A | 0.7 | 27.1 | 41.7 |
| cdc2 | 1.0 | 41.2 | 41.2 |
| ***mCDE(-13)*** | | | |
| cdc25C | 7.6 | 11.6 | 1.5 |
| | | | |
| cyclin A | 13.4 | 23.9 | 1.8 |
| cdc2 | 11.3 | 33.9 | 3.0 |
| ***mCHR(-6/-3)*** | | | |
| cdc25C | 14.4 | 21.0 | 1.5 |
| | | | |
| cyclin A | 15.5 | 28.3 | 1.8 |
| cdc2 | 18.6 | 38.6 | 2.1 |

Ergebnisse transienter Transfektionen in HIH3T3-Zellen sind als RLUs/1000 dargestellt. mCDE: mutiertes CDE (Pos. -13: G → T); mCHR: mutiertes CHR (Pos. -6 bis -3).

### Tab. 2: Expression verschiedener Promotor-Luziferase-Konstrukte in normal proliferierenden (A) und serumstimulierenden (B) NIH3T3-Zellen. Nach DEAE-Transfektion von 7µ g Plasmid-DNA wurden die NIH3T3-Zellen für 40 h in serumhaltigem (A) oder serumfreien (B) Medium inkubiert, für 4 h mit 20% FKS stimuliert (B) und die Expression des Luziferase-Reportergens bestimmt.

**Tabelle 2:**

| A. **Expression in proliferierenden Zellen** | |
|---|---|
| (RLUx10³) | |
| TIMP-3Δ-1010 | 189,5 ± 6,4 |
| pRSV-LTR | 308,1 ± 23,4 |
| p5xTRE | 59,1 ± 2,2 |
| Cyclin D1 Δ-973 | 27,8 ± 3,5 |
| pXP2 | 0,2 |

| B. **Relative Induktion durch 20% FKS in FKS-stimulierten versus G0-Zellen** | |
|---|---|
| (Faktor) | |
| TIMP-3Δ-1010 | 8,4 ± 0,4 |
| p5xTRE | 2,4 ± 0,2 |
| Cyclin D1 Δ-973 | 3,5 ± 0,3 |
| pT81 | 1,0 ± 0,2 |
| pXP2 | 1,2 |

## Patentansprüche

1. Wirkstoff zur Prophylaxe oder Therapie von Erkrankungen, die durch das Immunsystem bedingt sind dadurch charakterisiert, daß er ein DNA-Konstrukt enthält, welches aus einer Aktivatorsequenz, einem zellzyklusreguliertem Promotormodul und einer DNA-Sequenz für die Wirksubstanz besteht.

2. Wirkstoff nach Anspruch 1), bei welchem das Promotormodul die Elemente CDE-CHR-Inr besitzt und die Positionen ≤ -20 bis ≥ +30 des cdc25C Promotorbereiches enthält (Nukleotidsequenz GGCTGGCGGAAGGTTT-GAATGGTCAACGCCTGCGGCTGTTGATATTCTTG), wobei CDE das "cell cycle dependent element (Nukleotidsequenz TGGCGG), CHR die cell cycle gene homology region (Nukleotidsequenz GTTTGAA) und Inr die Initiationsstelle (Position +1) sowie die für die Initiation wichtigen benachbarten Sequenzen darstellt.

3. Wirkstoff nach Anspruch 1), enthaltend eine Aktivatorsequenz (Promotor- oder Enhancersequenz), welche durch Transkriptionsfaktoren, im besonderen Maße gebildet in Zellen des blutbildenden Systems, in Synovialzellen, in virusinfizierten Zellen, in Parasiten, in Makrophagen, in Lymphozyten oder in Leukämiezellen reguliert wird.

4. Wirkstoff nach Anspruch 3), enthaltend die CMV-Promotorsequenz, die CMV-Enhancersequenz oder die SV40-Promotorsequenz.

5. Wirkstoff nach Anspruch 3) zur Therapie einer unzureichenden Bildung von Blutzellen, bei welchem die Aktivatorsequenz die Promotorsequenz eines Gens für ein Cytokin oder des Rezeptors für dieses Cytokin darstellt, welches in gering- oder undifferenzierten Zellen des blutbildenden Systems oder in direkt benachbarten Stromazellen aktiviert ist.

6. Wirkstoff nach Anspruch 5), enthaltend die Promotorsequenz für Stem Cell Factor, Interleukin (IL)-1α, IL-1β, IL-3, IL-6, LIF oder Granulozyten-Makrophagen-koloniestimulierenden Faktor oder die Promotorsequenz der Rezeptoren für Stem Cell Factor, IL-1 oder IL-3, IL-6, LIF, GM-CSF oder die Promotorsequenz für Interferon Regulatory Factor 1 (IRF-1).

7. Wirkstoff nach Anspruch 3) zur Therapie von Autoimmunerkrankungen, Allergien, Entzündungen und zur Verhütung von Organabstoßungen, bei welchem die Aktivatorsequenz die Promotorsequenz eines Gens für ein Protein darstellt, das bei Aktivierung von Makrophagen oder von Lymphozyten verstärkt gebildet wird.

8. Wirkstoff nach Anspruch 7), enthaltend die Promotorsequenz für Interleukin (IL)-1α oder IL-1β, IL-1-Rezeptor, IL-2, IL-2-Rezeptor, Interferon γ, IL-3, IL-3-Rezeptor, IL-4, IL-4Rezeptor, IL-5, IL-6, Interferon Regulatory Factor 1, IFN-γ Responsive Promotor, IL-7, IL-8, IL-9, IL-10, IL-11, IL-12, IL-13, Granulozyten-Makrophagen-koloniestimulierender Faktor (GM-CSF), GM-CSF-Rezeptor, Granulozyten-koloniestimulierender Faktor (G-CSF), Makrophagen-Colony Stimulating Factor Rezeptor, Makrophagen Scavenger Typ I oder Typ II Rezeptor oder Leukämie-inhibierender Faktor (LIF), LFA-1, MAC-1 oder p150,95.

9. Wirkstoff nach Anspruch 1) zur Therapie der Arthritis, enthaltend eine Aktivatorsequenz (Promotor- oder Enhancersequenz), welche durch Transkriptionsfaktoren, gebildet in Synovialzellen oder Entzündungszellen, reguliert wird.

10. Wirkstoff nach Anspruch 9), enthaltend die Promotorsequenz für ein Matrixmetalloproteinase-Gen (MMP), ein Tissue Inhibitor of Metalloproteinases (TIMP)-Gen oder für den M-CSF Rezeptor oder Makrophagen Scavenger Typ I oder Typ II Rezeptor.

11. Wirkstoff nach Anspruch 10), enthaltend die Promotorsequenz für das MMP-1-, MMP-2-, MMP-3-, MMP-9-, TIMP-1- oder TIMP-2-Gen.

Wirkstoff nach Anspruch 10), enthaltend die Promotorsequenz für das Gen des Tissue Inhibitor of Metalloproteinase-3,
enthaltend promotoraktive DNA-Fragmente, enthaltend Position ≤ -463 bis ≥ -2 der folgenden Nukleotidsequenz:

-500 ATGGCTTCCC ATATCCCAGA GAGTAAGAAC CAGAGAGAGA GAGAGAAAGA GAGAGAGTTT
-440 GGGTCTTTCT CCTCTGTGCC TGCTCTCTCC AGAGAAACTG GAGGGGTAGC AGTTAGCATT
-380 CCCCCGCTGG TTCCACCAAG CACAGTCAAG GTCTCTAGGA CATGGCCACC CCTCACCTGT
-320 GGAAGCGGTC CTGCTGGGGT GGGTGGGTGT TAGTTGGTTC TGGTTTGGGT CAGAGACACC

NF1
-260 CAGTGGCCCA GGTGGGCGTG GGGCCAGGGC GCAGACGAGA AGGGGCACGA GGGCTCCGCT

-200 CCGAGGACCC AGCGGCAAGC ACCGGTCCCG GGCGCGCCCC AGCCCACCCA CTCGCGTGCC

Sp1 Sp1
-140 CACGGCGGCA TTATTCCCTA TAAGGATCTG AACGATCCGG GGGCGGCCCC GCCCCGTTAC

Sp1 C/EBP
-80 CCCTTGCCCC CGGCCCCGCC CCCTTTTTGG AGGGCCGATG AGGTAATGCG GCTCTGCCAT

Sp1 ↓Start
-20 TGGTCTGAGG GGGCGGGCCC CAACAGCCCG AGGCGGGGTC CCCGGGGGCC CAGCGCTATA

13. Wirkstoff nach Anspruch 12), enthaltend promotoraktive DNA-Fragmente für das Gen des Tissue Inhibitor of Metalloproteinase-3, enthaltend
- Position ≥ -463 bis ≤ -10
oder
- Position ≥ -112 bis ≤ - 2
oder
- Position ≥ -112 bis ≤ -10
der unter Anspruch 12) aufgeführten Nukleotidsequenz.

14. Wirkstoff nach Anspruch 1-3) zur Therapie von Virusinfektionen, bei welchem die Aktivatorsequenz die Promotorsequenz für Viren darstellt, die die von ihnen infizierten Zellen transformieren und zur Proliferation anregen.

15. Wirkstoff nach Anspruch 14), enthaltend die Promotorsequenz des Hepatitis B Virus, Hepatitis C Virus, Herpes Simplex Virus I und II, der humanen Papillomaviren, insbesondere HPV-16 und HPV-18, des humanen immundefizienten Virus, im besonderen HIV-1, HIV-2 und HIV-3, des Epstein-Barr Virus oder des humanen T-Zell-Leukämie-Virus.

16. Wirkstoff nach Anspruch 1) zur Prophylaxe von Infektionen, enthaltend die Promotorsequenz für Interleukin (IL)-1α oder IL-1β, IL-1-Rezeptor, IL-2, IL-2-Rezeptor, Interferon γ, IL-3, IL-3-Rezeptor, IL-4, IL-4-Rezeptor, IL-5, IL-6, Interferon Regulatory Factor 1, IFN-γ Responsive Promotor IL-7, IL-8, IL-9, IL-10, IL-11, IL-12, IL-13, Granulozyten-Makrophagen-koloniestimulierender Faktor (GM-CSF), M-CSF Rezeptor, Makrophagen Scavenger Typ I oder Typ II Rezeptor, GM-CSF-Rezeptor, Granulozyten-koloniestimulierender Faktor (G-CSF) oder Leukämie-inhibierender Faktor (LIF), LFA-1, MAC-1 oder p150,95.

17. Wirkstoff nach Anspruch 1) zur Therapie von Leukämien, enthaltend eine Aktivatorsequenz (Promotor- oder Enhancersequenz), welche durch Transkriptionsfaktoren, gebildet in Leukämiezellen, aktiviert wird.

18. Wirkstoff nach Anspruch 17), enthaltend
- die Promotorsequenz für c-myc, bcl-2, bcl-1, IL-6, IL-10, TNFα oder TNFβ oder
- die Bindungssequenz für Proteine, gebildet in HOX-11, BCR-Abl, E2A-PBX-1, PML/RARA oder
- die humane myc E-Box in einfacher oder mehrfacher Form.

19. Wirkstoff nach Anspruch 5), bei welchem die DNA-Sequenz für die Wirksubstanz Erythropoietin, G-CSF, GM-CSF, IL-3, LIF, IL-11 und/oder Thrombopoietin kodiert.

20. Wirkstoff nach Anspruch 7), bei welchem die DNA-Sequenz für die Wirksubstanz
- IFNα, IFNβ, IFN γ, IL-10, lösliche IL-4-Rezeptoren, TGFβ, IL-12, lösliche IL-1-Rezeptoren, lösliche IL-2-Rezeptoren, lösliche IL-6-Rezeptoren, IL-1-Rezeptorantagonisten, IL-1, IL-4, IL-6, IL-9, IL-13, TNFα oder TNFβ kodiert oder
- einen immunsuppressiven Antikörper, im besonderen mit Spezifität für CD4, CD8, CD3, IL-2-Rezeptor, IL-1-Rezeptor oder IL-4-Rezeptor, CD2, LFA-1, CD28, CD40-Linker oder CD40 kodiert oder
- Fragmente dieses Antikörpers, die VH und VL enthalten oder dessen über einen Linker verbundene VH und VL Fragmente darstellen kodiert.

21. Wirkstoff nach Anspruch 9), bei welchem die DNA-Sequenz für eine antientzündliche Substanz
- einen Inhibitor für entzündungserregende Interleukine oder Wachstumsfaktoren oder
- ein entzündungshemmendes Interleukin oder
- einen Wachstumsfaktor, welcher die Synthese der extrazellulären Matrix stimuliert oder
- die Superoxiddismutase oder
- einen Proteinaseinhibitor kodiert.

22. Wirkstoff nach Anspruch 21), bei welchem
- der Inhibitor für entzündungserregende Interleukine oder Wachstumsfaktoren der lnterleukin-1-Rezeptorantagonist, der lösliche Interkeukin-1-Rezeptor oder der lösliche Tumor Necrosis Factor α (TNFα) Rezeptor ist oder
- das entzündungshemmende Interleukin IL-4, -6 oder -10 ist oder
- der Wachstumfaktor, welcher die Synthese der extrazellulären Matrix stimuliert, Insulin-like Growth Factor oder Transforming Growth Factor β (TGFβ) ist oder
- der Proteinaseinhibitor Tissue Inhibitor of Metalloproteinase-1 (TIMP-1), TIMP-2 oder TIMP-3 darstellt.

23. Wirkstoff nach Anspruch 14), bei welchem die DNA-Sequenz für die Wirksubstanz
- IFNα, IFNβ, IFN γ, TNF, im besonderen TNFα IL-1 oder TGFβ kodiert oder
- einen Antikörper einer Spezifität kodiert, die das jeweilige Virus inaktiviert oder
- ein VH und VL enthaltendes Fragment dieses Antikörpers oder dessen über einen Linker verbundene VH und VL Fragment kodiert oder
- ein Rev-bindendes Protein kodiert, im besonderen RBP9-27, RBP1-8U oder RBP1-8D.

24. Wirkstoff nach Anspruch 16), bei welchem die DNA-Sequenz für die Wirksubstanz
- das Neutralisations- oder Inaktivierungsantigen eines Virus, eines Bakteriums oder eines Parasiten kodiert oder
- für einen Antiidiotyp-Antikörper oder ein Fragment dieses Antikörpers, dessen "complementarity determining regions" eine Kopie der Protein- oder Kohlenhydratstruktur des Neutralisationsantigens eines Infektionserregers ist.

25. Wirkstoff nach Anspruch 24), bei welchem die DNA-Sequenz kodiert für die Antigene von Influenza, HIV, Tollwut, HSV, RSV, Parainfluenza-V, Rotavirus, VZV, CMV, Masern-V, HPV, HBV, HCV, HDV, HEV, HAV, Vibriocholera-Toxin, Borrelia-Burgdorferi, Helicobacter pylori oder Malaria.

26. Wirkstoff nach den Ansprüchen 7), 14) und 17), bei welchen die Wirksubstanz eine DNA-Sequenz für einen Zellzyklusinhibitor darstellt.

27. Wirkstoff nach Anspruch 26), bei welchem die DNA-Sequenz für den Zellzyklusinhibitor
- das Retinoblastomprotein pRb/p110 oder für die verwandten p107 und p130 Proteine oder
- das p53 Protein oder
- das p21 Protein, das P16 Protein oder einen anderen "Cyclin-dependent Kinase (cdK)"- Inhibitor oder
- das GADD45 Protein oder
- das bak Protein oder
- ein zytotoxisches Protein oder
- ein zytostatisches Cytokin oder oder
- ein Enzym für die Spaltung von Vorstufen von Zytostatika in Zytostatika kodiert.

28. Wirkstoff nach Anspruch 27),
- bei welchem die DNA-Sequenz für das Retinoblastomprotein (pRb/p110) durch Austausch der Aminosäuren in den Positionen 246, 350, 601, 605, 780, 786, 787, 800 und 804 nicht mehr phosphorylierbar wird, ohne daß jedoch das mutierte pRb/p110 seine Bindungsaktivität mit dem großen T-Antigen einbüßt, im besonderen, daß die Aminosäuren Thr-246, Ser-601, Ser-605, Ser-780, Ser-786, Ser-787 und Ser-800 mit Ala, der Aminosäure Thr-350 mit Arg und der Aminosäure Ser-804 mit Glu ausgetauscht werden oder
- bei welchen die DNA-Sequenz für das p107 oder das P130 Protein in analoger Weise wie beim mutierten pRb/p110 mutiert ist.

29. Wirkstoff nach Anspruch 27), bei welchem die DNA-Sequenz für das Protein p53 C-terminal verkürzt ist um das Serin 392.

30. Wirkstoff nach Anspruch 27), bei welchem die DNA-Sequenz für das zytotoxische Protein bzw. zytostatische Cytokin Perforin, Granzym. IL-2, IL-4, IFNα, IFNβ, IFNγ, TNFα, TNFβ, TGFβ oder Oncostatin M kodiert.

31. Wirkstoff nach Anspruch 27), bei welchem das Enzym eine Herpes Simplex Virus Thymidin-Kinase, Cytosindeaminase, Varizella Zoster Virus ThymidinKinase, Nitroreduktase, β-Glucuronidase (im besonderen eine humane, pflanzliche oder bakterielle β-Glucuronidase), Carboxypeptidase (vorzugsweise von Pseudomonas), Lactamase (vorzugsweise von Bazillus cereus), Pyroglutamat-Aminopeptidase, D-Aminopeptidase, Oxidase, Peroxidase, Phosphatase, Hydroxynitrillyase, Protease, Esterase oder eine Glycosidase ist, wobei die homologe Signalsequenz oder zur besseren zellulären Ausschleusung eine heterologe Signalsequenz verwendet wird.

32. Wirkstoff nach Anspruch 31), bei welchem durch Punktmutationen der DNA-Sequenz des Enzyms die lysosomale Speicherung verringert und die extrazelluläre Ausschleusung erhöht ist.

33. Wirkstoff nach Anspruch 19-32), welcher die DNA-Sequenzen von mehreren gleichen oder unterschiedlichen Wirksubstanzen enthält, wobei zwei DNA-Sequenzen durch eine DNA-Sequenz für die internal ribosome entry site miteinander verbunden sind.

34. Wirkstoff nach Anspruch 33), bei welchem eine Wirksubstanz das Antigen oder den Antiidiotyp-Antikörper für dieses Antigen von einem Infektionserreger kodiert und die zweite Wirksubstanz ein Cytokin oder einen Cytokinrezeptor kodiert, im besonderen
- TGFβ, IFNα, IFNβ, IFN γ, IL-12 oder lösliche IL-4-Rezeptoren oder
- IL-4, IL-6, LIF, IL-9, IL-10, IL-13, TNFα oder TNFβ oder
- IL-1, IL-2, M-CSF oder GM-CSF.

35. Wirkstoff nach Anspruch 1-34), eingefügt in einen Vektor.

36. Wirkstoff nach Anspruch 35), bei welchem der Vektor ein Virus ist.

37. Wirkstoff nach Anspruch 36), bei welchem das Virus ein Retrovirus, Adenovirus, Adeno-assoziiertes Virus, Herpes Simplex Virus oder Vaccinia Virus darstellt.

38. Wirkstoff nach Anspruch 1-34) eingefügt in ein Plasmid.

39. Wirkstoff nach Anspruch 35-38), zubereitet in einem kolloidalen Dispersionssystem.

40. Wirkstoff nach Anspruch 39), bei welchem das kolloidale Dispersionssystem Liposomen sind.

41. Wirkstoff nach Anspruch 39), bei welchem das kolloidale Dispersionssystem Polylysin-Liganden sind.

42. Wirkstoff nach Anspruch 1-41) ergänzt um einen Liganden, welcher an Membranstrukturen von haematopoietischen Zellen, an aktivierten Lymphozyten, aktivierten Makrophagen, aktivierten Synovialzellen, virusinfizierten Zellen oder an Leukämiezellen bindet.

43. Wirkstoff nach Anspruch 42), bei welchem der Ligand
- ein polyklonaler oder monoklonaler Antikörper oder ein Antikörperfragment hiervon ist, der mit seinen variablen Domänen spezifisch an die Zellmembran bindet oder
- ein polyklonaler oder monoklonaler Antikörper oder ein Antikörperfragment hiervon ist, der mit seinen konstanten Domänen an Fc-Rezeptoren auf der Zellmembran bindet oder
- ein Cytokin oder Wachstumsfaktor oder ein Fragment bzw. eine Teilsequenz hiervon ist, der an die entsprechenden Rezeptoren auf der Zellmembran bindet oder
- ein endständig Galactose enthaltender Ligand ist, der den Asialoglycoproteinrezeptor bindet oder
- ein Transferrin oder Fragment hiervon ist, das an den Transferrin-Rezeptor bindet oder
- ein Insulin oder ein Fragment hiervon ist, das an den Insulin-Rezeptor bindet oder
- ein endständiger, Mannose enthaltender Ligand ist, der an den Mannose-6-Phosphat-Rezeptor bindet.

44. Wirkstoff nach Anspruch 43), bei welchem die Zellmembranstrukturen Rezeptoren für Cytokine, Wachstumsfaktoren, Interferone, Chemokine darstellen, inbesondere Rezeptoren für den Stem Cell Factor, für IL-1, IL-2, IL-3, IL-4, IL-6, IL-8, IL-10, Interferon α, Interferon β, Interferon γ, LIF, GM-CSF, G-CSF, TNF∝, EGF, FGF, TGFβ, PDGF oder IGF.

45. Wirkstoff nach Anspruch 21), bei welchem die Membranstruktur von Synovialzellen oder Entzündungszellen Vimentin, Fibronectin, IL-1-Rezeptor, IL-2-Rezeptor, TNFα-Rezeptor, IL-4-Rezeptor, IL-10-Rezeptor, IGF-Rezeptor, TGFβ-Rezeptor oder Mannose-6-Phosphatrezeptor ist.

46. Wirkstoff nach Anspruch 43), bei welchem die Zellmembranstrukturen Antigene sind, die durch die Infektion mit Viren hervorgerufen werden, insbesondere durch HBV, HCV, HAV, HSV, HPV, EBV, HTLV oder HIV.

47. Wirkstoff nach Anspruch 43), bei welchem die Membranstruktur von Leukämiezellen Rezeptoren für IFNα, IL-2, FGF, TGFβ oder für Retinoide sind.

48. Wirkstoff nach Anspruch 1-47), bei welchem der Vektor gemischt wird mit einem Cytokin, einem Cytokinrezeptor, einem Adjuvans oder mit Substanzen, welche die Aufnahme und Immunisierung über die Schleimhaut erleichtern.

49. Wirkstoff nach Anspruch 48), bei welchem zugemischt wurde
- IL-1α, IL-1β, IL-2, IL-3, IL-4, IL-5, IL-6, IL-10, IL-12, IFN-γ, M-CSF, GM-CSF und/oder IL-4-Rezeptor oder
- Liposomen, bioabbaubare Polymeren, Muramyldipeptide, Lipopolysaccharide, Lipid A, oder Al(OH)₃ oder Ca(OH)₃.

50. Wirkstoff nach Anspruch 1-49) in einer Arzneimittelzubereitung für die Injektion in ein Gewebe wie Muskel, Bindegewebe, Leber, Niere, Milz, Lunge, Haut, für die lokale Applikation auf die Haut oder auf Schleimhäute, für die Injektion in Körperhöhlen wie Gelenke, Pleuralraum, Peritonealraum, Subarachinoidalraum oder für die Injektion in das Blutgefäßsystem, wie intraarterielle oder intravenöse Injektion oder für die orale Aufnahme.

## Claims

1. An active compound for the prophylaxis or therapy of diseases which are associated with the immune system, which active compound contains a DNA construct which is composed of an activator sequence, a cell cycle-regulated promoter module and a DNA sequence for the active substance.

2. The active compound as claimed in claim 1, in which the promoter module possesses the CDE-CHR-Inr elements and contains positions ≤ -20 to ≥ +30 of the cdc25C promoter region (nucleotide sequence: GGCTGGCGGAAGGTTTGAATGGTCAACGCCTGCGGCTGTTGATATTCTTG), where CDE constitutes the cell cycle-dependent element (nucleotide sequence: TGGCGG), CHR constitutes the cell cycle gene homology region (nucleotide sequence: GTTTGAA) and Inr constitutes the initiation site (position +1) and also the neighboring sequences which are important for initiation.

3. The active compound as claimed in claim 1, containing an activator sequence (promoter sequence or enhancer sequence) which is regulated by transcription factors which are formed to a particularly great degree in cells of the hematopoietic system, in synovial cells, in virus-infected cells, in parasites, in macrophages, in lymphocytes or in leukemia cells.

4. The active compound as claimed in claim 3, containing the CMV promoter sequence, the CMV enhancer sequence or the SV40 promoter sequence.

5. The active compound as claimed in claim 3, for the therapy of an inadequate formation of blood cells, in which the activator sequence constitutes the promoter sequence of a gene for a cytokine, or of the receptor for this cytokine, which is activated in undifferentiated, or only slightly differentiated, cells of the hematopoietic system or in directly adjacent stroma cells.

6. The active compound as claimed in claim 5, containing the promoter sequence for stem cell factor, interleukin (IL)-1α, IL-1β, IL-3, IL-6, LIF or granulocyte macrophage colony stimulating factor, or the promoter sequence of the receptors for stem cell factor, IL-1 or IL-3, IL-6, LIF or GM-CSF, or the promoter sequence for interferon regulatory factor 1 (IRF-1).

7. The active compound as claimed in claim 3, for the therapy of autoimmune diseases, allergies and inflammations, and for preventing organ rejections, in which the activator sequence constitutes the promoter sequence of a gene for a protein which is formed to an increased extent when macrophages or lymphocytes are activated.

8. The active compound as claimed in claim 7, containing the promoter sequence for interleukin (IL)-1α or IL-1β, IL-1 receptor, IL-2, IL-2 receptor, interferon γ, IL-3, IL-3 receptor, IL-4, IL-4 receptor, IL-5, IL-6, interferon regulatory factor 1, IFN-γ responsive promoter, IL-7, IL-8, IL-9, IL-10, IL-11, IL-12, IL-13, granulocyte macrophage colony stimulating factor (GM-CSF), GM-CSF receptor, granulocyte colony stimulating factor (G-CSF), macrophage colony stimulating factor receptor, macrophage scavenger type I or type II receptor or leukemia-inhibiting factor (LIF), LFA-1, MAC-1 or p150.95.

9. The active compound as claimed in claim 1 for the therapy of arthritis, containing an activator sequence (promoter sequence or enhancer sequence) which is regulated by transcription factors which are formed in synovial cells or inflammatory cells.

10. The active compound as claimed in claim 9, containing the promoter sequence for a matrix metalloproteinase gene (MMP), a tissue inhibitor of metalloproteinases (TIMP) gene, or for the M-CSF receptor or macrophage scavenger type I or type II receptor.

11. The active compound as claimed in claim 10, containing the promoter sequence for the MMP-1 gene, the MMP-2 gene, the MMP-3 gene, the MMP-9 gene, the TIMP-1 gene or the TIMP-2 gene.

12. The active compound as claimed in claim 10, containing the promoter sequence for the tissue inhibitor of metalloproteinase-3 gene, containing promoter-active DNA fragments, containing position ≤ -463 to ≥ -2 of the following nucleotide sequence:

-500 ATGGCTTCCC ATATCCCAGA GAGTAAGAAC CAGAGAGAGA GAGAGAAAGA GAGAGAGTTT
-440 GGGTCTTTCT CCTCTGTGCC TGCTCTCTCC AGAGAAACTG GAGGGGTAGC AGTTAGCATT
-380 CCCCCGCTGG TTCCACCAAG CACAGTCAAG GTCTCTAGGA CATGGCCACC CCTCACCTGT
-320 GGAAGCGGTC CTGCTGGGGT GGGTGGGTGT TAGTTGGTTC TGGTTTGGGT CAGAGACACC

NF1

-260 CAGTGGCCCA GGTGGGCGTG GGGCCAGGGC GCAGACGAGA AGGGGCACGA GGGCTCCGCT

-200 CCGAGGACCC AGCGGCAAGC ACCGGTCCCG GGCGCGCCCC AGCCCACCCA CTCGCGTGCC

Sp1 Sp1

-140 CACGGCGGCA TTATTCCCTA TAAGGATCTG AACGATCCGG GGGCGGCCCC GCCCCGTTAC

Sp1 C/EBP

-80 CCCTTGCCCC CGGCCCCGCC CCCTTTTTGG AGGGCCGATG AGGTAATGCG GCTCTGCCAT

Sp1 ↓ Start

-20 TGGTCTGAGG GGGCGGGCCC CAACAGCCCG AGGCGGGGTC CCCGGGGGCC CAGCGCTATA

13. The active compound as claimed in claim 12, containing promoter-active DNA fragments for the tissue inhibitor of metalloproteinase-3 gene, containing
- position ≥ -463 to < -10
or
- position ≥ -112 to ≤ -2
or
- position ≥ -112 to ≤ -10
of the nucleotide sequence listed in claim 12.

14. The active compound as claimed in claims 1-3 for the therapy of viral infections, in which active compound the activator sequence constitutes the promoter sequence of viruses which transform the cells which they have infected and stimulate these cells to proliferate.

15. The active compound as claimed in claim 14, containing the promoter sequence of hepatitis B virus, hepatitis C virus, herpes simplex virus I and II, human papilloma viruses, in particular HPV-16 and HPV-18, human immunodeficiency virus, in particular HIV-1, HIV-2 and HIV-3, Epstein Barr virus or human T cell leukemia virus.

16. The active compound as claimed in claim 1 for the prophylaxis of infections, containing the promoter sequence for interleukin (IL)-1α or IL-1β, IL-1 receptor, IL-2, IL-2 receptor, interferon γ, IL-3, IL-3 receptor, IL-4, IL-4 receptor, IL-5, IL-6, interferon regulatory factor 1, IFN-γ responsive promoter, IL-7, IL-8, IL-9, IL-10, IL-11, IL-12, IL-13, granulocyte macrophage colony stimulating factor (GM-CSF), M-CSF receptor, macrophage scavenger type I or type II receptor, GM-CSF receptor, granulocyte colony stimulating factor (G-CSF) or leukemia-inhibiting factor (LIF), LFA-1, MAC-1 or p150.95.

17. The active compound as claimed in claim 1 for the therapy of leukemias, containing an activator sequence (promoter sequence or enhancer sequence) which is activated by transcription factors which are formed in leukemia cells.

18. The active compound as claimed in claim 17, containing
- the promoter sequence for c-myc, bcl-2, bcl-1, IL-6, IL-10, TNFα or TNFβ
or
- the binding sequence for proteins which are formed in HOX-11, BCR-Ab1, E2A-PBX-1 or PML/RARA, or
- the human myc E box in single or multiple form.

19. The active compound as claimed in claim 5, wherein the DNA sequence for the active substance encodes erythropoietin, G-CSF, GM-CSF, IL-3, LIF, IL-11 and/or thrombopoietin.

20. The active compound as claimed in claim 7, wherein the DNA sequence for the active substance encodes
- IFNα, IFNβ, IFNγ, IL-10, soluble IL-4 receptors, TGFβ, IL-12, soluble IL-1 receptors, soluble IL-2 receptors, soluble IL-6 receptors, IL-1 receptor antagonists, IL-1, IL-4, IL-6, IL-9, IL-13, TNFα or TNFβ, or
- an immunosuppressive antibody, in particular having specificity for CD4, CD8, CD3, IL-2 receptor, IL-1 receptor or IL-4 receptor, CD2, LFA-1, CD28, CD40 linker or CD40, or
- fragments of this antibody which contain VH and VL or constitute its VH and VL fragments which are connected via a linker.

21. The active compound as claimed in claim 9, wherein the DNA sequence for an antiinflammatory substance encodes
- an inhibitor for inflammatory interleukins or growth factors, or
- an antiinflammatory interleukin, or
- a growth factor which stimulates synthesis of the extracellular matrix, or
- superoxide dismutase, or
- a proteinase inhibitor.

22. The active compound as claimed in claim 21, wherein
- the inhibitor for inflammatory interleukins or growth factors is the interleukin-1 receptor antagonist, the soluble interleukin-1 receptor or the soluble tumor necrosis factor α (TNFα) receptor, or
- the antiinflammatory interleukin IL-4, IL-6 or IL-10, or
- the growth factor which stimulates synthesis of the extracellular matrix, insulin-like growth factor or transforming growth factor β (TGFβ), or
- constitutes the proteinase inhibitor tissue inhibitor of metalloproteinase-1 (TIMP-1), TIMP-2 or TIMP-3.

23. The active compound as claimed in claim 14, wherein the DNA sequence for the active substance encodes
- IFNα, IFNβ, IFNγ, TNF, in particular TNFα, IL-1 or TGFβ, or
- an antibody of a specificity which inactivates the relevant virus, or
- a VH and VL-containing fragment of this antibody or its VH and VL fragments which are connected via a linker, or
- a rev-binding protein, in particular RBP9-27, RBP1-8U or RBP1-8D.

24. The active compound as claimed in claim 16, wherein the DNA sequence for the active substance encodes
- the neutralization or inactivation antigen of a virus, of a bacterium or of a parasite, or
- an antiidiotype antibody, or a fragment of this antibody, whose complementarity determining regions are a copy of the protein structure or carbohydrate structure of the neutralization antigen of an infectious pathogen.

25. The active compound as claimed in claim 24, wherein the DNA sequence encodes the antigens of influenza, HIV, rabies, HSV, RSV, parainfluenza virus, rota-virus, VZV, CMV, measles virus, HPV, HBV, HCV, HDV, HEV, HAV, Vibriocholera toxin, Borrelia burgdorferi, Helicobacter pylori or malaria.

26. The active compound as claimed in claims 7, 14 and 17, wherein the active substance is a DNA sequence for a cell cycle inhibitor.

27. The active compound as claimed in claim 26, wherein the DNA sequence for the cell cycle inhibitor encodes
- the retinoblastoma protein pRb/p110, or the related p107 and p130 proteins, or
- the p53 protein, or
- the p21 protein, the P16 protein or another cyclin-dependent kinase (cdK) inhibitor, or
- the GADD45 protein, or
- the bak protein, or
- a cytotoxic protein, or
- a cytostatic cytokine, or
- an enzyme for cleaving precursors of cytostatic agents to form cytostatic agents.

28. The active compound as claimed in claim 27,
- wherein the DNA sequence for the retinoblastoma protein (pRb/p110) can no longer be phosphorylated as a result of the replacement of the amino acids in positions 246, 350, 601, 605, 780, 786, 787, 800 and 804, without, however, the mutated pRb/p110 forfeiting its binding activity with the large T antigen, in particular with the amino acids Thr-246, Ser-601, Ser-605, Ser-780, Ser-786, Ser-787 and Ser-800 being replaced with Ala, the amino acid Thr-350 being replaced with Arg and the amino acid Ser-804 being replaced with Glu, or
- wherein the DNA sequence for the p107 protein or the P130 protein is mutated in a manner which is analogous to that in the case of the mutated pRb/p110 protein.

29. The active compound as claimed in claim 27, wherein the DNA sequence for the p53 protein is truncated at the C terminus by removing serine 392.

30. The active compound as claimed in claim 27, wherein the DNA sequence encodes the cytotoxic protein or cytostatic cytokine perforin, granzyme, IL-2, IL-4, IFNα, IFNβ, IFNγ, TNFα, TNFβ, TGFβ or oncostatin M.

31. The active compound as claimed in claim 27, wherein the enzyme is a herpes simplex virus thymidine kinase, cytosine deaminase, varicella zoster virus thymidine kinase, nitroreductase, β-glucuronidase (in particular a human, plant or bacterial β-glucuronidase), carboxypeptidase (preferably from Pseudomonas), lactamase (preferably from Bacillus cereus), pyroglutamate aminopeptidase, D-aminopeptidase, oxidase, peroxidase, phosphatase, hydroxynitrile lyase, protease, esterase or a glycosidase, with the homologous signal sequence, or, for ensuring improved cellular secretion, a heterologous signal sequence, being used.

32. The active compound as claimed in claim 31, wherein lysosomal storage is decreased, and extracellular secretion is increased, by means of point mutations in the DNA sequence of the enzyme.

33. The active compound as claimed in claims 19-32, which contains the DNA sequences of several identical or different active substances, with two DNA sequences being connected to each other through a DNA sequence for the internal ribosome entry site.

34. The active compound as claimed in claim 33, wherein one active substance encodes the antigen, or the antiidiotype antibody for this antigen, of an infectious pathogen and the second active substance encodes a cytokine or a cytokine receptor, in particular
- TGFβ, IFNα, IFNβ, IFNγ, IL-12 or soluble IL-4 receptors, or
- IL-4, IL-6, LIF, IL-9, IL-10, IL-13, TNFα or TNFβ, or
- IL-1, IL-2, M-CSF or GM-CSF.

35. The active compound as claimed in claims 1-34, which is inserted into a vector.

36. The active compound as claimed in claim 35, wherein the vector is a virus.

37. The active compound as claimed in claim 36, wherein the virus is a retrovirus, adenovirus, adeno-associated virus, herpes simplex virus or vaccinia virus.

38. The active compound as claimed in claims 1-34, which is inserted into a plasmid.

39. The active compound as claimed in claims 35-38, which is prepared in a colloidal dispersion system.

40. The active compound as claimed in claim 39, wherein the colloidal dispersion system is liposomes.

41. The active compound as claimed in claim 39, wherein the colloidal dispersion system is polylysine ligands.

42. The active compound as claimed in claims 1-41, which is supplemented with a ligand which binds to membrane structures of hematopoietic cells, to activated lymphocytes, to activated macrophages, to activated synovial cells, to virus-infected cells or to leukemia cells.

43. The active compound as claimed in claim 42, wherein the ligand
- is a polyclonal or monoclonal antibody, or an antibody fragment thereof, which specifically binds, by its variable domains, to the cell membrane, or
- is a polyclonal or monoclonal antibody, or an antibody fragment thereof, which binds, by its constant domains, to Fc receptors on the cell membrane, or
- is a cytokine or growth factor, or a fragment or a constituent sequence thereof, which binds to the corresponding receptors on the cell membrane, or
- is a ligand containing a terminal galactose, which ligand binds to the asialoglycoprotein receptor, or
- is a transferrin, or fragment thereof, which binds to the transferrin receptor, or
- is an insulin, or a fragment thereof, which binds to the insulin receptor, or
- is a ligand which contains a terminal mannose, which ligand binds to the mannose 6-phosphate receptor.

44. The active compound as claimed in claim 43, wherein the cell membrane structures are receptors for cytokines, growth factors, interferons or chemokines, in particular receptors for stem cell factor, IL-1, IL-2, IL-3, IL-4, IL-6, IL-8, IL-10, interferon α, interferon β, interferon γ, LIF, GM-CSF, G-CSF, TNF∝, EGF, FGF, TGFβ, PDGF or IGF.

45. The active compound as claimed in claim 21, wherein the membrane structure of synovial cells or inflammatory cells is vimentin, fibronectin, IL-1 receptor, IL-2 receptor, TNFα receptor, IL-4 receptor, IL-10 receptor, IGF receptor, TGFβ receptor or mannose 6-phosphate receptor.

46. The active compound as claimed in claim 43, wherein the cell membrane structures are antigens which are elicited by infection with viruses, in particular by HBV, HCV, HAV, HSV, HPV, EBV, HTLV or HIV.

47. The active compound as claimed in claim 43, wherein the membrane structures of leukemia cells are receptors for IFNá, IL-2, FGF, TGFâ or retinoids.

48. The active compound as claimed in claims 1-47, wherein the vector is mixed with a cytokine, a cytokine receptor or an adjuvant, or with substances which facilitate uptake and immunization by way of the mucous membrane.

49. The active compound as claimed in claim 48, wherein
- IL-1α, IL-1β, IL-2, IL-3, IL-4, IL-5, IL-6, IL-10, IL-12, IFNγ, M-CSF, GM-CSF and/or IL-4 receptor, or
- liposomes, biodegradable polymers, muramyl dipeptides, lipopolysaccharides, lipid A, or Al(OH)₃ or Ca(OH)₃
were admixed.

50. The active compound as claimed in claims 1-49, in a pharmaceutical preparation for injection into a tissue, such as muscle, connective tissue, liver, kidney, spleen, lung or skin, for local application to the skin or to mucous membranes, for injection into body cavities, such as joints, pleural space, peritoneal space or subarachinoid space, or for injection into the blood vessel system, such as intraarterial or intravenous injection, or for oral uptake.

## Revendications

1. Principe actif pour la prophylaxie ou la thérapie de maladies qui sont dues au système immunitaire, caractérisé en ce qu'il contient un produit de construction d'ADN qui consiste en une séquence d'activateur, un module promoteur régulé par le cycle cellulaire et une séquence d'ADN codant pour la substance active.

2. Principe actif selon la revendication 1, dans lequel le module promoteur comporte les éléments CDE-CHR-Inr et contient les positions ≤ -20 à ≥ +30 de la région de promoteur cdc25C (séquence nucléotidique CGCTGGCGGAAGGTTTGAATGGTCAACGCCTGCGGCTGTTGATATTC-TTG), CDE représentant "l'élément dépendant du cycle cellulaire" (séquence nucléotidique TGGCGG), CHR représentant la région d'homologie de gène de cycle cellulaire (séquence nucléotidique GTTTGAA) et Inr représentant le site d'initiation (position +1) ainsi que les séquences voisines importantes pour l'initiation.

3. Principe actif selon la revendication 1, contenant une séquence d'activateur (séquence de promoteur ou d'amplificateur), qui est régulée par des facteurs de transcription formés à un degré particulier dans des cellules du système hématopoïétique, dans des cellules synoviales, dans des cellules infectées par des virus, dans des parasites, dans des macrophages, dans des lymphocytes ou dans des cellules leucémiques.

4. Principe actif selon la revendication 3, contenant la séquence de promoteur de CMV, la séquence d'activateur de CMV ou la séquence de promoteur de SV40.

5. Principe actif selon la revendication 3, pour le traitement d'une formation insuffisante de cellules sanguines, dans lequel la séquence d'activateur est la séquence de promoteur d'un gène codant pour une cytokine ou du récepteur pour cette cytokine, qui est activé dans des cellules indifférenciées ou faiblement différenciées du système hématopoïétique ou dans des cellules de stroma immédiatement voisines.

6. Principe actif selon la revendication 5, contenant la séquence de promoteur pour le facteur de prolifération des mastocytes, l'interleukine (IL)-1α, IL-1β, IL-3, IL-6, LIF ou le facteur stimulant la formation de colonies de granulocytes et de macrophages, ou la séquence de promoteur des récepteurs pour le facteur de prolifération des mastocytes, IL-1 ou IL-3, IL-6, LIF, GM-CSF, ou la séquence de promoteur pour le facteur régulateur d'interféron 1 (IRF-1).

7. Principe actif selon la revendication 3, pour le traitement de maladies auto-immunes, d'allergies, d'inflammations et pour la prévention de rejets d'organes, dans lequel la séquence d'activateur représente la séquence de promoteur d'un gène codant pour une protéine qui est formée de façon accentuée lors de l'activation de macrophages ou de lymphocytes.

8. Principe actif selon la revendication 7, contenant la séquence de promoteur pour l'interleukine (IL)-1α ou IL-1β, le récepteur d'IL-1, IL-2, le récepteur d'IL-2, l'interféron γ, IL-3, le récepteur d'IL-3, IL-4, le récepteur d'IL-4, IL-5, IL-6, le facteur régulateur d'interféron 1, le promoteur répondant à IFN-γ, IL-7, IL-8, IL-9, IL-10, IL-11, IL-12, IL-13, le facteur stimulant la formation de colonies de granulocytes et de macrophages (GM-CSF), le récepteur de GM-CSF, le facteur stimulant la formation de colonies de granulocytes (G-CSF), le récepteur du facteur de stimulation de la formation de colonies de macrophages, le récepteur de capteur de macrophages de type I ou de type II, ou le facteur inhibiteur de leucémie (LIF), LFA-1, MAC-1 ou p150,95.

9. Principe actif selon la revendication 1, pour le traitement de l'arthrite, contenant une séquence d'activateur (séquence de promoteur ou d'amplificateur), qui est régulé par des facteurs de transcription formés dans des cellules synoviales ou dans des cellules inflammatoires.

10. Principe actif selon la revendication 9, contenant la séquence de promoteur pour un gène de métalloprotéase de matrice (MMP), un gène d'inhibiteur tissulaire de métalloprotéases (TIMP) ou pour le récepteur de M-CSF ou le récepteur de capteur de macrophages de type I ou de type II.

11. Principe actif selon la revendication 10, contenant la séquence de promoteur pour le gène de MMP-1, MMP-2, MMP-3, MMP-9, TIMP-1 ou TIMP-2.

12. Principe actif selon la revendication 10, contenant la séquence de promoteur pour le gène de l'inhibiteur tissulaire de métalloprotéase 3, contenant des fragments d'ADN à activité de promoteur, contenant la position ≤ -463 à ≥ -2 de la séquence nucléotidique suivante :

-500 ATGGCTTCCC ATATCCCAGA GAGTAAGAAC CAGAGAGAGA GAGAGAAAGA GAGAGAGTTT
-440 GGGTCTTTCT CCTCTGTGCC TGCTCTCTCC AGAGAAACTG GAGGGGTAGC AGTTAGCATT
-380 CCCCCGCTGG TTCCACCAAG CACAGTCAAG GTCTCTAGGA CATGGCCACC CCTCACCTGT
-320 GGAAGCGGTC CTGCTGGGGT GGGTGGGTGT TAGTTGGTTC TGGTTTGGGT CAGAGACACC

NF1
-260 CAGTGGCCCA GGTGGGCGTG GGGCCAGGGC GCAGACGAGA AGGGGCACGA GGGCTCCGCT

-200 CCGAGGACCC AGCGGCAAGC ACCGGTCCCG GGCGCGCCCC AGCCCACCCA CTCGCGTGCC

Sp1 Sp1
-140 CACGGCGGCA TTATTCCCTA TAAGGATCTG AACGATCCGG GGGCGGCCCC GCCCCGTTAC
C/EBP

Sp1
-80 CCCTTGCCCC CGGCCCCGCC CCCTTTTTGG AGGGCCGATG AGGTAATGCG GCTCTGCCAT

Sp1 Start
-20 TGGTCTGAGG GGGCGGGCCC CAACAGCCCG AGGCGGGGTC CCCGGGGGCC CAGCGCTATA

13. Principe actif selon la revendication 12, contenant des fragments d'ADN à activité de promoteur, pour le gène de l'inhibiteur tissulaire de la métalloprotéase 3, contenant
- la position ≥ -463 à ≤ -10
ou
- la position ≥ -112 à ≤ -2
ou
- la position ≥ -112 à ≤ -10
de la séquence nucléotidique indiquée dans la revendication 12.

14. Principe actif selon l'une des revendications 1 à 3, pour le traitement d'infections virales, dans lequel la séquence d'activateur représente la séquence de promoteur pour des virus qui transforment les cellules infectées par ceux-ci et stimulent leur prolifération.

15. Principe actif selon la revendication 14, contenant la séquence de promoteur du virus de l'hépatite B, du virus de l'hépatite C, des herpès simplex virus I et II, des papillomavirus humains, en particulier HPV-16 et HPV-18, du virus de l'immunodéficience humaine, en particulier VIH-1, VIH-2 et VIH-3, du virus d'Epstein-Barr ou du virus de la leucémie à lymphocytes T humaine.

16. Principe actif selon la revendication 1, pour la prophylaxie d'infections, contenant la séquence de promoteur pour l'interleukine (IL)-1α ou IL-1β, le récepteur d'IL-1, IL-2, le récepteur d'IL-2, l'interféron γ, IL-3, le récepteur d'IL-3, IL-4, le récepteur d'IL-4, IL-5, IL-6, le facteur régulateur d'interféron 1, le promoteur répondant à IFN-γ, IL-7, IL-8, IL-9, IL-10, IL-11, IL-12, IL-13, le facteur stimulant la formation de colonies de granulocytes et de macrophages (GM-CSF), le récepteur de M-CSF, le récepteur de capteur de macrophages de type I ou de type II, le récepteur de GM-CSF, le facteur stimulant la formation de colonies de granulocytes (G-CSF) ou le facteur inhibiteur de leucémie (LIF), LFA-1, Mac-1 ou p150,95.

17. Principe actif selon la revendication 1, pour le traitement de leucémies, contenant une séquence d'activateur (séquence de promoteur ou d'amplificateur), qui est activée par des facteurs de transcription formés dans des cellules leucémiques.

18. Principe actif selon la revendication 17, contenant
- la séquence de promoteur pour c-myc, bcl-2, bcl-1, IL-6, IL-10, TNFα ou TNFβ, ou
- la séquence de liaison pour des protéines, formée dans HOX-11, BCR-Ab1, E2A-PBX-1, PML/RARA, ou
- la boîte myc E humaine sous forme simple ou multiple.

19. Principe actif selon la revendication 5, dans lequel la séquence d'ADN code pour la substance active érythropoïétine, G-CSF, GM-CSF, IL-3, LIF, IL-11 et/ou thrombopoïétine.

20. Principe actif selon la revendication 7, dans lequel la séquence d'ADN pour la substance active
- code pour IFNα, IFNβ, IFNγ, IL-10, les récepteurs d'IL-4 solubles, TGFβ, IL-12, les récepteurs d'IL-1 solubles, les récepteurs d'IL-2 solubles, les récepteurs d'IL-6 solubles, les antagonistes de récepteurs d'IL-1, IL-1, IL-4, IL-6, IL-9, IL-13, TNFα ou TNFβ, ou
- code pour un anticorps immunosuppresseur, en particulier à spécificité pour CD4, CD8, CD3, le récepteur d'IL-2, le récepteur d'IL-1 ou le récepteur d'IL-4, CD2, LFA-1, CD28, le lieur de CD40 ou CD40, ou
- code pour des fragments de cet anticorps qui contiennent VH et VL ou représentent des fragments VH et VL de celui-ci liés par un linker.

21. Principe actif selon la revendication 9, dans lequel la séquence d'ADN code pour une substance anti-inflammatoire
- un inhibiteur de facteurs de croissance ou d'interleukine induisant l'inflammation ou
- une interleukine inhibant l'inflammation ou
- un facteur de croissance qui stimule la synthèse de la matrice extracellulaire ou
- la superoxyde dismutase ou
- un inhibiteur de protéinase.

22. Principe actif selon la revendication 21, dans lequel
- l'inhibiteur de facteurs de croissance ou d'interleukines induisant l'inflammation est l'antagoniste des récepteurs d'interleukine 1, le récepteur d'interleukine 1 soluble ou le récepteur de facteur de nécrose tumorale α (TNFα) soluble, ou
- est l'interleukine inhibitrice d'inflammation IL-4, -6 ou -10, ou
- est le facteur de croissance qui stimule la synthèse de la matrice extracellulaire, le facteur de croissance ressemblant à l'insuline ou le facteur de croissance transformant β (TGFβ), ou
- représente l'inhibiteur de protéinase inhibiteur tissulaire de métalloprotéase 1 (TIMP-1), TIMP-2 ou TIMP-3.

23. Principe actif selon la revendication 14, dans lequel la séquence d'ADN pour la substance active
- code pour IFNα, IFNβ, IFNγ, TNF, en particulier TNFα, IL-1 ou TGFβ, ou
- code pour un anticorps ayant une spécificité qui inactive le virus respectif, ou
- code pour un fragment contenant VH et VL de cet anticorps ou un fragment VH et VL de celui-ci lié par un linker, ou
- code pour une protéine se liant à Rev, en particulier RBP9-27, RBP1-8U ou RBP1-8D.

24. Principe actif selon la revendication 16, dans lequel la séquence d'ADN pour la substance active
- code pour l'antigène de neutralisation ou d'inactivation d'un virus, d'une bactérie ou d'un parasite, ou
- code pour un anticorps anti-idiotype ou un fragment de cet anticorps dont la "région déterminant la complémentarité" est une copie de la structure protéique ou glucidique de l'antigène de neutralisation d'un agent infectieux.

25. Principe actif selon la revendication 24, dans lequel la séquence d'ADN code pour les antigènes du virus de la grippe, VIH, du virus de la rage, HSV, RSV, du virus para-influenza, d'un rotavirus, VZV, CMV, du virus de la rougeole V, HPV, HBV, HCV, HDV, HEV, HAV, la toxine de *Vibrio cholerae, Borrelia Burgdorferi, Helicobacter pylori* ou du paludisme.

26. Principe actif selon les revendications 7, 14 et 17, dans lequel la substance active représente une séquence d'ADN codant pour un inhibiteur de cycle cellulaire.

27. Principe actif selon la revendication 26, dans lequel la séquence d'ADN code pour l'inhibiteur de cycle cellulaire
- la protéine de rétinoblastome pRb/p110 ou les protéines apparentées p107 et p130, ou
- la protéine p53 ou
- la protéine p21, la protéine P16 ou un autre inhibiteur de "kinase dépendante de la cycline (cdK)" ou
- la protéine GADD45 ou
- la protéine bak ou
- une protéine cytotoxique ou
- une cytokine cytostatique ou
- une enzyme pour la coupure de précurseurs d'agents cytostatiques en agents cytostatiques.

28. Principe actif selon la revendication 27,
- dans lequel la séquence d'ADN pour la protéine de rétinoblastome (pRb/p110) n'est plus phosphorylable en raison d'un remplacement des aminoacides aux positions 246, 350, 601, 605, 780, 786, 787, 800 et 804, sans que toutefois la pRb/p110 mutée perde son activité de liaison avec le grand antigène T, en particulier par le fait que les aminoacides Thr-246, Ser-601, Ser-605, Ser-780, Ser-786, Ser-787 et Ser-800 sont remplacés par Ala, l'aminoacide Thr-350 est remplacé par Arg et l'aminoacide Ser-804 est remplacé par Glu, ou
- dans lequel la séquence d'ADN pour la protéine p107 ou la protéine p130 est mutée de la même façon que dans le cas de la rPb/p110 mutée.

29. Principe actif selon la revendication 27, dans lequel la séquence d'ADN pour la protéine p53 est raccourcie de la sérine 392 à l'extrémité C-terminale.

30. Principe actif selon la revendication 27, dans lequel la séquence d'ADN code pour la protéine cytotoxique ou la cytokine cytostatique perforine, granzyme, IL-2, IL-4, IFNα, IFNβ, IFNγ, TNFα, TNFβ, TGFβ ou oncostatine M.

31. Principe actif selon la revendication 27, dans lequel l'enzyme est une thymidine kinase d'herpès simplex virus, la cytosine désaminase, la thymidine kinase du virus varicelle-zona, la nitroréductase, une β-glucuronidase (en particulier une β-glucuronidase humaine, végétale ou bactérienne), une carboxypeptidase (de préférence de *Pseudomonas*), une lactamase (de préférence de *Bacillus cereus*), la pyroglutamate aminopeptidase, une D-aminopeptidase, une oxydase, une peroxydase, une phosphatase, l'hydroxynitrile lyase, une protéase, une estérase ou une glycosidase, la séquence de signal homologue ou, pour une meilleure excrétion cellulaire, une séquence signal hétérologue, étant utilisée.

32. Principe actif selon la revendication 31, dans lequel le stockage lysosomique est diminué et l'excrétion extracellulaire est accrue par des mutations ponctuelles de la séquence d'ADN de l'enzyme.

33. Principe actif selon les revendications 19 à 32, qui contient les séquences d'ADN de plusieurs substances actives identiques ou différentes, deux séquences d'ADN étant reliées l'une à l'autre par une séquence d'ADN pour le site d'entrée interne du ribosome.

34. Principe actif selon la revendication 33, dans lequel une substance active code pour l'antigène d'un agent infectieux ou l'anticorps anti-idiotype pour cet antigène, et la seconde substance active code pour une cytokine ou un récepteur de cytokine, en particulier
- TGFβ, IFNα, IFNβ, IFNγ, IL-12 ou des récepteurs d'IL-4 solubles, ou
- IL-4, IL-6, LIF, IL-9, IL-10, IL-13, TNFα ou TNFβ ou
- IL-1, IL-2, M-CSF ou GM-CSF.

35. Principe actif selon les revendications 1 à 34, introduit dans un vecteur.

36. Principe actif selon la revendication 35, dans lequel le vecteur est un virus.

37. Principe actif selon la revendication 36, dans lequel le virus représente un rétrovirus, un adénovirus, un virus adéno-associé, l'herpès simplex virus ou le virus de la vaccine.

38. Principe actif selon les revendications 1 à 34, introduit dans un plasmide.

39. Principe actif selon les revendications 35 à 38, préparé dans un système de dispersion colloïdal.

40. Principe actif selon la revendication 39, dans lequel le système de dispersion colloïdal consiste en liposomes.

41. Principe actif selon la revendication 39, dans lequel le système de dispersion colloïdal consiste en ligands de polylysine.

42. Principe actif selon les revendications 1 à 41, complété par un ligand qui se fixe à des structures membranaires de cellules hématopoïétiques, à des lymphocytes activés, des macrophages activés, des cellules synoviales activées, des cellules infectées par des virus ou à des cellules leucémiques.

43. Principe actif selon la revendication 42, dans lequel le ligand
- est un anticorps monoclonal ou polyclonal ou un fragment d'anticorps de celui-ci, qui se lie spécifiquement par ses domaines variables à la membrane cellulaire ou
- est un anticorps monoclonal ou polyclonal ou un fragment d'anticorps de celui-ci, qui se lie, par ses domaines constants, aux récepteurs Fc sur la membrane cellulaire ou
- est une cytokine ou un facteur de croissance ou un fragment ou une séquence partielle de ceux-ci, qui se lie aux récepteurs correspondants sur la membrane cellulaire ou
- est un ligand contenant un résidu galactose en bout de chaîne, qui se lie au récepteur d'asialoglycoprotéine ou
- est une transferrine ou un fragment de celle-ci, qui se lie au récepteur de transferrine ou
- est une insuline ou un fragment de celle-ci, qui se lie au récepteur de l'insuline ou
- est un ligand contenant un résidu mannose en bout de chaîne, qui se lie au récepteur de mannose-6-phosphate.

44. Principe actif selon la revendication 43, dans lequel les structures de cellules membranaires représentent des récepteurs pour des cytokines, des facteurs de croissance, des interférons, des chémokines, en particulier des récepteurs pour le facteur de prolifération des mastocytes, pour IL-1, IL-2, IL-3, IL-4, IL-6, IL-8, IL-10, l'interféron α, l'interféron β, l'interféron γ, LIF, GM-CSF, G-CSF, TNFα, EGF, FGF, TGFβ, PDGF ou IGF.

45. Principe actif selon la revendication 21, dans lequel la structure membranaire de cellules synoviales ou de cellules inflammatoires est la vimentine, la fibronectine, le récepteur d'IL-1, le récepteur d'IL-2, le récepteur de TNFα, le récepteur d'IL-4, le récepteur d'IL-10, le récepteur d'IGF, le récepteur de TGFβ ou le récepteur de mannose-6-phosphate.

46. Principe actif selon la revendication 43, dans lequel les structures membranaires cellulaires sont des antigènes qui sont suscités par l'infection par des virus, en particulier par HBV, HCV, HAV, HSV, HPV, EBV, HTLV ou VIH.

47. Principe actif selon la revendication 43, dans lequel la structure membranaire de cellules leucémiques consiste en des récepteurs pour IFNα, IL-2, FGF, TGFβ ou pour des rétinoïdes.

48. Principe actif selon les revendications 1 à 47, dans lequel le vecteur est mélangé avec une cytokine, un récepteur de cytokine, un adjuvant ou avec des substances qui facilitent l'absorption et l'immunisation par la muqueuse.

49. Principe actif selon la revendication 48, dans lequel on a mélangé
- IL-1α, IL-1β, IL-2, IL-3, IL-4, IL-5, IL-6, IL-10, IL-12, IFNγ, M-CSF, GM-CSF et/ou le récepteur d'IL-4 ou
- des liposomes, des polymères biodégradables, des muramyldipeptides, des lipopolysaccharides, le lipide A ou Al(OH)₃ ou Ca(OH)₃.

50. Principe actif selon les revendications 1 à 49, dans une composition pharmaceutique pour l'injection dans un tissu tel que le muscle, le tissu conjonctif, le foie, le rein, la rate, le poumon, la peau, pour l'application locale sur la peau ou sur les muqueuses, pour l'injection dans des cavités corporelles telles que les articulations, la cavité pleurale, la cavité péritonéale, l'espace sous-arachnoïdien ou pour l'injection dans le système vasculaire sanguin, telle qu'une injection intra-artérielle ou intraveineuse, ou pour l'absorption orale.
